# EUROPEAN PATENT SPECIFICATION

(11) **EP 2 859 104 B1**
(45) Date of publication and mention of the grant of the patent: **31.05.2017**
(21) Application number: 13800385.0
(22) Date of filing: 05.06.2013
(51) Int. Cl.: C12N 15/82, A01H 5/00

(54) **CONSTRUCT AND METHOD FOR EXPRESSING TRANSGENES USING A BRASSICA BIDIRECTIONAL CONSTITUTIVE PROMOTER**
KONSTRUKT UND VERFAHREN ZUR EXPRESSION VON TRANSGENEN MIT EINEM BIDIREKTIONALEN KONSTITUTIVEN BRASSICA-PROMOTOR
CONSTRUCTION ET PROCÉDÉ D'EXPRESSION DE TRANSGÈNES AU MOYEN D'UN PROMOTEUR CONSTITUTIF BIDIRECTIONNEL DE BRASSICA

(30) Priority: 07.06.2012 US 201261656634 P
(43) Date of publication of application: 15.04.2015
(73) Proprietor: Dow AgroSciences LLC, Indianapolis IN 46268-1054 (US)
(72) Inventor: GUPTA, Manju, Carmel Indiana 46032 (US); RUSSELL, Sean Michael, Carmel Indiana 46032 (US); SHEN, Liu Yin, Westfield Indiana 46074 (US); CHENNAREDDY, Sivarama Reddy, West Lafayette Indiana 47906 (US); ROUT, Jyoti R., Portland Oregon 97229 (US); NOVAK, Stephen, Westfield Indiana 46074 (US)
(74) Representative: Weickmann & Weickmann PartmbB
(86) International application number: PCT/US2013/044262
(87) International publication number: WO 2013/184764

(56) References cited:
- US-A1- 2013 104 257
- US-B1- 6 388 170
- US-B2- 7 053 265
- US-B2- 7 129 343
- DATABASE EMBL [Online] 5 January 2006 (2006-01-05), "KBrH075B12F KBrH (HindIII) BAC library Brassica rapa subsp. pekinensis Brassica rapa subsp. pekinensis genomic clone KBrH075B12, genomic survey sequence.", XP002751661, retrieved from EBI accession no. EMBL:DU984581 Database accession no. DU984581
- DATABASE GENBANK [Online] 31 May 2010 'National Academy of Agricultural Science. Brassica Rapa Subsp. Pekinensis Clone KBrH003H20.', XP055176636 Retrieved from NCBI Database accession no. AC241089.1

### SEQUENCE LISTING

<110> Dow AgroSciences LLC
   Gupta, Manju
   Russell, Sean
   Shen, Liu
   Chennareddy, Sivarama
   Rout, Jyoti
   Novak, Stephen
<120> CONSTRUCT AND METHOD FOR EXPRESSING TRANSGENES USING A
<130> 70136-US-NP
<160> 33
<170> PatentIn version 3.5
<210> 1
   <211> 739
   <212> DNA
   <213> Brassica napus
<400> 1
   acagagagaa aggaatatgc tttcgtagga agtgaacgaa aaagaaatgg ttaagctcaa      60
   tacttgctcg gattctaaac caaattgaga gagagttccg aattgccgtg gtttatccta     120
   aaccgaacct cagatcggtt tgattgtttg gggtttgtga catatataac tggaaaaaga     180
   catcccccgg tttgattatg ttccaaactc taaaccagat tgagattatt ttccgataag     240
   cttatacatt cctgtcgggt attttcggat atgtttgaat cttggataat atccgatccg     300
   aaccggtacc cgaaacataa atgaaagcat acccaatcgg atatttcacc atatctagat     360
   ccgacctaga acccgatttt ttggatcgga tccagacatg gatgttacca tcgtcttcga     420
   ttaaaggtaa tctggccggt tactcttcga tttatgtttc ctctccaata ttctagtagt     480
   aaaccgcaat cgattattta tgagttagat tggttcatgg gtttttgtgt taaaaaggcc     540
   cagtaacttc ttggaggccc attttaaaac caccaaatct taaccctttc aagtcttcac     600
   ctaaccaaaa ccttccttcc cacttgctgt ctgtaacacg tggcaggttc tcattggcta     660
   ataggaaatg ctcatacacc atcgtttgag gtgggttggt gaaggatttt gatggctacc     720
   ttcttcgtca ccaaacccc                                                  739
<210> 2
   <211> 1232
   <212> DNA
   <213> Artificial Sequence
<220>
   <223> Full promoter including the addition of a NcoI restriction enzyme site
<400> 2
   catggacttg aaaactgaat ctctctgttt cagacaaaac cctcctacaa agaatgttcc      60
   ttttcagaca aaaacccaac taatattcta gatcttgaag tgaatcatca tcatcatcaa 120
   acccgtaaag atctttctag tgtctacagt gttagttccc ctataaagtt cacagctttt 180
   tcgaatgagg ctcgagtata gtggtgatct gaatctagag tttcagatgg aagacaaaag 240
   agatcacctg gaatgattta gctttgtgtc tgtgtataca gcactattca gacaggaaga 300
   agcttcgcgt cgaataatgt ttttgatcag ctttcacgcc acgtaagtag aagaaaacga 360
   aacatcaagc agctctaagc cggatccttt tttagcccgg aggtgaagga gatacagaga 420
   gaaaggaata tgctttcgta ggaagtgaac gaaaaagaaa tggttaagct caatacttgc 480
   tcggattcta aaccaaattg agagagagtt ccgaattgcc gtggtttatc ctaaaccgaa 540
   cctcagatcg gtttgattgt ttggggtttg tgacatatat aactggaaaa agacatcccc 600
   cggtttgatt atgttccaaa ctctaaacca gattgagatt attttccgat aagcttatac 660
   attcctgtcg ggtattttcg gatatgtttg aatcttggat aatatccgat ccgaaccggt 720
   acccgaaaca taaatgaaag catacccaat cggatatttc accatatcta gatccgacct 780
   agaacccgat tttttggatc ggatccagac atggatgtta ccatcgtctt cgattaaagg 840
   taatctggcc ggttactctt cgatttatgt ttcctctcca atattctagt agtaaaccgc 900
   aatcgattat ttatgagtta gattggttca tgggtttttg tgttaaaaag gcccagtaac 960
   ttcttggagg cccattttaa aaccaccaaa tcttaaccct ttcaagtctt cacctaacca 1020
   aaaccttcct tcccacttgc tgtctgtaac acgtggcagg ttctcattgg ctaataggaa 1080
   atgctcatac accatcgttt gaggtgggtt ggtgaaggat tttgatggct accttcttcg 1140
   tcaccaaacc ccctctaaag tttattaaat ctgaggaaga cagagagtgg gtttaggtga 1200
   gcgagtccaa agatcgaaac tttttctcca tg 1232
<210> 3
   <211> 1232
   <212> DNA
   <213> Artificial Sequence
<220>
   <223> Reverse complement of full promoter with the addition of
   nucleotide sequences to include a restriction site
<400> 3
   catggagaaa aagtttcgat ctttggactc gctcacctaa acccactctc tgtcttcctc      60
   agatttaata aactttagag ggggtttggt gacgaagaag gtagccatca aaatccttca      120
   ccaacccacc tcaaacgatg gtgtatgagc atttcctatt agccaatgag aacctgccac     180
   gtgttacaga cagcaagtgg gaaggaaggt tttggttagg tgaagacttg aaagggttaa     240
   gatttggtgg ttttaaaatg ggcctccaag aagttactgg gcctttttaa cacaaaaacc     300
   catgaaccaa tctaactcat aaataatcga ttgcggttta ctactagaat attggagagg     360
   aaacataaat cgaagagtaa ccggccagat tacctttaat cgaagacgat ggtaacatcc     420
   atgtctggat ccgatccaaa aaatcgggtt ctaggtcgga tctagatatg gtgaaatatc     480
   cgattgggta tgctttcatt tatgtttcgg gtaccggttc ggatcggata ttatccaaga     540
   ttcaaacata tccgaaaata cccgacagga atgtataagc ttatcggaaa ataatctcaa     600
   tctggtttag agtttggaac ataatcaaac cgggggatgt ctttttccag ttatatatgt     660
   cacaaacccc aaacaatcaa accgatctga ggttcggttt aggataaacc acggcaattc     720
   ggaactctct ctcaatttgg tttagaatcc gagcaagtat tgagcttaac catttctttt     780
   tcgttcactt cctacgaaag catattcctt tctctctgta tctccttcac ctccgggcta     840
   aaaaaggatc cggcttagag ctgcttgatg tttcgttttc ttctacttac gtggcgtgaa     900
   agctgatcaa aaacattatt cgacgcgaag cttcttcctg tctgaatagt gctgtataca     960
   cagacacaaa gctaaatcat tccaggtgat ctcttttgtc ttccatctga aactctagat    1020
   tcagatcacc actatactcg agcctcattc gaaaaagctg tgaactttat aggggaacta    1080
   acactgtaga cactagaaag atctttacgg gtttgatgat gatgatgatt cacttcaaga    1140
   tctagaatat tagttgggtt tttgtctgaa aaggaacatt ctttgtagga gggttttgtc    1200
   tgaaacagag agattcagtt ttcaagtcca tg                                  1232
<210> 4
   <211> 4760
   <212> DNA
   <213> Artificial Sequence
<220>
   <223> Bidirectional promoter sequence
<400> 4
   cgacggcccg ggctggttct tcgcagctgg atcaaacgag taagcaagcc tgtgcaataa      60
   ccatatagac tttgctagct tcaaaaaggc ctgataaaac gcggtcctcg ggtgcccccc     120
   tcctgtcacg tactcacgct ggtctaggtt tccaaagaaa gaagcttcca tctttgggtg     180
   gatcaagatg agatacttgc tcctgcagaa cttaccaaag ttggaatcag ggtttgtagc     240
   cagagcatct agtggatcca tatcctttaa agcaagaaac tgagtgaaga aggtctcagt     300
   gtcattatct gagtctaatg agaaagtctc ctgctgaaac ccactgaaca tcctctggca     360
   tatgtatgac tcaaacgcat acttcttgtg aggcctcttg gcgtaagcaa caccaggctc     420
   aatggactca gctgcagaat caagatccca tcccgcagct ttcatcatgt tgatcaacgg     480
   ctttgagaaa tcgtgaacag ctttagaagc agcttcatat gtagattcaa aaagcgcaat     540
   ggtcaagtca ggaaactgca aatcaccgga agattcgtta gtggacgaca ttcctcttaa     600
   cttaagattc ttctcgagct taagacgttt ctgattagct tcctcgatct tctccaacat     660
   atgagtgatc tcggagtcct tgttctgaat ctcagactga aacttcttca ccatgacctc     720
   gtaagtcttc aacagactct gctgctcttg gatctctgcg gctagacgag agtctttggg 780
   agagacgcac acaggcttgg ggttgttctc tctgtaaaca tgtttcagct cggagagatt 840
   cttgagttcg gagatcacag ccttgtcggc ttcctgaatc ttgtcggggt cgtaaggagt 900
   gtgagcagat tgaagctgga tgtaagcaga ttttagagaa gagatgtttc caaagaggtt 960
   ggagataagt gcttccatag cttcagggtt ttggttgata cattgttcca ttggttgagg 1020
   atggaccttc tggttgttgt tgttgttgtt gagttgagtt tctttcaacc cacttggtag 1080
   catacttgaa aactgaatct ctctgtttca gacaaaaccc tcctacaaag aatgttcctt 1140
   ttcagacaaa aacccaacta atattctaga tcttgaagtg aatcatcatc atcatcaaac 1200
   ccgtaaagat ctttctagtg tctacagtgt tagttcccct ataaagttca cagctttttc 1260
   gaatgaggct cgagtatagt ggtgatctga atctagagtt tcagatggaa gacaaaagag 1320
   atcacctgga atgatttagc tttgtgtctg tgtatacagc actattcaga caggaagaag 1380
   cttcgcgtcg aataatgttt ttgatcagct ttcacgccac gtaagtagaa gaaaacgaaa 1440
   catcaagcag ctctaagccg gatccttttt tagcccggag gtgaaggaga tacagagaga 1500
   aaggaatatg ctttcgtagg aagtgaacga aaaagaaatg gttaagctca atacttgctc 1560
   ggattctaaa ccaaattgag agagagttcc gaattgccgt ggtttatcct aaaccgaacc 1620
   tcagatcggt ttgattgttt ggggtttgtg acatatataa ctggaaaaag acatcccccg 1680
   gtttgattat gttccaaact ctaaaccaga ttgagattat tttccgataa gcttatacat 1740
   tcctgtcggg tattttcgga tatgtttgaa tcttggataa tatccgatcc gaaccggtac 1800
   ccgaaacata aatgaaagca tacccaatcg gatatttcac catatctaga tccgacctag 1860
   aacccgattt tttggatcgg atccagacat ggatgttacc atcgtcttcg attaaaggta 1920
   atctggccgg ttactcttcg atttatgttt cctctccaat attctagtag taaaccgcaa 1980
   tcgattattt atgagttaga ttggttcatg ggtttttgtg ttaaaaaggc ccagtaactt 2040
   cttggaggcc cattttaaaa ccaccaaatc ttaacccttt caagtcttca cctaaccaaa 2100
   accttccttc ccacttgctg tctgtaacac gtggcaggtt ctcattggct aataggaaat 2160
   gctcatacac catcgtttga ggtgggttgg tgaaggattt tgatggctac cttcttcgtc 2220
   accaaacccc ctctaaagtt tattaaatct gaggaagaca gagagtgggt ttaggtgagc 2280
   gagtccaaag atcgaaactt tttctccaat ggcgcaagct agcagaatct gccatggcgt 2340
   gcagcagaac ccatgtgcta tctccaatct ctccaaatca aaccaccgca aatctccctt 2400
   ctctgtctcg ctgaagacgc accagcagca gcgtggagct tatcagatat cttcgcgggg 2460
   gttgaagaag agcgcgatgg tgctaaaccg ttctgtaact cgtccggtta aggttatggc 2520
   ctctgtttcc acggcggaga aagcttcgga gattgtgctt caacccatta gagaaatctc 2580
   gggtctgatc aagctacccg gatccaaatc tctgtccaac cggattcttc ttcttgccgc 2640
   tttatccgag gtttgcttct ttctttgttt gcttagtgtt gcgtttttaa cggcgtgagg 2700
   atgaagaaag gttctgactt tgttgtggtt ttatagggaa ctactgtagt tgacaacttg 2760
   ttgaacagtg atgacattaa ctacatgctt gatgcgttga acaagttggg gcttaatgtg 2820
   gaacgtgaca gtgagaacaa ccgtgcggtt gttgaaggat gtggcgggat attcccagct 2880
   tctttagatt ctaagggtga tatcgagttg taccttggga atgcaggaac agccatgcgt 2940
   ccacttacag ctgcagttac tgctgctggt ggcaacgcaa ggtaaggtta aggacttatt 3000
   ctgttagtta gttttgatta ttttaagaat cggtcttgta ctgatgcttt ttagttgggt 3060
   ttgtttacca gttatgtgct tgatggggtg cctagaatga gggaaagacc tataggagat 3120
   ttggttgttg gtcttaagca gcttggtgct gatgttgaat gtactcttgg tactaactgt 3180
   cctcctgttc gtgtcaatgc taatggtggc ctgcccggtg gaaaggtgag tttgtaattt 3240
   cagcatttgc tatgtgaaaa gttgcagcaa tctttgttca tcacactgcg ttagcttgac 3300
   atgattttag cttttgtatg gtttcttgat tgacacatta gacatgtttt tgcatttttc 3360
   aggtgaagct ttctggatca atcagtagtc aatacttgac tgcactgctc atggcagctc 3420
   ccttagctct tggagacgtt gagattgaga tcattgataa attgatttct gttccatatg 3480
   ttgaaatgac attgaagttg atggaacgtt ttggtgttag tgccgagcat agtgacagtt 3540
   gggatcgttt ctttgtcaag ggcggtcaga aatacaagta agagttgttt ctaaaatcac 3600
   tgaacttata attagattga cagaagagtg actaaccaaa tggtaaaatt tgattcaggt 3660
   cgcctggtaa tgcttacgta gaaggtgatg cttctagtgc tagttatttc ttggctggtg 3720
   ctgccattac tggtgaaacc gttactgttg aaggttgtgg aacaaccagc ctgcaggtaa 3780
   cactaagttt ataataaaat ttgcttagtt caattttttt ttgtctttct aaggcttggc 3840
   tagttgtgtc acttgtgtgt aacatatgaa gaatctaagt ttagtttttt ttggtgatga 3900
   atctcaaagg gagatgtgaa gttcgctgag gttcttgaga aaatgggatg taaagtgtca 3960
   tggacagaga acagtgtgac tgtgactgga ccatctagag atgcttttgg aatgagacac 4020
   ttgcgtgctg ttgatgtcaa catgaacaaa atgcctgatg tagccatgac tcttgccgtt 4080
   gtagctctct ttgcagatgg cccaaccacc attagagatg gtaagcacac cctctaattg 4140
   ttttttttaa agattcatag tcacttagtt ctcctctcat ccattctttt ttatcatata 4200
   tagtggctag ctggagagta aaggagacag aaaggatgat tgccatttgc acagagctta 4260
   ggaaggtaaa acatttttct ttctgtctcg ctctcactct cactctcttg gttttatgtg 4320
   ctcagtctaa gttaagttct gcataacttt tgcgtacagc ttggagctac agtggaagag 4380
   ggttcagatt attgtgtgat aactccacca gcaaagctga aaccggcgga gattgacaca 4440
   tatgatgacc atagaatggc aatggcattc tcccttgcag cttgtgctga tgttccagtg 4500
   accatcaaag atcctggttg taccaggaaa actttccctg actacttcca agtccttgaa 4560
   agtatcacaa agcactaaaa aaaccctttt tttttaccac tgcactaaaa agaccttaaa 4620
   gcccatttgt cttttctttt tgatccaatt gagatcagtt tcctctgttg tcactgtaag 4680
   attacgaaaa acaaagagta ttaagattgc ttgcttgtac cttaaactgt ttgatgcaat 4740
   cgttgaatca gttttgggcc 4760
<210> 5
   <211> 361
   <212> PRT
   <213> Brassica napus
<400> 5
   Met Leu Pro Ser Gly Leu Lys Glu Thr Gln Leu Asn Asn Asn Asn Asn
   1               5                   10                  15
   Asn Gln Lys Val His Pro Gln Pro Met Glu Gln Cys Ile Asn Gln Asn
               20                  25                  30
   Pro Glu Ala Met Glu Ala Leu Ile Ser Asn Leu Phe Gly Asn Ile Ser
           35                  40                  45
   Ser Leu Lys Ser Ala Tyr Ile Gln Leu Gln Ser Ala His Thr Pro Tyr
       50                  55                  60
   Asp Pro Asp Lys Ile Gln Glu Ala Asp Lys Ala Val Ile Ser Glu Leu
   65                  70                  75                  80
   Lys Asn Leu Ser Glu Leu Lys His Val Tyr Arg Glu Asn Asn Pro Lys
                   85                  90                  95
   Pro Val Cys Val Ser Pro Lys Asp Ser Arg Leu Ala Ala Glu Ile Gln
               100                 105                 110
   Glu Gln Gln Ser Leu Leu Lys Thr Tyr Glu Val Met Val Lys Lys Phe
           115                 120                 125
   Gln Ser Glu Ile Gln Asn Lys Asp Ser Glu Ile Thr His Met Leu Glu
       130                 135                 140
   Lys Ile Glu Glu Ala Asn Gln Lys Arg Leu Lys Leu Glu Lys Asn Leu
   145                 150                 155                 160
   Lys Leu Arg Gly Met Ser Ser Thr Asn Glu Ser Ser Gly Asp Leu Gln
                   165                 170                 175
   Phe Pro Asp Leu Thr Ile Ala Leu Phe Glu Ser Thr Tyr Glu Ala Ala
               180                 185                 190
   Ser Lys Ala Val His Asp Phe Ser Lys Pro Leu Ile Asn Met Met Lys
           195                 200                 205
   Ala Ala Gly Trp Asp Leu Asp Ser Ala Ala Glu Ser Ile Glu Pro Gly
       210                 215                 220
   Val Ala Tyr Ala Lys Arg Pro His Lys Lys Tyr Ala Phe Glu Ser Tyr
   225                 230                 235                 240
   Ile Cys Gln Arg Met Phe Ser Gly Phe Gln Gln Glu Thr Phe Ser Leu
                   245                 250                 255
   Asp Ser Asp Asn Asp Thr Glu Thr Phe Phe Thr Gln Phe Leu Ala Leu
               260                 265                 270
   Lys Asp Met Asp Pro Leu Asp Ala Leu Ala Thr Asn Pro Asp Ser Asn
           275                 280                 285
   Phe Gly Lys Phe Cys Arg Ser Lys Tyr Leu Ile Leu Ile His Pro Lys
       290                 295                 300
   Met Glu Ala Ser Phe Phe Gly Asn Leu Asp Gln Arg Glu Tyr Val Thr
   305                 310                 315                 320
   Gly Gly Gly His Pro Arg Thr Ala Phe Tyr Gln Ala Phe Leu Lys Leu
                   325                 330                 335
   Ala Lys Ser Ile Trp Leu Leu His Arg Leu Ala Tyr Ser Phe Asp Pro
               340                 345                 350
   Ala Ala Lys Asn Gln Pro Gly Pro Ser
           355                 360
<210> 6
   <211> 1083
   <212> DNA
   <213> Brassica napus
<220>
   <221> misc_feature
   <222> (279) .. (280)
   <223> n is a, c, g, or t
<400> 6
   atgctaccaa gtgggttgaa agaaactcaa ctcaacaaca acaacaacaa ccagaaggtc 60
   catcctcaac caatggaaca atgtatcaac caaaaccctg aagctatgga agcacttatc 120
   tccaacctct ttggaaacat ctcttctcta aaatctgctt acatccagct tcaatctgct 180
   cacactcctt acgaccccga caagattcag gaagccgaca aggctgtgat ctccgaactc 240
   aagaatctct ccgagctgaa acatgtttac agagagaann accccaagcc tgtgtgcgtc 300
   tctcccaaag actctcgtct agccgcagag atccaagagc agcagagtct gttgaagact 360
   tacgaggtca tggtgaagaa gtttcagtct gagattcaga acaaggactc cgagatcact 420
   catatgttgg agaagatcga ggaagctaat cagaaacgtc ttaagctcga gaagaatctt 480
   aagttaagag gaatgtcgtc cactaacgaa tcttccggtg atttgcagtt tcctgacttg 540
   accattgcgc tttttgaatc tacatatgaa gctgcttcta aagctgttca cgatttctca 600
   aagccgttga tcaacatgat gaaagctgcg ggatgggatc ttgattctgc agctgagtcc 660
   attgagcctg gtgttgctta cgccaagagg cctcacaaga agtatgcgtt tgagtcatac 720
   atatgccaga ggatgttcag tgggtttcag caggagactt tctcattaga ctcagataat 780
   gacactgaga ccttcttcac tcagtttctt gctttaaagg atatggatcc actagatgct 840
   ctggctacaa accctgattc caactttggt aagttctgca ggagcaagta tctcatcttg 900
   atccacccaa agatggaagc ttctttcttt ggaaacctag accagcgtga gtacgtgaca 960
   ggaggggggc acccgaggac cgcgttttat caggcctttt tgaagctagc aaagtctata 1020
   tggttattgc acaggcttgc ttactcgttt gatccagctg cgaagaacca gcccgggccg 1080
   tcg 1083
<210> 7
   <211> 1571
   <212> DNA
   <213> Brassica napus
<400> 7
   gcagcagcgt ggagcttatc agatatcttc gcgggggttg aagaagagcg cgatggtgct      60
   aaaccgttct gtaactcgtc cggttaaggt tatggcctct gtttccacgg cggagaaagc     120
   ttcggagatt gtgcttcaac ccattagaga aatctcgggt ctgatcaagc tacccggatc     180
   caaatctctg tccaaccgga ttcttcttct tgccgcttta tccgaggttt gcttctttct     240
   ttgtttgctt agtgttgcgt ttttaacggc gtgaggatga agaaaggttc tgactttgtt     300
   gtggttttat agggaactac tgtagttgac aacttgttga acagtgatga cattaactac     360
   atgcttgatg cgttgaacaa gttggggctt aatgtggaac gtgacagtga gaacaaccgt     420
   gcggttgttg aaggatgtgg cgggatattc ccagcttctt tagattctaa gggtgatatc     480
   gagttgtacc ttgggaatgc aggaacagcc atgcgtccac ttacagctgc agttactgct     540
   gctggtggca acgcaaggta aggttaagga cttattctgt tagttagttt tgattatttt     600
   aagaatcggt cttgtactga tgctttttag ttgggtttgt ttaccagtta tgtgcttgat     660
   ggggtgccta gaatgaggga aagacctata ggagatttgg ttgttggtct taagcagctt     720
   ggtgctgatg ttgaatgtac tcttggtact aactgtcctc ctgttcgtgt caatgctaat     780
   ggtggcctgc ccggtggaaa ggtgagtttg taatttcagc atttgctatg tgaaaagttg     840
   cagcaatctt tgttcatcac actgcgttag cttgacatga ttttagcttt tgtatggttt     900
   cttgattgac acattagaca tgtttttgca tttttcaggt gaagctttct ggatcaatca     960
   gtagtcaata cttgactgca ctgctcatgg cagctccctt agctcttgga gacgttgaga    1020
   ttgagatcat tgataaattg atttctgttc catatgttga aatgacattg aagttgatgg    1080
   aacgttttgg tgttagtgcc gagcatagtg acagttggga tcgtttcttt gtcaagggcg    1140
   gtcagaaata caagtaagag ttgtttctaa aatcactgaa cttataatta gattgacaga    1200
   agagtgacta accaaatggt aaaatttgat tcaggtcgcc tggtaatgct tacgtagaag    1260
   gtgatgcttc tagtgctagt tatttcttgg ctggtgctgc cattactggt gaaaccgtta    1320
   ctgttgaagg ttgtggaaca accagcctgc aggtaacact aagtttataa taaaatttgc    1380
   ttagttcaat ttttttttgt ctttctaagg cttggctagt tgtgtcactt gtgtgtaaca    1440
   tatgaagaat ctaagtttag ttttttttgg tgatgaatct caaagggaga tgtgaagttc    1500
   gctgaggttc ttgagaaaat gggatgtaaa gtgtcatgga cagagaacag tgtgactgtg    1560
   actggaccat c                                                         1571
<210> 8
   <211> 2270
   <212> DNA
   <213> Brassica napus
<400> 8
   atggcgcaag ctagcagaat ctgccatggc gtgcagcaga acccatgtgc tatctccaat      60
   ctctccaaat caaaccaccg caaatctccc ttctctgtct cgctgaagac gcaccagcag     120
   cagcgtggag cttatcagat atcttcgcgg gggttgaaga agagcgcgat ggtgctaaac     180
   cgttctgtaa ctcgtccggt taaggttatg gcctctgttt ccacggcgga gaaagcttcg     240
   gagattgtgc ttcaacccat tagagaaatc tcgggtctga tcaagctacc cggatccaaa     300
   tctctgtcca accggattct tcttcttgcc gctttatccg aggtttgctt ctttctttgt     360
   ttgcttagtg ttgcgttttt aacggcgtga ggatgaagaa aggttctgac tttgttgtgg     420
   ttttataggg aactactgta gttgacaact tgttgaacag tgatgacatt aactacatgc     480
   ttgatgcgtt gaacaagttg gggcttaatg tggaacgtga cagtgagaac aaccgtgcgg     540
   ttgttgaagg atgtggcggg atattcccag cttctttaga ttctaagggt gatatcgagt     600
   tgtaccttgg gaatgcagga acagccatgc gtccacttac agctgcagtt actgctgctg     660
   gtggcaacgc aaggtaaggt taaggactta ttctgttagt tagttttgat tattttaaga     720
   atcggtcttg tactgatgct ttttagttgg gtttgtttac cagttatgtg cttgatgggg      780
   tgcctagaat gagggaaaga cctataggag atttggttgt tggtcttaag cagcttggtg      840
   ctgatgttga atgtactctt ggtactaact gtcctcctgt tcgtgtcaat gctaatggtg      900
   gcctgcccgg tggaaaggtg agtttgtaat ttcagcattt gctatgtgaa aagttgcagc      960
   aatctttgtt catcacactg cgttagcttg acatgatttt agcttttgta tggtttcttg     1020
   attgacacat tagacatgtt tttgcatttt tcaggtgaag ctttctggat caatcagtag     1080
   tcaatacttg actgcactgc tcatggcagc tcccttagct cttggagacg ttgagattga     1140
   gatcattgat aaattgattt ctgttccata tgttgaaatg acattgaagt tgatggaacg     1200
   ttttggtgtt agtgccgagc atagtgacag ttgggatcgt ttctttgtca agggcggtca     1260
   gaaatacaag taagagttgt ttctaaaatc actgaactta taattagatt gacagaagag     1320
   tgactaacca aatggtaaaa tttgattcag gtcgcctggt aatgcttacg tagaaggtga     1380
   tgcttctagt gctagttatt tcttggctgg tgctgccatt actggtgaaa ccgttactgt     1440
   tgaaggttgt ggaacaacca gcctgcaggt aacactaagt ttataataaa atttgcttag     1500
   ttcaattttt ttttgtcttt ctaaggcttg gctagttgtg tcacttgtgt gtaacatatg     1560
   aagaatctaa gtttagtttt ttttggtgat gaatctcaaa gggagatgtg aagttcgctg     1620
   aggttcttga gaaaatggga tgtaaagtgt catggacaga gaacagtgtg actgtgactg     1680
   gaccatctag agatgctttt ggaatgagac acttgcgtgc tgttgatgtc aacatgaaca     1740
   aaatgcctga tgtagccatg actcttgccg ttgtagctct ctttgcagat ggcccaacca     1800
   ccattagaga tggtaagcac accctctaat tgtttttttt aaagattcat agtcacttag     1860
   ttctcctctc atccattctt ttttatcata tatagtggct agctggagag taaaggagac     1920
   agaaaggatg attgccattt gcacagagct taggaaggta aaacattttt ctttctgtct     1980
   cgctctcact ctcactctct tggttttatg tgctcagtct aagttaagtt ctgcataact     2040
   tttgcgtaca gcttggagct acagtggaag agggttcaga ttattgtgtg ataactccac     2100
   cagcaaagct gaaaccggcg gagattgaca catatgatga ccatagaatg gcaatggcat     2160
   tctcccttgc agcttgtgct gatgttccag tgaccatcaa agatcctggt tgtaccagga     2220
   aaactttccc tgactacttc caagtccttg aaagtatcac aaagcactaa                2270
<210> 9
   <211> 521
   <212> PRT
   <213> Brassica napus
<400> 9
   Met Ala Gln Ala Ser Arg Ile Cys His Gly Val Gln Gln Asn Pro Cys
   1               5                   10                  15
   Ala Ile Ser Asn Leu Ser Lys Ser Asn His Arg Lys Ser Pro Phe Ser
               20                  25                  30
   Val Ser Leu Lys Thr His Gln Gln Gln Arg Gly Ala Tyr Gln Ile Ser
           35                  40                  45
   Ser Arg Gly Leu Lys Lys Ser Ala Met Val Leu Asn Arg Ser Val Thr
       50                  55                  60
   Arg Pro Val Lys Val Met Ala Ser Val Ser Thr Ala Glu Lys Ala Ser
   65                  70                  75                  80
   Glu Ile Val Leu Gln Pro Ile Arg Glu Ile Ser Gly Leu Ile Lys Leu
                   85                  90                  95
   Pro Gly Ser Lys Ser Leu Ser Asn Arg Ile Leu Leu Leu Ala Ala Leu
               100                 105                 110
   Ser Glu Gly Thr Thr Val Val Asp Asn Leu Leu Asn Ser Asp Asp Ile
           115                 120                 125
   Asn Tyr Met Leu Asp Ala Leu Asn Lys Leu Gly Leu Asn Val Glu Arg
       130                 135                 140
   Asp Ser Glu Asn Asn Arg Ala Val Val Glu Gly Cys Gly Gly Ile Phe
   145                 150                 155                 160
   Pro Ala Ser Leu Asp Ser Lys Gly Asp Ile Glu Leu Tyr Leu Gly Asn
                   165                 170                 175
   Ala Gly Thr Ala Met Arg Pro Leu Thr Ala Ala Val Thr Ala Ala Gly
               180                 185                 190
   Gly Asn Ala Ser Tyr Val Leu Asp Gly Val Pro Arg Met Arg Glu Arg
           195                 200                 205
   Pro Ile Gly Asp Leu Val Val Gly Leu Lys Gln Leu Gly Ala Asp Val
       210                 215                 220
   Glu Cys Thr Leu Gly Thr Asn Cys Pro Pro Val Arg Val Asn Ala Asn
   225                 230                 235                 240
   Gly Gly Leu Pro Gly Gly Lys Val Lys Leu Ser Gly Ser Ile Ser Ser
                   245                 250                 255
   Gln Tyr Leu Thr Ala Leu Leu Met Ala Ala Pro Leu Ala Leu Gly Asp
               260                 265                 270
   Val Glu Ile Glu Ile Ile Asp Lys Leu Ile Ser Val Pro Tyr Val Glu
           275                 280                 285
   Met Thr Leu Lys Leu Met Glu Arg Phe Gly Val Ser Ala Glu His Ser
       290                 295                 300
   Asp Ser Trp Asp Arg Phe Phe Val Lys Gly Gly Gln Lys Tyr Lys Ser
   305                 310                 315                 320
   Pro Gly Asn Ala Tyr Val Glu Gly Asp Ala Ser Ser Ala Ser Tyr Phe
                   325                 330                 335
   Leu Ala Gly Ala Ala Ile Thr Gly Glu Thr Val Thr Val Glu Gly Cys
               340                 345                 350
   Gly Thr Thr Ser Leu Gln Gly Asp Val Lys Phe Ala Glu Val Leu Glu
           355                 360                 365
   Lys Met Gly Cys Lys Val Ser Trp Thr Glu Asn Ser Val Thr Val Thr
       370                 375                 380
   Gly Pro Ser Arg Asp Ala Phe Gly Met Arg His Leu Arg Ala Val Asp
   385                 390                 395                 400
   Val Asn Met Asn Lys Met Pro Asp Val Ala Met Thr Leu Ala Val Val
                   405                 410                 415
   Ala Leu Phe Ala Asp Gly Pro Thr Thr Ile Arg Asp Val Ala Ser Trp
               420                 425                 430
   Arg Val Lys Glu Thr Glu Arg Met Ile Ala Ile Cys Thr Glu Leu Arg
           435                 440                 445
   Lys Leu Gly Ala Thr Val Glu Glu Gly Ser Asp Tyr Cys Val Ile Thr
       450                 455                 460
   Pro Pro Ala Lys Leu Lys Pro Ala Glu Ile Asp Thr Tyr Asp Asp His
   465                 470                 475                 480
   Arg Met Ala Met Ala Phe Ser Leu Ala Ala Cys Ala Asp Val Pro Val
                   485                 490                 495
   Thr Ile Lys Asp Pro Gly Cys Thr Arg Lys Thr Phe Pro Asp Tyr Phe
               500                 505                 510
   Gln Val Leu Glu Ser Ile Thr Lys His
           515                 520
<210> 10
   <211> 1566
   <212> DNA
   <213> Brassica napus
<400> 10
   atggcgcaag ctagcagaat ctgccatggc gtgcagcaga acccatgtgc tatctccaat      60
   ctctccaaat caaaccaccg caaatctccc ttctctgtct cgctgaagac gcaccagcag     120
   cagcgtggag cttatcagat atcttcgcgg gggttgaaga agagcgcgat ggtgctaaac     180
   cgttctgtaa ctcgtccggt taaggttatg gcctctgttt ccacggcgga gaaagcttcg     240
   gagattgtgc ttcaacccat tagagaaatc tcgggtctga tcaagctacc cggatccaaa     300
   tctctgtcca accggattct tcttcttgcc gctttatccg agggaactac tgtagttgac     360
   aacttgttga acagtgatga cattaactac atgcttgatg cgttgaacaa gttggggctt     420
   aatgtggaac gtgacagtga gaacaaccgt gcggttgttg aaggatgtgg cgggatattc     480
   ccagcttctt tagattctaa gggtgatatc gagttgtacc ttgggaatgc aggaacagcc     540
   atgcgtccac ttacagctgc agttactgct gctggtggca acgcaagtta tgtgcttgat     600
   ggggtgccta gaatgaggga aagacctata ggagatttgg ttgttggtct taagcagctt     660
   ggtgctgatg ttgaatgtac tcttggtact aactgtcctc ctgttcgtgt caatgctaat     720
   ggtggcctgc ccggtggaaa ggtgaagctt tctggatcaa tcagtagtca atacttgact     780
   gcactgctca tggcagctcc cttagctctt ggagacgttg agattgagat cattgataaa     840
   ttgatttctg ttccatatgt tgaaatgaca ttgaagttga tggaacgttt tggtgttagt     900
   gccgagcata gtgacagttg ggatcgtttc tttgtcaagg gcggtcagaa atacaagtcg     960
   cctggtaatg cttacgtaga aggtgatgct tctagtgcta gttatttctt ggctggtgct    1020
   gccattactg gtgaaaccgt tactgttgaa ggttgtggaa caaccagcct gcagggagat    1080
   gtgaagttcg ctgaggttct tgagaaaatg ggatgtaaag tgtcatggac agagaacagt    1140
   gtgactgtga ctggaccatc tagagatgct tttggaatga gacacttgcg tgctgttgat    1200
   gtcaacatga acaaaatgcc tgatgtagcc atgactcttg ccgttgtagc tctctttgca    1260
   gatggcccaa ccaccattag agatgtggct agctggagag taaaggagac agaaaggatg    1320
   attgccattt gcacagagct taggaagctt ggagctacag tggaagaggg ttcagattat    1380
   tgtgtgataa ctccaccagc aaagctgaaa ccggcggaga ttgacacata tgatgaccat    1440
   agaatggcaa tggcattctc ccttgcagct tgtgctgatg ttccagtgac catcaaagat    1500
   cctggttgta ccaggaaaac tttccctgac tacttccaag tccttgaaag tatcacaaag    1560
   cactaa                                                               1566
<210> 11
   <211> 2388
   <212> DNA
   <213> Brassica napus
<220>
   <221> misc_feature
   <222> (1035)..(1035)
   <223> n is a, c, g, or t
<400> 11
   atggcgcaag ctagcagaat ctgccagaac ccatgtgtta tctccaatct ctccaaatca      60
   aaccaacgca aatcgccctt gtctgtctcg atgaagacgc accagatatc ttcgtggggg     120
   ttgaagaaga gtaacaacgg ctctgtgatt cgtccggttc gggtaacggc gtctgtttcc     180
   acggctgaga aatcttcgga gattgtgctt cagcccatta gagaaatctc gggtctgatc     240
   aagctacccg gacccaaatc tctgtccaat cgaatccttc ttctagccgc tctatccgag     300
   gtcggtttgc ttctttcttt ctttgttagc ttagtgttgc gtttttaacg gcgtgagatt     360
   gaagaaaggt tcacactttg ttgtggtgtt atagggaacc actgtagttg acaacttgtt     420
   gaacagtgat gacatcaatt acatgcttga tgcgttgaag aaattggggc ttaatgtgga     480
   acgtgacagt gagaataacc gtgcggttgt tgaaggatgt ggcgggatat tcccagcttc     540
   tttagattcc aagagtgata tcgagttgta ccttgggaat gctggaacag ccatgcgtcc     600
   acttaccgct gcagttactg ctgcaggtgg caacgcaagg taaggttaag gagtgtgatt     660
   ttgttagtta gttttgtgtt atgtcaagaa ccgatcttgt cctcatgctt ttagttcggt     720
   ttattttcca gttatattct tggtggggtg cctagaatga gggaaaggcc tattggagat     780
   ttggttgttg gtcttaagca gcttggtgct gatgttgaat gtactcttgg aactaactgc     840
   cctcctgttc gcgtcaatgc taatggtggc cttcccggtg gaaaggtgag tttgtaatct     900
   cagcatctac tatgtggaaa gttgcaggaa tttttgttca tcacactgcg tttgctcgat     960
   atgatggcct ttgtatggtt tcttgattga catattagat atgatttgca tttttcaggt    1020
   gaagctatct ggttnaatca gtagtcaata cttgactgct ctgctcatgg cagctccttt    1080
   agctcttgga gacgttgaga ttgagatcgt tgataaactg atctctgttc cgtatgttga    1140
   aatgacattg aagttgatgg aacgttttgg tgttagtgcc gagcatagtg acagttggga    1200
   tcgtttcttt gtcaagggcg gtcagaaata caagtaagcg ttgtttctga aatcactgaa    1260
   cttatagtta gattgacaga agagtgacta accaaatggt aaaatttgat tcaggtcgcc    1320
   tggtaatgct tacgtagaag gtgatgcttc tagtgctagt tatttcttgg ctggtgccgc    1380
   cattactggt gagactgtta ctgttgaagg ttgtggaaca accagcctgc aggtaacact    1440
   aagtttataa tgaaatttgc ttagttcaat ttgttttttt gtctttctaa ggctttggct    1500
   agttatgtgt aacatatgtt agaatctaag ctcatttttg ttgttgtgat gaatctcaaa     1560
   gggagatgtg aagttcgctg aggttcttga gaaaatggga tgtaaagtgt catggacaga     1620
   gaacagtgtg actgtgactg gaccatctag agatgctttt ggaatgagac acttgcgcgc     1680
   tgttgatgtc aacatgaaca aaatgcctga tgtagccatg actcttgccg ttgttgctct     1740
   ctttgcagat ggtccaacca ccattagaga tggtaagtac tccctctaac catctaattg     1800
   aggtttttaa gattcatagt cacttagttc tcctctcatc caatcgtttt atcatatata     1860
   gtggctagct ggagagtaaa ggagacagaa aggatgattg ccatttgcac agagcttagg     1920
   aaggtaaaac aattttcttt ctgtcccgct ctcactctct tggttttatg tgctcagtct     1980
   aggttaagtt ctgcataact tttgcgtgca gcttggagct acagtggaag agggttcaga     2040
   ttattgtgtg ataactccac cagcaaagct gaaaccggcg gagattgaca catatgatga     2100
   tcatagaatg gcaatggcat tctcccttgc agcttgtgct gatgttccag taaccatcaa     2160
   agatcctggt tgcaccagga aaactttccc tgactacttc caggtccttg aaagtatcac     2220
   aaagcactaa acagacctta aagcccattt gtcttttctt tttgatccaa ttgggatcag     2280
   tttcctctgt tatcactgta agattacgaa aaacaaagag tattaagatt gtttgcttgt     2340
   accttaaact gtttgatgca atcgttgaat cagttttggg ccaagggc                  2388
<210> 12
   <211> 510
   <212> PRT
   <213> Brassica napus
<220>
   <221> misc_feature
   <222> (239) .. (239)
   <223> Xaa can be any naturally occurring amino acid
<220>
   <221> misc_feature
   <222> (508)..(508)
   <223> Xaa can be any naturally occurring amino acid
<400> 12
   Met Ala Gln Ala Ser Arg Ile Cys Gln Asn Pro Cys Val Ile Ser Asn
   1               5                   10                  15
   Leu Ser Lys Ser Asn Gln Arg Lys Ser Pro Leu Ser Val Ser Met Lys
               20                  25                  30
   Thr His Gln Ile Ser Ser Trp Gly Leu Lys Lys Ser Asn Asn Gly Ser
           35                  40                  45
   Val Ile Arg Pro Val Arg Val Thr Ala Ser Val Ser Thr Ala Glu Lys
       50                  55                  60
   Ser Ser Glu Ile Val Leu Gln Pro Ile Arg Glu Ile Ser Gly Leu Ile
   65                  70                  75                  80
   Lys Leu Pro Gly Pro Lys Ser Leu Ser Asn Arg Ile Leu Leu Leu Ala
                   85                  90                  95
   Ala Leu Ser Glu Gly Thr Thr Val Val Asp Asn Leu Leu Asn Ser Asp
               100                 105                 110
   Asp Ile Asn Tyr Met Leu Asp Ala Leu Lys Lys Leu Gly Leu Asn Val
           115                 120                 125
   Glu Arg Asp Ser Glu Asn Asn Arg Ala Val Val Glu Gly Cys Gly Gly
       130                 135                 140
   Ile Phe Pro Ala Ser Leu Asp Ser Lys Ser Asp Ile Glu Leu Tyr Leu
   145                 150                 155                 160
   Gly Asn Ala Gly Thr Ala Met Arg Pro Leu Thr Ala Ala Val Thr Ala
                   165                 170                 175
   Ala Gly Gly Asn Ala Ser Tyr Ile Leu Gly Gly Val Pro Arg Met Arg
               180                 185                 190
   Glu Arg Pro Ile Gly Asp Leu Val Val Gly Leu Lys Gln Leu Gly Ala
           195                 200                 205
   Asp Val Glu Cys Thr Leu Gly Thr Asn Cys Pro Pro Val Arg Val Asn
       210                 215                 220
   Ala Asn Gly Gly Leu Pro Gly Gly Lys Val Lys Leu Ser Gly Xaa Ile
   225                 230                 235                 240
   Ser Ser Gln Tyr Leu Thr Ala Leu Leu Met Ala Ala Pro Leu Ala Leu
                   245                 250                 255
   Gly Asp Val Glu Ile Glu Ile Val Asp Lys Leu Ile Ser Val Pro Tyr
               260                 265                 270
   Val Glu Met Thr Leu Lys Leu Met Glu Arg Phe Gly Val Ser Ala Glu
           275                 280                 285
   His Ser Asp Ser Trp Asp Arg Phe Phe Val Lys Gly Gly Gln Lys Tyr
       290                 295                 300
   Lys Ser Pro Gly Asn Ala Tyr Val Glu Gly Asp Ala Ser Ser Ala Ser
   305                 310                 315                 320
   Tyr Phe Leu Ala Gly Ala Ala Ile Thr Gly Glu Thr Val Thr Val Glu
                   325                 330                 335
   Gly Cys Gly Thr Thr Ser Leu Gln Gly Asp Val Lys Phe Ala Glu Val
               340                 345                 350
   Leu Glu Lys Met Gly Cys Lys Val Ser Trp Thr Glu Asn Ser Val Thr
           355                 360                 365
   Val Thr Gly Pro Ser Arg Asp Ala Phe Gly Met Arg His Leu Arg Ala
       370                 375                 380
   Val Asp Val Asn Met Asn Lys Met Pro Asp Val Ala Met Thr Leu Ala
   385                 390                 395                 400
   Val Val Ala Leu Phe Ala Asp Gly Pro Thr Thr Ile Arg Asp Val Ala
                   405                 410                 415
   Ser Trp Arg Val Lys Glu Thr Glu Arg Met Ile Ala Ile Cys Thr Glu
               420                 425                 430
   Leu Arg Lys Leu Gly Ala Thr Val Glu Glu Gly Ser Asp Tyr Cys Val
           435                 440                 445
   Ile Thr Pro Pro Ala Lys Leu Lys Pro Ala Glu Ile Asp Thr Tyr Asp
       450                 455                 460
   Asp His Arg Met Ala Met Ala Phe Ser Leu Ala Ala Cys Ala Asp Val
   465                 470                 475                 480
   Pro Val Thr Ile Lys Asp Pro Gly Cys Thr Arg Lys Thr Phe Pro Asp
                   485                 490                 495
   Tyr Phe Gln Val Leu Glu Ser Ile Thr Lys His Xaa Thr Asp
               500                 505                 510
<210> 13
   <211> 1530
   <212> DNA
   <213> Brassica napus
<220>
   <221> misc_feature
   <222> (716)..(716)
   <223> n is a, c, g, or t
<400> 13
   atggcgcaag ctagcagaat ctgccagaac ccatgtgtta tctccaatct ctccaaatca      60
   aaccaacgca aatcgccctt gtctgtctcg atgaagacgc accagatatc ttcgtggggg     120
   ttgaagaaga gtaacaacgg ctctgtgatt cgtccggttc gggtaacggc gtctgtttcc     180
   acggctgaga aatcttcgga gattgtgctt cagcccatta gagaaatctc gggtctgatc     240
   aagctacccg gacccaaatc tctgtccaat cgaatccttc ttctagccgc tctatccgag     300
   ggaaccactg tagttgacaa cttgttgaac agtgatgaca tcaattacat gcttgatgcg     360
   ttgaagaaat tggggcttaa tgtggaacgt gacagtgaga ataaccgtgc ggttgttgaa     420
   ggatgtggcg ggatattccc agcttcttta gattccaaga gtgatatcga gttgtacctt     480
   gggaatgctg gaacagccat gcgtccactt accgctgcag ttactgctgc aggtggcaac     540
   gcaagttata ttcttggtgg ggtgcctaga atgagggaaa ggcctattgg agatttggtt     600
   gttggtctta agcagcttgg tgctgatgtt gaatgtactc ttggaactaa ctgccctcct     660
   gttcgcgtca atgctaatgg tggccttccc ggtggaaagg tgaagctatc tggttnaatc     720
   agtagtcaat acttgactgc tctgctcatg gcagctcctt tagctcttgg agacgttgag     780
   attgagatcg ttgataaact gatctctgtt ccgtatgttg aaatgacatt gaagttgatg     840
   gaacgttttg gtgttagtgc cgagcatagt gacagttggg atcgtttctt tgtcaagggc     900
   ggtcagaaat acaagtcgcc tggtaatgct tacgtagaag gtgatgcttc tagtgctagt     960
   tatttcttgg ctggtgccgc cattactggt gagactgtta ctgttgaagg ttgtggaaca    1020
   accagcctgc agggagatgt gaagttcgct gaggttcttg agaaaatggg atgtaaagtg    1080
   tcatggacag agaacagtgt gactgtgact ggaccatcta gagatgcttt tggaatgaga    1140
   cacttgcgcg ctgttgatgt caacatgaac aaaatgcctg atgtagccat gactcttgcc    1200
   gttgttgctc tctttgcaga tggtccaacc accattagag atgtggctag ctggagagta    1260
   aaggagacag aaaggatgat tgccatttgc acagagctta ggaagcttgg agctacagtg    1320
   gaagagggtt cagattattg tgtgataact ccaccagcaa agctgaaacc ggcggagatt    1380
   gacacatatg atgatcatag aatggcaatg gcattctccc ttgcagcttg tgctgatgtt    1440
   ccagtaacca tcaaagatcc tggttgcacc aggaaaactt tccctgacta cttccaggtc    1500
   cttgaaagta tcacaaagca ctaaacagac                                    1530
<210> 14
   <211> 2834
   <212> DNA
   <213> Brassica napus
<400> 14
   atggcgcaag ctagcagaat ctgccatggc gtgcagaacc catgtgttat catctccaat        60
   ctctccaaat caaaccaaaa caaatcacct ttctccgtct cgctgaagac gcagcagtct       120
   cgagcttctt cgtggggact aaagaagagt ggaacgatgc taaacggttc tgtaattcgc     180
   ccggttaagg taacagcttc cgtttccacg gccgagaaag cttcagagat tgtgcttcaa     240
   ccaattagag aaatctcggg tctcattaag ctacccggat ccaaatctct ctccaatcgg     300
   atcctccttc ttgctgctct atctgaggta catatacttg attagtgtta ggcctttgct     360
   gtgagatttt gggaactata gacaatttag taagaattta tatattattt ttaaaaaatt     420
   aaaagcctat atatatatat atttaaaatt ttcaaaaaat tatggaggtt tgagactgaa     480
   gaaagttttt ttttaattat tattataggg aactactgta gtggacaact tgttgaacag     540
   tgatgacatc aactacatgc ttgatgcgtt gaagaagctg gggcttaacg tggaacgtga     600
   cagggtaaac aaccgtgctg tagttgaagg atgtggtgga atattcccag cttccttaga     660
   ttccaagagt gatattgagt tgtaccttgg gaatgcagga acagccatgc gtccactcac     720
   cgctgccgtt actgctgcag gtggcaacgc aaggtaaagg ttaaggagct ttttgttatt     780
   gtcaagaaat tgattttgtg tttgatgctt ttagtttggt ttgttttcta gttatgtgct     840
   tgatggggtg cctagaatga gggagagacc tataggagat ttggttgttg gtcttaagca     900
   gcttggtgct gatgttgaat gtactctcgg cactaactgt cctcctgttc gtgtcaatgc     960
   taatggtggc cttcccggtg gaaaggtgat cttgtttgca gcagtctttg ttcatcacag    1020
   cctttgcttc acattattac atcttttagt ttgttgttgt gacttgatgg atcttaaaaa    1080
   aaggaattgg gaactggtgt gaaagtgatt agcaatcttt ctcgattcct tgcagggccg    1140
   tgggcattac taagtgaaac attagcctat taacccccaa atattttgaa aaaaatttag    1200
   tatatggccc caaaatagtt tttaagaaat tagaaaaact tttaataaat cgtctacggt    1260
   ccccatttta gagccgaccc tgcttgtatg gtttcttgag tgagatattt tacatgtttt    1320
   gcattttcag gtgaagcttt ctggatcaat cagtagtcaa tacttgactg ccttgctcat    1380
   ggcagctcct ttagctcttg gagacgtgga gattgagatc attgataaac tgatttctgt    1440
   tccatatgtt gaaatgacat tgaagttgat ggaacgtttt ggtgttagtg ccgagcatag    1500
   tgatagctgg gatcgtttct ttgtcaaggg cggtcagaag tacaagtaag aattctttaa    1560
   attaaagaat tagattgaag aaaatgactg attaaccaaa tggcaaaact gattcaggtc    1620
   gcctggtaat gcttatgtag aaggtgatgc ttctagtgct agctacttct tggctggtgc    1680
   tgctattacc ggtgaaaccg tcactgttga aggttgtgga acaactagcc tccaggtagt    1740
   ttctccactc tgaatcatca aatattatac tccctccgtt ttgtattaag tgtcatttta    1800
   gcttttaaat tttgtctcat taaaagtgtc attttacatt ttcaatgtat atattaaata    1860
   aattttccag tttttactaa ttcattatat taaataatat aaaacagaaa atttaacaat    1920
   tatcgtaatt cgtgtgcaaa gttgattagt tcaaagttgt gtgtaacatg ttttgaagaa    1980
   tctaagctca ttctcttttt attttttttg tgatgaatcc caaagggaga tgtgaaattc     2040
   gcagaggtac ttgagaaaat gggatgtaaa gtgtcatgga cagagaacag tgtgactgtg     2100
   actggaccat ctagagatgc ttttggaatg agacacttgc gtgctgttga tgtcaacatg     2160
   aataaaatgc ccgatgtagc catgactctt gccgttgttg ctctctttgc cgatggtcca     2220
   accaccatca gagatggtaa agcaaaaccc tctctttgaa tcagcgtctt ttaaaagatt     2280
   catggttgct ttaactctat ttggtcaatg tagtggctag ctggagagtt aaggagacag     2340
   aaaggatgat agccatctgc acagagcttc gaaaggtaag tttccttttc tctcatgctc     2400
   tcattctaag ttaatcgttg cataactttt tggggttttt tttttgcgtt cagcttggag     2460
   ctacagtgga agaaggttca gattattgtg tgataactcc accagcgaag gtgaaaccgg     2520
   cggagattga tacgtatgat gatcatagaa tggcgatggc gttctcgctt gcagcatgtg     2580
   ctgatgttcc agtcaccatc aaggatcctg gctgcaccag aaagactttc cctgactact     2640
   ttcaagtcct tgaaagtatc acaaagcact aaaaagatca tttcctttga atccaaatgt     2700
   gagaatgtgt ttcttcctct ctctgttgcc actgtaacat ttattagaag aacaaagtgt     2760
   gtgtgtttaa gagtgtgttt gcttgtaatg aactgagtga gatgcaatcg ttgaatcagt     2820
   tttgggccaa gggc                                                       2834
<210> 15
   <211> 516
   <212> PRT
   <213> Brassica napus
<400> 15
   Met Ala Gln Ala Ser Arg Ile Cys His Gly Val Gln Asn Pro Cys Val
   1               5                   10                  15
   Ile Ile Ser Asn Leu Ser Lys Ser Asn Gln Asn Lys Ser Pro Phe Ser
               20                  25                  30
   Val Ser Leu Lys Thr Gln Gln Ser Arg Ala Ser Ser Trp Gly Leu Lys
           35                  40                  45
   Lys Ser Gly Thr Met Leu Asn Gly Ser Val Ile Arg Pro Val Lys Val
       50                  55                  60
   Thr Ala Ser Val Ser Thr Ala Glu Lys Ala Ser Glu Ile Val Leu Gln
   65                  70                  75                  80
   Pro Ile Arg Glu Ile Ser Gly Leu Ile Lys Leu Pro Gly Ser Lys Ser
                   85                  90                  95
   Leu Ser Asn Arg Ile Leu Leu Leu Ala Ala Leu Ser Glu Gly Thr Thr
               100                 105                 110
   Val Val Asp Asn Leu Leu Asn Ser Asp Asp Ile Asn Tyr Met Leu Asp
           115                 120                 125
   Ala Leu Lys Lys Leu Gly Leu Asn Val Glu Arg Asp Arg Val Asn Asn
       130                 135                 140
   Arg Ala Val Val Glu Gly Cys Gly Gly Ile Phe Pro Ala Ser Leu Asp
   145                 150                 155                 160
   Ser Lys Ser Asp Ile Glu Leu Tyr Leu Gly Asn Ala Gly Thr Ala Met
                   165                 170                 175
   Arg Pro Leu Thr Ala Ala Val Thr Ala Ala Gly Gly Asn Ala Ser Tyr
               180                 185                 190
   Val Leu Asp Gly Val Pro Arg Met Arg Glu Arg Pro Ile Gly Asp Leu
           195                 200                 205
   Val Val Gly Leu Lys Gln Leu Gly Ala Asp Val Glu Cys Thr Leu Gly
       210                 215                 220
   Thr Asn Cys Pro Pro Val Arg Val Asn Ala Asn Gly Gly Leu Pro Gly
   225                 230                 235                 240
   Gly Lys Val Lys Leu Ser Gly Ser Ile Ser Ser Gln Tyr Leu Thr Ala
                   245                 250                 255
   Leu Leu Met Ala Ala Pro Leu Ala Leu Gly Asp Val Glu Ile Glu Ile
               260                 265                 270
   Ile Asp Lys Leu Ile Ser Val Pro Tyr Val Glu Met Thr Leu Lys Leu
           275                 280                 285
   Met Glu Arg Phe Gly Val Ser Ala Glu His Ser Asp Ser Trp Asp Arg
       290                 295                 300
   Phe Phe Val Lys Gly Gly Gln Lys Tyr Lys Ser Pro Gly Asn Ala Tyr
   305                 310                 315                 320
   Val Glu Gly Asp Ala Ser Ser Ala Ser Tyr Phe Leu Ala Gly Ala Ala
                   325                 330                 335
   Ile Thr Gly Glu Thr Val Thr Val Glu Gly Cys Gly Thr Thr Ser Leu
               340                 345                 350
   Gln Gly Asp Val Lys Phe Ala Glu Val Leu Glu Lys Met Gly Cys Lys
           355                 360                 365
   Val Ser Trp Thr Glu Asn Ser Val Thr Val Thr Gly Pro Ser Arg Asp
       370                 375                 380
   Ala Phe Gly Met Arg His Leu Arg Ala Val Asp Val Asn Met Asn Lys
   385                 390                 395                 400
   Met Pro Asp Val Ala Met Thr Leu Ala Val Val Ala Leu Phe Ala Asp
                   405                 410                 415
   Gly Pro Thr Thr Ile Arg Asp Val Ala Ser Trp Arg Val Lys Glu Thr
               420                 425                 430
   Glu Arg Met Ile Ala Ile Cys Thr Glu Leu Arg Lys Leu Gly Ala Thr
           435                 440                 445
   Val Glu Glu Gly Ser Asp Tyr Cys Val Ile Thr Pro Pro Ala Lys Val
       450                 455                 460
   Lys Pro Ala Glu Ile Asp Thr Tyr Asp Asp His Arg Met Ala Met Ala
   465                 470                 475                 480
   Phe Ser Leu Ala Ala Cys Ala Asp Val Pro Val Thr Ile Lys Asp Pro
                   485                 490                 495
   Gly Cys Thr Arg Lys Thr Phe Pro Asp Tyr Phe Gln Val Leu Glu Ser
               500                 505                 510
   Ile Thr Lys His
           515
<210> 16
   <211> 1554
   <212> DNA
   <213> Brassica napus
<400> 16
   atggcgcaag ctagcagaat ctgccatggc gtgcagaacc catgtgttat catctccaat 60
   ctctccaaat caaaccaaaa caaatcacct ttctccgtct cgctgaagac gcagcagtct 120
   cgagcttctt cgtggggact aaagaagagt ggaacgatgc taaacggttc tgtaattcgc 180
   ccggttaagg taacagcttc cgtttccacg gccgagaaag cttcagagat tgtgcttcaa 240
   ccaattagag aaatctcggg tctcattaag ctacccggat ccaaatctct ctccaatcgg 300
   atcctccttc ttgctgctct atctgaggga actactgtag tggacaactt gttgaacagt 360
   gatgacatca actacatgct tgatgcgttg aagaagctgg ggcttaacgt ggaacgtgac 420
   agggtaaaca accgtgctgt agttgaagga tgtggtggaa tattcccagc ttccttagat 480
   tccaagagtg atattgagtt gtaccttggg aatgcaggaa cagccatgcg tccactcacc 540
   gctgccgtta ctgctgcagg tggcaacgca agttatgtgc ttgatggggt gcctagaatg 600
   agggagagac ctataggaga tttggttgtt ggtcttaagc agcttggtgc tgatgttgaa 660
   tgtactctcg gcactaactg tcctcctgtt cgtgtcaatg ctaatggtgg ccttcccggt 720
   ggaaaggtga agctttctgg atcaatcagt agtcaatact tgactgcctt gctcatggca 780
   gctcctttag ctcttggaga cgtggagatt gagatcattg ataaactgat ttctgttcca 840
   tatgttgaaa tgacattgaa gttgatggaa cgttttggtg ttagtgccga gcatagtgat 900
   agctgggatc gtttctttgt caagggcggt cagaagtaca agtcgcctgg taatgcttat 960
   gtagaaggtg atgcttctag tgctagctac ttcttggctg gtgctgctat taccggtgaa 1020
   accgtcactg ttgaaggttg tggaacaact agcctccagg gagatgtgaa attcgcagag 1080
   gtacttgaga aaatgggatg taaagtgtca tggacagaga acagtgtgac tgtgactgga 1140
   ccatctagag atgcttttgg aatgagacac ttgcgtgctg ttgatgtcaa catgaataaa 1200
   atgcccgatg tagccatgac tcttgccgtt gttgctctct ttgccgatgg tccaaccacc 1260
   atcagagatg tggctagctg gagagttaag gagacagaaa ggatgatagc catctgcaca 1320
   gagcttcgaa agcttggagc tacagtggaa gaaggttcag attattgtgt gataactcca 1380
   ccagcgaagg tgaaaccggc ggagattgat acgtatgatg atcatagaat ggcgatggcg 1440
   ttctcgcttg cagcatgtgc tgatgttcca gtcaccatca aggatcctgg ctgcaccaga 1500
   aagactttcc ctgactactt tcaagtcctt gaaagtatca caaagcacta aaaa 1554
<210> 17
   <211> 2958
   <212> DNA
   <213> Brassica napus
<400> 17
   atggcgcaag ctagcagaat ctgccatggc gtgcagaacc catgtgttat catctccaat      60
   ctctccaaat ccaaccaaaa caaatcacct ttctccgtct ccttgaagac gcatcagcct     120
   cgagcttctt cgtggggatt gaagaagagt ggaacgatgc taaacggttc tgtaattcgc     180
   ccggttaagg taacagcttc tgtttccacg tccgagaaag cttcagagat tgtgcttcaa     240
   ccaatcagag aaatctcggg tctcattaag ctacccggat ccaaatctct ctccaatcgg     300
   atcctccttc ttgccgctct atctgaggta catatacttg cttagtgtta ggcctttgct     360
   gtgagatttt gggaactata gacaatttag taagaattta tatataattt ttttaaaaaa     420
   aatcagaagc ctatatatat ttaaattttt ccaaaatttt tggaggttat aggcttatgt     480
   tacaccattc tagtctgcat ctttcggttt gagactgaag aattttattt tttaaaaaat     540
   tattataggg aactactgta gtggacaact tgttgaacag tgatgacatc aactacatgc      600
   ttgatgcgtt gaagaagctg gggcttaacg tggaacgtga cagtgtaaac aaccgtgcgg      660
   ttgttgaagg atgcggtgga atattcccag cttccttaga ttccaagagt gatattgagt      720
   tgtaccttgg gaatgcagga acagccatgc gtccactcac cgctgcagtt acagctgcag      780
   gtggcaacgc gaggtaaggt taacgagttt tttgttattg tcaagaaatt gatcttgtgt      840
   ttgatgcttt tagtttggtt tgttttctag tgatgtactt gatggggtgc ctagaatgag      900
   ggaaagacct ataggagatt tggttgttgg tcttaagcag cttggtgctg atgttgagtg      960
   tactcttggc actaactgtc ctcctgttcg tgtcaatgct aatggtggcc ttcccggtgg     1020
   gaaggtgatc ttcacattta ctctatgaat tgtttgcagc agtctttgtt catcacagcc     1080
   tttgcttcac attatttcat cttttagttt gttgttatat tacttgatgg atctttaaaa     1140
   aggaattggg tctggtgtga aagtgattag caatctttct cgattccttg cagggccgtg     1200
   ggcattacta agtgaaacat tagcctatta acccccaaaa tttttgaaaa aaatttagta     1260
   tatggcccca aaatagtttt taaaaaatta gaaaaacttt taataaatcg tctacagtcc     1320
   caaaaatctt agagccggcc ctgcttgtat ggtttctcga ttgatatatt agactatgtt     1380
   ttgaattttc aggtgaagct tcctggatcg atcagtagtc agtacttgac tgccctcctc     1440
   atggcagctc ctttagctct tggagacgtg gagattgaga tcattgataa actgatatct     1500
   gttccatatg ttgaaatgac attgaagttg atggagcgtt ttggtgttag tgccgagcat     1560
   agtgatagct gggatcgttt ctttgtcaag ggcggtcaga aatacaagta atgagttctt     1620
   ttaagttgag agttagattg aagaatgaat gactgattaa ccaaatggca aaactgattc     1680
   aggtcgcctg gtaatgctta tgtagaaggt gatgcttcta gtgctagcta cttcttggct     1740
   ggtgctgcca ttactggtga aactgttact gtcgaaggtt gtggaacaac tagcctccag     1800
   gtagtttatc cactctgaat catcaaatat tatactccct ccgttttatg ttaagtgtca     1860
   ttagctttta aattttgttt cattaaaagt gtcattttac attttcaatg catatattaa     1920
   ataaattttc cagtttttac taattcatta attagcaaaa tcaaacaaaa attatattaa     1980
   ataatgtaaa attcgtaatt tgtgtgcaaa taccttaaac cttatgaaac ggaaacctta     2040
   tgaaacagag ggagtactaa ttttataata aaatttgatt agttcaaagt tgtgtataac     2100
   atgttctgta agaatctaag ctcattctct ttttattttt tgtgatgaat cccaaaggga     2160
   gatgtgaaat tcgcagaggt tcttgagaaa atgggatgta aggtgtcatg gacagagaac     2220
   agtgtgactg tgactggacc atcaagagat gcttttggaa tgaggcactt gcgtgctgtt     2280
   gatgtcaaca tgaacaaaat gcctgatgta gccatgactc tagccgttgt tgctctcttt     2340
   gccgatggtc caaccaccat cagagatggt aaagcaaaac cctctctttg aatcagcgtg     2400
   ttttaaaaga ttcatggttg cttaaactct atttggtcaa tgtagtggct agctggagag 2460
   ttaaggagac agagaggatg attgccattt gcacagagct tagaaaggta agtttccttt 2520
   tctctcatgc tctctcattc gaagttaatc gttgcataac tttttgcggt tttttttttt 2580
   gcgttcagct tggagctaca gtggaagaag gttcagatta ttgtgtgata actccaccag 2640
   caaaggtgaa accggcggag attgatacgt atgatgatca tagaatggcg atggcgttct 2700
   cgcttgcagc ttgtgctgat gttccagtca ccatcaagga tcctggctgc accaggaaga 2760
   ctttccctga ctacttccaa gtccttgaaa gtatcacaaa gcattaaaag accctttcct 2820
   ctgatccaaa tgtgagaatc tgttgctttc tctttgttgc caccgtaaca tttattagaa 2880
   gaacaaagtg tgtgtgttaa gagtgtgttt gcttgtaatg aactgagtga gatgcaatcg 2940
   ttgaatcagt tttgggcc 2958
<210> 18
   <211> 515
   <212> PRT
   <213> Brassica napus
<400> 18
   Met Ala Gln Ser Ser Arg Ile Cys His Gly Val Gln Asn Pro Cys Val
   1 5 10 15
   Ile Ile Ser Asn Leu Ser Lys Ser Asn Gln Asn Lys Ser Pro Phe Ser
   20 25 30
   Val Ser Leu Lys Thr His Gln Pro Arg Ala Ser Ser Trp Gly Leu Lys
   35 40 45
   Lys Ser Gly Thr Met Leu Asn Gly Ser Val Ile Arg Pro Val Lys Val
   50 55 60
   Thr Ala Ser Val Ser Thr Ser Glu Lys Ala Ser Glu Ile Val Leu Gln
   65 70 75 80
   Pro Ile Arg Glu Ile Ser Gly Leu Ile Lys Leu Pro Gly Ser Lys Ser
   85 90 95
   Leu Ser Asn Arg Ile Leu Leu Leu Ala Ala Leu Ser Glu Gly Thr Thr
   100 105 110
   Val Val Asp Asn Leu Leu Asn Ser Asp Asp Ile Asn Tyr Met Leu Asp
   115 120 125
   Ala Leu Lys Lys Leu Gly Leu Asn Val Glu Arg Asp Ser Val Asn Asn
   130 135 140
   Arg Ala Val Val Glu Gly Cys Gly Gly Ile Phe Pro Ala Ser Leu Asp
   145                 150                 155                 160
   Ser Lys Ser Asp Ile Glu Leu Tyr Leu Gly Asn Ala Gly Thr Ala Met
                   165                 170                 175
   Arg Pro Leu Thr Ala Ala Val Thr Ala Ala Gly Gly Asn Ala Ser Tyr
               180                 185                 190
   Val Leu Asp Gly Val Pro Arg Met Arg Glu Arg Pro Ile Gly Asp Leu
           195                 200                 205
   Val Val Gly Leu Lys Gln Leu Gly Ala Asp Val Glu Cys Thr Leu Gly
       210                 215                 220
   Thr Asn Cys Pro Pro Val Arg Val Asn Ala Asn Gly Gly Leu Pro Gly
   225                 230                 235                 240
   Gly Lys Val Lys Leu Ser Gly Ser Ile Ser Ser Gln Tyr Leu Thr Ala
                   245                 250                 255
   Leu Leu Met Ala Ala Pro Leu Ala Leu Gly Asp Val Glu Ile Glu Ile
               260                 265                 270
   Ile Asp Lys Leu Ile Ser Val Pro Tyr Val Glu Met Thr Leu Lys Leu
           275                 280                 285
   Met Glu Arg Phe Gly Val Ser Ala Glu His Ser Asp Ser Trp Asp Arg
       290                 295                 300
   Phe Phe Val Lys Gly Gly Gln Lys Tyr Lys Ser Pro Gly Asn Ala Tyr
   305                 310                 315                 320
   Val Glu Gly Asp Ala Ser Ser Ala Ser Tyr Phe Leu Ala Gly Ala Ala
                   325                 330                 335
   Ile Thr Gly Glu Thr Val Thr Val Glu Gly Cys Gly Thr Thr Ser Leu
               340                 345                 350
   Gln Gly Asp Val Lys Phe Ala Glu Val Leu Glu Lys Met Gly Cys Lys
           355                 360                 365
   Val Ser Trp Thr Glu Asn Ser Val Thr Val Thr Gly Pro Ser Arg Asp
       370                 375                 380
   Ala Phe Gly Met Arg His Leu Arg Ala Val Asp Val Asn Met Asn Lys
   385                 390                 395                 400
   Met Pro Asp Val Ala Met Thr Leu Ala Val Val Ala Leu Phe Ala Asp
                   405                 410                 415
   Gly Pro Thr Thr Ile Arg Asp Val Ala Ser Trp Arg Val Lys Glu Thr
               420                 425                 430
   Glu Arg Met Ile Ala Ile Cys Thr Glu Leu Arg Lys Leu Gly Ala Thr
           435                 440                 445
   Val Glu Glu Gly Ser Asp Tyr Cys Val Ile Thr Pro Pro Ala Lys Val
       450                 455                 460
   Lys Pro Ala Glu Ile Asp Thr Tyr Asp Asp His Arg Met Ala Met Ala
   465                 470                 475                 480
   Phe Ser Leu Ala Ala Cys Ala Asp Val Pro Val Thr Ile Lys Asp Pro
                   485                 490                 495
   Gly Cys Thr Arg Lys Thr Phe Pro Asp Tyr Phe Gln Val Leu Glu Ser
               500                 505                 510
   Ile Thr Lys
           515
<210> 19
   <211> 1547
   <212> DNA
   <213> Brassica napus
<400> 19
   atggcgcaat ctagcagaat ctgccatggc gtgcagaacc catgtgttat catctccaat 60
   ctctccaaat ccaaccaaaa caaatcacct ttctccgtct ccttgaagac gcatcagcct 120
   cgagcttctt cgtggggatt gaagaagagt ggaacgatgc taaacggttc tgtaattcgc 180
   ccggttaagg taacagcttc tgtttccacg tccgagaaag cttcagagat tgtgcttcaa 240
   ccaatcagag aaatctcggg tctcattaag ctacccggat ccaaatctct ctccaatcgg 300
   atcctccttc ttgccgctct atctgaggga actactgtag tggacaactt gttgaacagt 360
   gatgacatca actacatgct tgatgcgttg aagaagctgg ggcttaacgt ggaacgtgac 420
   agtgtaaaca accgtgcggt tgttgaagga tgcggtggaa tattcccagc ttccttagat 480
   tccaagagtg atattgagtt gtaccttggg aatgcaggaa cagccatgcg tccactcacc 540
   gctgcagtta cagctgcagg tggcaacgcg agttatgtac ttgatggggt gcctagaatg 600
   agggaaagac ctataggaga tttggttgtt ggtcttaagc agcttggtgc tgatgttgag 660
   tgtactcttg gcactaactg tcctcctgtt cgtgtcaatg ctaatggtgg ccttcccggt 720
   ggaaaggtga agctttctgg atcgatcagt agtcagtact tgactgccct cctcatggca 780
   gctcctttag ctcttggaga cgtggagatt gagatcattg ataaactgat atctgttcca 840
   tatgttgaaa tgacattgaa gttgatggag cgttttggtg ttagtgccga gcatagtgat 900
   agctgggatc gtttctttgt caagggcggt cagaaataca agtcgcctgg taatgcttat 960
   gtagaaggtg atgcttctag tgctagctac ttcttggctg gtgctgccat tactggtgaa 1020
   actgttactg tcgaaggttg tggaacaact agcctccagg gagatgtgaa attcgcagag 1080
   gttcttgaga aaatgggatg taaagtgtca tggacagaga acagtgtgac tgtgactgga 1140
   ccatcaagag atgcttttgg aatgaggcac ttgcgtgctg ttgatgtcaa catgaacaaa 1200
   atgcctgatg tagccatgac tctagccgtt gttgctctct ttgccgatgg tccaaccacc 1260
   atcagagatg tggctagctg gagagttaag gagacagaga ggatgattgc catttgcaca 1320
   gagcttagaa agcttggagc tacagtggaa gaaggttcag attattgtgt gataactcca 1380
   ccagcaaagg tgaaaccggc ggagattgat acgtatgatg atcatagaat ggcgatggcg 1440
   ttctcgcttg cagcttgtgc tgatgttcca gtcaccatca aggatcctgg ctgcaccagg 1500
   aagactttcc ctgactactt ccaagtcctt gaaagtatca caaagca 1547
<210> 20
   <211> 4888
   <212> DNA
   <213> Artificial Sequence
<220>
   <223> Expression cassette from pDAB100331
<400> 20
   gagcataatt tttattaatg tactaaatta ctgttttgtt aaatgcaatt ttgctttctc      60
   gggattttaa tatcaaaatc tatttagaaa tacacaatat tttgttgcag gcttgctgga     120
   gaatcgatct gctatcataa aaattacaaa aaaattttat ttgcctcaat tattttagga     180
   ttggtattaa ggacgcttaa attatttgtc gggtcactac gcatcattgt gattgagaag     240
   atcagcgata cgaaatattc gtagtactat cgcgataatt tatttgaaaa ttcatatgaa     300
   aagcaaacgt tacatgaatt gatgaaacaa tacaaagaca gataaagcca cgcacattta     360
   ggatattggc cgagattact gaatattgag taagatcacg gaatttctga caggagcatg     420
   tcttcaattc agcccaaatg gcagttgaaa tactcaaacc gccccatatg caggagcgga     480
   tcattcattg tttgtttggt tgcctttgcc aacatgggag tccaaggttt ggtgacctcg     540
   aggcttaaga ctttactaaa acttcaaaag aaaaacaata taaaaacgat aatccaaatg     600
   cattattgat ctatataaca tcaagacaaa aatacatatg tgactcttat tcaggtctta     660
   ggtttattac agcaaagatc atgacttgat cacttcaaac aaagtacgta actataaaaa     720
   cgagtcaaat agattgtctt acactaacgt gtcgatagaa taatttgacc aaaaggtgat     780
   cttattacag aaatagccac tgagctcggt agcaattccc gaggctgtag ccgacgatgg     840
   tgcgccagga gagttgttga ttcattgttt gcctccctgc tgcggttttt caccgaagtt     900
   catgccagtc cagcgttttt gcagcagaaa agccgccgac ttcggtttgc ggtcgcgagt     960
   gaagatccct ttcttgttac cgccaacgcg caatatgcct tgcgaggtcg caaaatcggc    1020
   gaaattccat acctgttcac cgacgacggc gctgacgcga tcaaagacgc ggtgatacat    1080
   atccagccat gcacactgat actcttcact ccacatgtcg gtgtacattg agtgcagccc    1140
   ggctaacgta tccacgccgt attcggtgat gataatcggc tgatgcagtt tctcctgcca    1200
   ggccagaagt tctttttcca gtaccttctc tgccgtttcc aaatcgccgc tttggacata    1260
   ccatccgtaa taacggttca ggcacagcac atcaaagaga tcgctgatgg tatcggtgtg    1320
   agcgtcgcag aacattacat tgacgcaggt gatcggacgc gtcgggtcga gtttacgcgt    1380
   tgcttccgcc agtggcgcga aatattcccg tgcaccttgc ggacgggtat ccggttcgtt    1440
   ggcaatactc cacatcacca cgcttgggtg gtttttgtca cgcgctatca gctctttaat    1500
   cgcctgtaag tgcgcttgct gagtttcccc gttgactgcc tcttcgctgt acagttcttt    1560
   cggcttgttg cccgcttcga aaccaatgcc taaagagagg ttaaagccga cagcagcagt    1620
   ttcatcaatc accacgatgc catgttcatc tgcccagtcg agcatctctt cagcgtaagg    1680
   gtaatgcgag gtacggtagg agttggcccc aatccagtcc attaatgcgt ggtcgtgcac    1740
   catcagcacg ttatcgaatc ctttgccacg caagtccgca tcttcatgac gaccaaagcc    1800
   agtaaagtag aacggtttgt ggttaatcag gaactgttcg cccttcactg ccactgaccg    1860
   gatgccgacg cgaagcgggt agatatcaca ctctgtctgg cttttggctg tgacgcacag    1920
   ttcatagaga taaccttcac ccggttgcca gaggtgcgga ttcaccactt gcaaagtccc    1980
   gctagtgcct tgtccagttg caaccacctg ttgatccgca tcacgcagtt caacgctgac    2040
   atcaccattg gccaccacct gccagtcaac agacgcgtgg ttacagtctt gcgcgacatg    2100
   cgtcaccacg gtgatatcgt ccacccaggt gttcggcgtg gtgtagagca ttacgctgcg    2160
   atggattccg gcatagttaa agaaatcatg gaagtaagac tgctttttct tgccgttttc    2220
   gtcggtaatc accattcccg gcgggatagt ctgccagttc agttcgttgt tcacacaaac    2280
   ggtgatacgt acacttttcc cggcaataac atacggcgtg acatcggctt caaatggcgt    2340
   atagccgccc tgatgctcca tcacttcctg attattgacc cacactttgc cgtaatgagt    2400
   gaccgcatcg aaacgcagca cgatacgctg gcctgcccaa cctttcggta taaagacttc    2460
   gcgctgatac cagacgttgc ccgcataatt acgaatatct gcatcggcga actgatcgtt    2520
   aaaactgcct ggcacagcaa ttgcccggct ttcttgtaac gcgctttccc accaacgctg    2580
   atcaattcca cagttttcgc gatccagact gaatgcccac aggccgtcga gttttttgat    2640
   ttcacgggtt ggggtttcta caggacggac catggagaaa aagtttcgat ctttggactc 2700
   gctcacctaa acccactctc tgtcttcctc agatttaata aactttagag ggggtttggt 2760
   gacgaagaag gtagccatca aaatccttca ccaacccacc tcaaacgatg gtgtatgagc 2820
   atttcctatt agccaatgag aacctgccac gtgttacaga cagcaagtgg gaaggaaggt 2880
   tttggttagg tgaagacttg aaagggttaa gatttggtgg ttttaaaatg ggcctccaag 2940
   aagttactgg gcctttttaa cacaaaaacc catgaaccaa tctaactcat aaataatcga 3000
   ttgcggttta ctactagaat attggagagg aaacataaat cgaagagtaa ccggccagat 3060
   tacctttaat cgaagacgat ggtaacatcc atgtctggat ccgatccaaa aaatcgggtt 3120
   ctaggtcgga tctagatatg gtgaaatatc cgattgggta tgctttcatt tatgtttcgg 3180
   gtaccggttc ggatcggata ttatccaaga ttcaaacata tccgaaaata cccgacagga 3240
   atgtataagc ttatcggaaa ataatctcaa tctggtttag agtttggaac ataatcaaac 3300
   cgggggatgt ctttttccag ttatatatgt cacaaacccc aaacaatcaa accgatctga 3360
   ggttcggttt aggataaacc acggcaattc ggaactctct ctcaatttgg tttagaatcc 3420
   gagcaagtat tgagcttaac catttctttt tcgttcactt cctacgaaag catattcctt 3480
   tctctctgta tctccttcac ctccgggcta aaaaaggatc cggcttagag ctgcttgatg 3540
   tttcgttttc ttctacttac gtggcgtgaa agctgatcaa aaacattatt cgacgcgaag 3600
   cttcttcctg tctgaatagt gctgtataca cagacacaaa gctaaatcat tccaggtgat 3660
   ctcttttgtc ttccatctga aactctagat tcagatcacc actatactcg agcctcattc 3720
   gaaaaagctg tgaactttat aggggaacta acactgtaga cactagaaag atctttacgg 3780
   gtttgatgat gatgatgatt cacttcaaga tctagaatat tagttgggtt tttgtctgaa 3840
   aaggaacatt ctttgtagga gggttttgtc tgaaacagag agattcagtt ttcaagtcca 3900
   tggagtccga tgagagtggt ctcccagcta tggagattga atgcagaatc actggcactt 3960
   tgaacggtgt tgagtttgaa ctggtgggag gtggcgaagg gacacctgaa caagggagga 4020
   tgacaaacaa gatgaagtcc accaaaggtg cattgacctt ctctccgtat cttctcagcc 4080
   atgtcatggg ttacggtttc tatcactttg gcacctatcc gagtggctat gagaatccct 4140
   ttcttcatgc catcaacaat ggaggttaca ccaacacacg aattgagaag tatgaagatg 4200
   gtggagtgct ccacgtctcc ttctcttacc gttacgaggc tgggagggtc ataggagact 4260
   tcaaagtgat gggaactggc tttccagaag attcagtcat cttcacagac aagatcatta 4320
   gatccaatgc aactgttgag catcttcacc caatgggaga caatgacctg gatgggtcat 4380
   tcacaagaac cttctctctg cgtgatggag gctactatag ctctgttgtg gactcacaca 4440
   tgcacttcaa aagtgccatt catcctagca tcttgcagaa tggtggaccc atgtttgcct 4500
   ttcgaagggt ggaagaggat cactcaaaca ccgaacttgg catagttgag taccagcatg      4560
   ccttcaagac tcctgatgca gatgctgggg aagagtgagt agttagctta atcacctaga      4620
   gctcgaattt ccccgatcgt tcaaacattt ggcaataaag tttcttaaga ttgaatcctg      4680
   ttgccggtct tgcgatgatt atcatataat ttctgttgaa ttacgttaag catgtaataa      4740
   ttaacatgta atgcatgacg ttatttatga gatgggtttt tatgattaga gtcccgcaat      4800
   tatacattta atacgcgata gaaaacaaaa tatagcgcgc aaactaggat aaattatcgc      4860
   gcgcggtgtc atctatgtta ctagatcg                                          4888
<210> 21
   <211> 4888
   <212> DNA
   <213> Artificial Sequence
<220>
   <223> Expression cassette from pDAB100333
<400> 21
<210> 22
   <211> 739
   <212> DNA
   <213> Artificial Sequence
<220>
   <223> alternative BBCP sequence 739 nt
<400> 22
   acagagagaa agtaatatgc tttagtgcga agtgaacgaa aaagaaatgg ttaagctcaa      60
   tacttactcg gattctaaac caaattgaga gataattccg acttgccgtg gtttatcgta     120
   aaccgaacct cagatcggta taattgtttg gtgtgtgtga catatataac tggaaaaaga     180
   catcccgcgg tttcattatg ttccaaactc taaacgagat tgagattatt ttcggataag     240
   cttatacatt cctgccgggt attttcgtat atgtctgatt cgtggataat atccgatccg     300
   aaccggtacc cgaatcataa ctgaaagcat acccaatccg atagttcacc agctctagat     360
   cggaccgaga acccgatttt ttggatagga tccagacttg gaggtcacca tcgtcttcga     420
   ttaaaggaaa tctggtcggt tactcctcga tttctgtttc ctctccaata ttccagtagt     480
   aaaccgcgat caattattta tgagttggat tggttcatgg gtttttgtcg taaaaaggcc     540
   cactatcttc ttggaggccc attttaaaac caccaaatct taaccctttc aagtcttcac     600
   ctaaccaaaa ccttcctacc cacttgctgt ctgtagcacg cggcaggttc tcattggcta     660
   ataggaaatg ctcatacacc atcgtttgag gtgggttggt taaggatttt gatggctacc     720
   ttcttcgtca ccaaccccc                                                 739
<210> 23
   <211> 739
   <212> DNA
   <213> Artificial Sequence
<220>
   <223> additional alternative BBCP sequence 739 nt
<400> 23
   acagagagaa aggaatatgc tttcgtagga agtgaacgaa aaagacatgg ttaagctcaa      60
   tacatgctgg gattctaaac caaattgaga gagagttccg aattgccgtg gtttatccta     120
   aaccggacct cgaatcggtt tgattgtttg ggatttgtga caaatctaac tggaaaaaga     180
   catcccccgg tttgattatg ttccaaactc tgaaccagat tgagagtatt ttccgataag     240
   cttatacatt cctgtcgggt attttgggat atttttgaat cttggataat atccgatccg     300
   aaccggtccc cgagacttaa atgaaagcat accaactcgg attttgcacc atatctagat     360
   ccgtcctaga acacgatttt ttggatcgga tccagacaag gatgtaacca tcttcttcga     420
   ttaaaggtaa tctgaccggt tactctccga tttacgtttc ctctcgaata ttctcgtagt     480
   aaaccgcaat cgattattga tgagttagat tggttcatgg gtctttgtgt taaaaaggcc     540
   cagtaacttc ttggaggccc aatttaaaac cacaaaatct taaccatttc aagtcttaac     600
   caaagcataa ccttccttcc cacttgctgt ttgtaacacg tgtcaggtgc tcatggagta     660
   ataggaaatg ctcatacacc atcgtttgag gagggttggt gaacgatttt gatggctacc     720
   ttcttcgtca ccaaacccc                                                  739
<210> 24
   <211> 739
   <212> DNA
   <213> Artificial Sequence
<220>
   <223> Additional alternative BBCP sequence 739 nt
<400> 24
   acagagagaa aggaatatgc tttcgtagga agcgagcgaa aaagaaatgg ttaagctcta 60
   tagttgctcg gattctaaac caaattgaga gagtgttccg tattgccgtg gtttattcta 120
   aaccgaacct cagatcggtt tgattgtttg gggtttgtca catatctaac tggaaaaaga 180
   catgccccgg tttgattagg ttctaaactc ttaacgagat tgagattatt gtccgataag 240
   cttatacatt cctttcgagt attttcggat ttatttgaat cttggataat atccgatccg 300
   aaccggaacc cggaaaataa atgaaagcat acccaatcgg atacttcacc atatctagat 360
   cccccctaga acccgatttt ctggctcgga tccagacatg gatgtaatca tcgtcttcga 420
   ttaaatgtaa tctggctggt tactcttcga tttatgttcc ctttccaata ttctaatcgt 480
   aaaccgcaat cgattatcta tgagttagat tgcttcatgg gtttttgtgt taaataggcc 540
   cagtaactgc ttggaggccc attttaaaac caccaaatct taaccctttc acttcttcac 600
   ctaaccaaaa ccttccttcc aacttgctgt ctgtaacacg tggcaggttc tcagtggcaa 660
   atgggaaatg atcatacacc agcgtttgag gtgggttggt gaaggatttt gatggctacc 720
   tgcttcgtca ccatacccc 739
<210> 25
   <211> 739
   <212> DNA
   <213> Artificial Sequence
<220>
   <223> alternative BBCP sequence 739 nt
<400> 25
   acagagaaaa aggaatatgc tttcgcagga agtgaacgaa aaagaaaggg ttaagctcac 60
   tacttgctcg ttttctaaac caaattgaga gagagttccg aattgccgtg gtttctccga 120
   aaccgaagct cagatcggtt tgcttgtttg gggtttgtga catatatatc tggaaaaaga 180
   gatcccccgg ttggattatg ttccaaactc taaaccagat tgagattatt ttccgataag 240
   ctcatacatt cctgtcgtgt atgttcggat atgtttgaat cttggataat atccgatccg 300
   aaccggttcc cgaaacataa ataaaagcat acccaatcgg atatttcacc atgtcgagat 360
   caggcctaga acccgatgtt atggatcgaa tccagacatg gattttgcca tcgtcttcga 420
   ttcaatgtaa tctggccggt tactcttcga tttatgtttc ctctccaatc tactagtagc 480
   aaaccgcgat cgatggttta tgagttagat tggttcgtgg gttattgtgt taaacaggcc 540
   cagtaacttc ttggaggccc attgtaaaac caccaaatct taacccattc aagtcttccc 600
   ctaaccaaaa ccttccgtcc cacttgctga cggtaacacg tggcaggttt tcattggcta 660
   atagtagatg ctcatacacc atcgtttgag gtgggttggt gaaggattct gatgcctacc 720
   ttcttcgtca ccaaacccc 739
<210> 26
   <211> 179
   <212> DNA
   <213> Brassica napus
<400> 26
   aaaaaccctt tttttttacc actgcactaa aaagacctta aagcccattt gtcttttctt 60
   tttgatccaa ttgagatcag tttcctctgt tgtcactgta agattacgaa aaacaaagag 120
   tattaagatt gcttgcttgt accttaaact gtttgatgca atcgttgaat cagttttgg 179
<210> 27
   <211> 90
   <212> DNA
   <213> Brassica napus
<400> 27
   aggtttgctt ctttctttgt ttgcttagtg ttgcgttttt aacggcgtga ggatgaagaa 60
   aggttctgac tttgttgtgg ttttataggg 90
<210> 28
   <211> 94
   <212> DNA
   <213> Brassica napus
<400> 28
   aggtaaggtt aaggacttat tctgttagtt agttttgatt attttaagaa tcggtcttgt 60
   actgatgctt tttagttggg tttgtttacc agtt 94
<210> 29
   <211> 141
   <212> DNA
   <213> Brassica napus
<400> 29
   gagtttgtaa tttcagcatt tgctatgtga aaagttgcag caatctttgt tcatcacact 60
   gcgttagctt gacatgattt tagcttttgt atggtttctt gattgacaca ttagacatgt 120
   ttttgcattt ttcaggtgaa g 141
<210> 30
   <211> 85
   <212> DNA
   <213> Brassica napus
<400> 30
   aagtaagagt tgtttctaaa atcactgaac ttataattag attgacagaa gagtgactaa 60
   ccaaatggta aaatttgatt caggt 85
<210> 31
   <211> 137
   <212> DNA
   <213> Brassica napus
<400> 31
   aggtaacact aagtttataa taaaatttgc ttagttcaat ttttttttgt ctttctaagg 60
   cttggctagt tgtgtcactt gtgtgtaaca tatgaagaat ctaagtttag ttttttttgg 120
   tgatgaatct caaaggg 137
<210> 32
   <211> 87
   <212> DNA
   <213> Brassica napus
<400> 32
   tggtaagcac accctctaat tgtttttttt aaagattcat agtcacttag ttctcctctc 60
   atccattctt ttttatcata tatagtg 87
<210> 33
   <211> 98
   <212> DNA
   <213> Brassica napus
<400> 33
   aggtaaaaca tttttctttc tgtctcgctc tcactctcac tctcttggtt ttatgtgctc 60
   agtctaagtt aagttctgca taacttttgc gtacagct 98

## Description

### FIELD OF THE INVENTION

This invention is generally related to the field of plant molecular biology, and more specifically the field of stable expression of multiple genes in transgenic plants.

### BACKGROUND OF THE INVENTION

Many plant species are capable of being transformed with transgenes from other species to introduce agronomically desirable traits or characteristics, for example, improving nutritional value quality, increasing yield, conferring pest or disease resistance, increasing drought and stress tolerance, improving horticultural qualities (such as pigmentation and growth), imparting herbicide resistance, enabling the production of industrially useful compounds and/or materials from the plant, and/or enabling the production of pharmaceuticals. The introduction of transgenes into plant cells and the subsequent recovery of fertile transgenic plants that contain a stably integrated copy of the transgene can be used to produce transgenic plants that possess the desirable traits.

Control and regulation of gene expression can occur through numerous mechanisms. Transcription initiation of a gene is a predominant controlling mechanism of gene expression. Initiation of transcription is generally controlled by polynucleotide sequences located in the 5'-flanking or upstream region of the transcribed gene. These sequences are collectively referred to as promoters and are categorized as a gene regulatory element. Promoters in plants that have been cloned and widely used for both basic research and biotechnological application are generally unidirectional, directing only one gene that has been fused at its 3' end (*i.e*., downstream). See, for example, Xie et al. (2001) Nat. Biotechnol. 19(7):677-9; U.S. Patent No. 6,388,170.

US 7,053,265 discloses a bi-directional promoter from *Lotus japonicus* PLP-IV as well as methods of producing proteins of interest and methods of controlling gene expression using said bi-directional promoter.

US 7,129,343 is directed to bi-directional promoter complexes that are effective for enhancing transcriptional activity of transgenes and wherein the bi-directional promoters include a modified enhancer region with at least two core promoters on either side of the modified enhancer in a divergent orientation.

Additional gene regulatory elements include sequences that interact with specific DNA-binding factors. These sequence motifs are sometimes referred to as *cis*-elements, and are usually position- and orientation-dependent, though they may be found 5' or 3' to a gene's coding sequence, or in an intron. Such *cis*-elements, to which tissue-specific or development-specific transcription factors bind, individually or in combination, may determine the spatiotemporal expression pattern of a promoter at the transcriptional level. These *cis*-elements vary widely in the type of control they exert on operably linked genes. Some elements act to increase the transcription of operably-linked genes in response to environmental responses (*e.g*., temperature, moisture, and wounding). Other *cis*-elements may respond to developmental cues (*e.g*., germination, seed maturation, and flowering) or to spatial information (*e.g*., tissue specificity). See, for example, Langridge et al. (1989) Proc. Natl. Acad. Sci. USA 86:3219-23.

It is often necessary to introduce multiple genes into plants for metabolic engineering and trait stacking, which genes are frequently controlled by identical or homologous promoters. However, homology-based gene silencing (HBGS) is likely to arise when multiple introduced transgenes have homologous promoters driving them. See *e.g*., Mol et al. (1989) Plant Mol. Biol. 13:287-94. HBGS has been reported to occur extensively in transgenic plants. See *e.g*., Vaucheret and Fagard (2001) Trends Genet. 17:29-35. Several mechanisms have been suggested to explain the phenomena of HBGS, all of which include the feature that sequence homology in the promoter triggers cellular recognition mechanisms that result in silencing of the repeated genes. See *e.g.,* Matzke and Matzke (1995) Plant Physiol. 107:679-85; Meyer and Saedler (1996) Ann. Rev. Plant Physiol. Plant Mol. Biol. 47:23-48; Fire (1999) Trends Genet. 15:358-63; Hamilton and Baulcombe (1999) Science 286:950-2; and Steimer et al. (2000) Plant Cell 12:1165-78.

Strategies to avoid HBGS in transgenic plants frequently involve the development of various promoters that are functionally equivalent but have minimal sequence homology. Thus, there remains a need for constructs and methods for stable expression of multiple transgenes effectively with minimum risk for recombination or loss of transgenes through breeding or multiple generations in transgenic plants.

### SUMMARY

Provided are constructs and methods for expressing multiple genes in plant cells and/or plant tissues using a disclosed bidirectional promoter from *Brassica napus* or *Brassica* bidirectional constitutive promoter (BBCP). The constructs provided comprise at least one such bi-directional promoter linked to multiple gene expression cassettes, wherein each of the gene expression cassettes comprises at least one transgene. The constructs and methods provided allow expression of genes between two and twenty.

In one aspect, provided is a nucleic acid construct for expressing multiple genes in plant cells and/or tissues. The nucleic acid construct comprises (a) a bi-directional promoter comprising a nucleotide sequence selected from SEQ ID NO: 2 or 3; and (b) two gene expression cassettes on opposite ends of the bi-directional promoter.

In one embodiment, the bi-directional promoter comprises at least one enhancer. In another embodiment, the bi-directional promoter does not comprise an enhancer. In another embodiment, the nucleic acid construct comprises a binary vector for plant transformation. In another embodiment, the nucleic acid construct comprises a binary vector for *Agrobacterium-*mediated transformation. In another embodiment, the bi-directional promoter comprises at least one intron. In another embodiment, the bi-directional promoter comprises at least one 5' untranslated region. The bi-directional promoter comprises a nucleotide sequence selected from SEQ ID NO: 2 or 3. Further described herein is a bi-directional promoter comprising a nucleotide sequence having at least 85 %, 90 %, 95 %, or 100 % identity to SEQ ID NO: 1. It is further described that the bi-directional promoter comprises a nucleotide sequence selected from SEQ ID NOs: 1, 22-25, or their complements. It is further described that the bi-directional promoter comprises a nucleotide sequence selected from SEQ ID NOs: 1, 22-24, or their complements. It is further described that the bi-directional promoter comprises a nucleotide sequence selected from SEQ ID NOs: 1, 22-23, or their complements. It is further described that the bi-directional promoter comprises a nucleotide sequence selected from SEQ ID NO: 1, 22, or their complements.

In one embodiment, at least one of the gene expression cassettes comprises two or more genes linked via a translation switch. In another embodiment, both the gene expression cassettes comprise two or more genes linked via a translation switch. In a further or alternative embodiment, the translation switch is selected from the group consisting of an internal ribosome entry site (IRES), an alternative splicing site, a ribozyme cleavage site, a polynucleotide sequence coding a 2A peptide, a polynucleotide sequence coding a 2A-like peptide, a polynucleotide sequence coding an intein, a polynucleotide sequence coding a protease cleavage site, and combinations thereof. It is further described that the translation switch comprises a cis-acting hydrolase element (CHYSEL). In a further embodiment, the CHYSEL is a 2A or 2A-like peptide sequence. In another embodiment, a gene upstream of the translational switch does not comprise a translation stop codon.

As described herein the nucleic acid construct may comprise at least one transgene. In another embodiment, the nucleic acid construct enables or allows expression of at least four genes. Further described is that all four genes are transgenes. In another embodiment, the nucleic acid construct enables expression of between three and twenty genes. In another embodiment, the nucleic acid construct enables expression of between four and eight genes. As described herein the genes may be transgenes. Further described herien, at least one gene expression cassette comprises a polynucleotide sequence encoding a fusion protein. The fusion proteins may comprise three to five genes. In another embodiment, both the gene expression cassettes do not comprise a EPSPS gene or paralog.

Further disclosed herein is a nucleic acid construct comprising a regulatory element useful for terminating the expression of a single or multiple genes in plant cells and/or tissues. The regulatory element comprises a paralog A 3'untranslated region (UTR) or poly A region which can be fused to the 3' end of a transgene. It is described that the paralog A 3' UTR comprises a functional polyadenylation sequence that is useful for the termination and regulation of transcription and translation. It is further described that the regulatory element comprises a polynucleotide sequence having at least 80%, 85%, 90%, 95% or 100% identity to SEQ ID NO: 26 or its complement. It is further described that the regulatory element comprises a polynucleotide sequence of SEQ ID NO: 26 or its complement.

In another aspect, provided is a method for generating a transgenic plant, comprising transforming a plant cell with the nucleic acid construct provided herein. In another aspect, provided is a method for generating a transgenic cell, comprising transforming the cell with the nucleic acid construct provided herein. In another aspect, provided is a plant cell comprising the nucleic acid construct provided herein. In a further or alternative embodiment, the nucleic acid construct is stably transformed into the plant cell. In another aspect, provided is a transgenic plant or seed comprising the nucleic acid construct provided herein. In a further or alternative embodiment, the nucleic acid construct is stably transformed into cells of the transgenic plant or seed. In a further embodiment, the transgenic plant is a dicotyledonous plant. In another further embodiment, the transgenic plant is a monocotyledonous plant. In another aspect, provide is a method for expressing multiple genes in plant cells and/or tissues, comprising introducing into the plant cells and/or tissues the nucleic acid construct provided herein. In a further or alternative embodiment, the plant cells and/or tissues are stably transformed with the nucleic acid construct provided herein. In another aspect, provided is a binary vector for *Agrobacterium-*mediated transformation. The binary vector comprises the nucleic acid construct provided herein. Also described herein is the use of a bi-directional promoter provided herein for multiple-transgenes expression in plants. The bi-directional promoter comprises a nucleotide sequence selected from SEQ ID NO: 2 or 3. In another aspect, provided is the use of a bi-directional promoter provided herein in the manufacturing of transgenic plants or seeds. The bi-directional promoter comprises a nucleotide sequence selected from SEQ ID NO: 2 or 3.

Further disclosed herein is a nucleic acid construct comprising at least one *Brassica* intron sequence in transgenic plant cells and/or tissues. It is described that the *Brassica* intron sequence is selected from SEQ ID NOs: 27-33. Further described is the use of at least one *Brassica* intron sequence in the manufacturing of transgenic plants or seeds. Further described is that the *Brassica* intron sequence is selected from SEQ ID NOs: 27-33.

### BRIEF DESCRIPTION OF THE DRAWINGS AND SEQUENCES

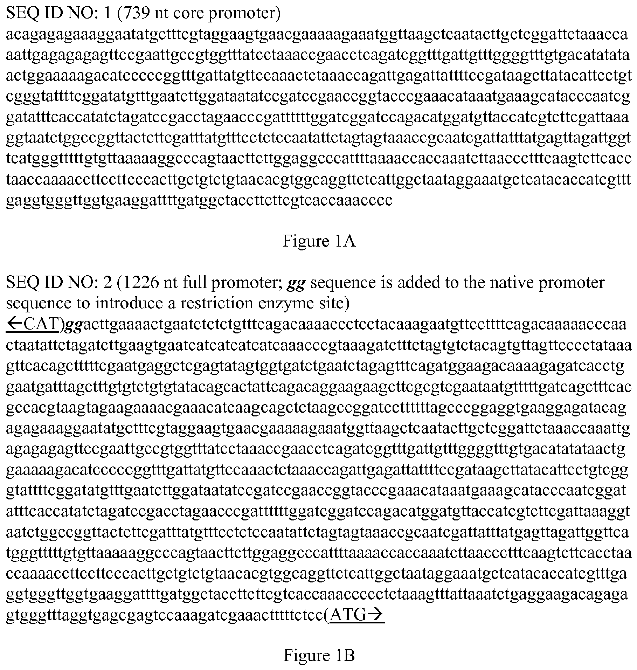

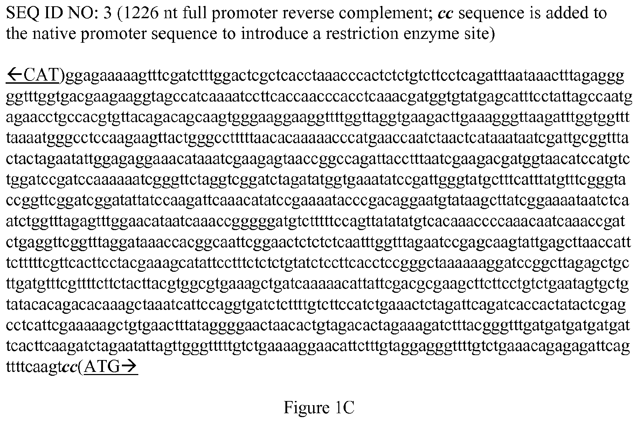

Figure 1A shows a sequence of 739 nt core bidirectional promoter of *Brassica* bidirectional constitutive promoter (BBCP) (SEQ ID NO: 1). Figure 1B shows a modified 1226 nt sequence of BBCP (SEQ ID NO: 2), where the nucleotide sequence "gg" is added to introduce a restriction enzyme cleavage site. The Figure 1C shows SEQ ID NO: 3 which is a reverse compliment of SEQ ID NO: 2, where the added "gg" sequence of SEQ ID NO: 2 is shown as "cc" sequence of SEQ ID NO: 3.

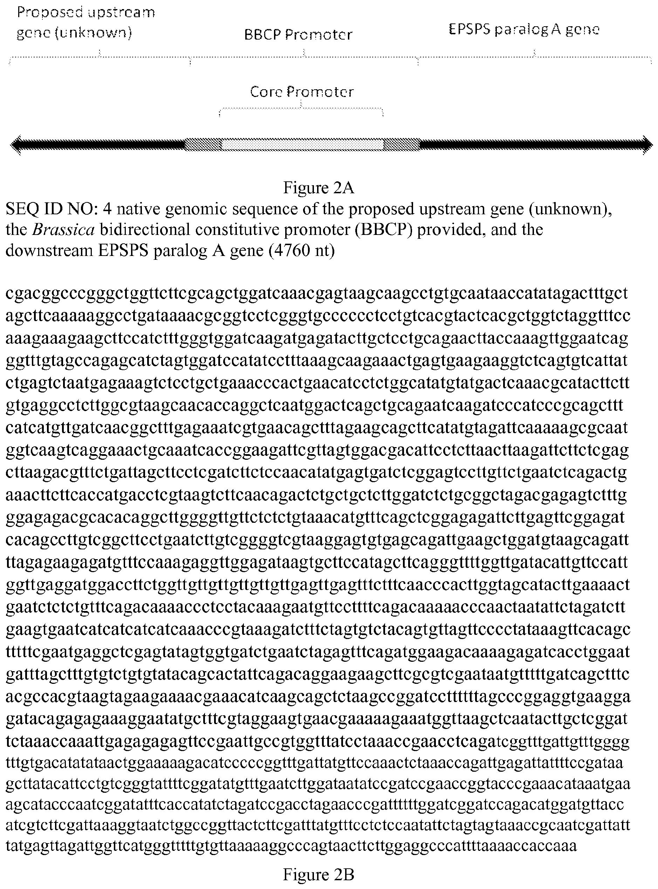

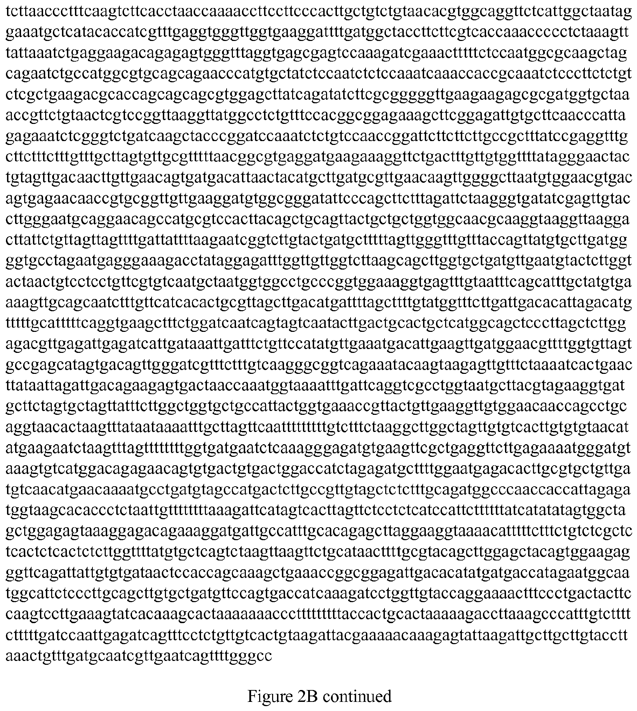

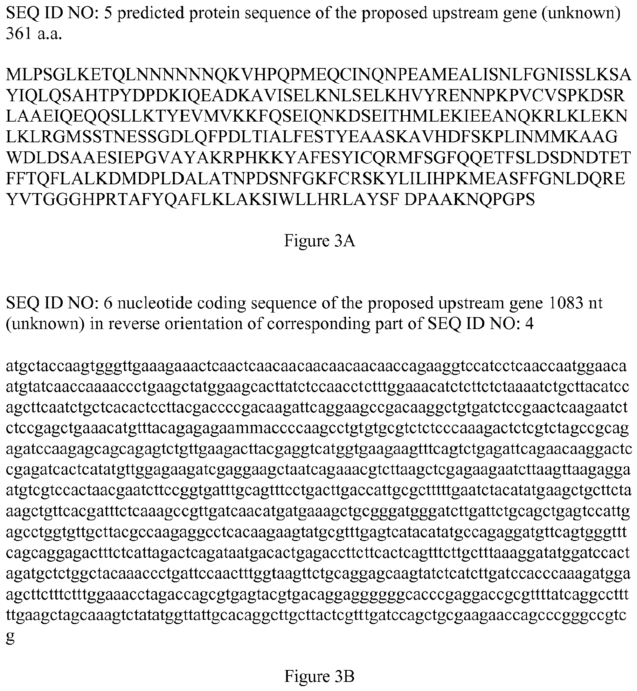

Figure 2A shows identification of BBCP from the genome of *Brassica napus,* where expression of EPSPS paralog A gene is driven by BBCP. On the opposite end of BBCP from EPSPS paralog A gene, a proposed unknown gene is also identified. Figure 2B shows polynucleotide sequence of native genomic sequence (SEQ ID NO: 4) comprising (1) the proposed unknown gene, (2) BBCP, and (3) EPSPS paralog A gene. Figure 3A further shows predicted protein sequence of this proposed unknown gene (SEQ ID NO: 5), and Figure 3B shows corresponding coding sequence (SEQ ID NO: 6).

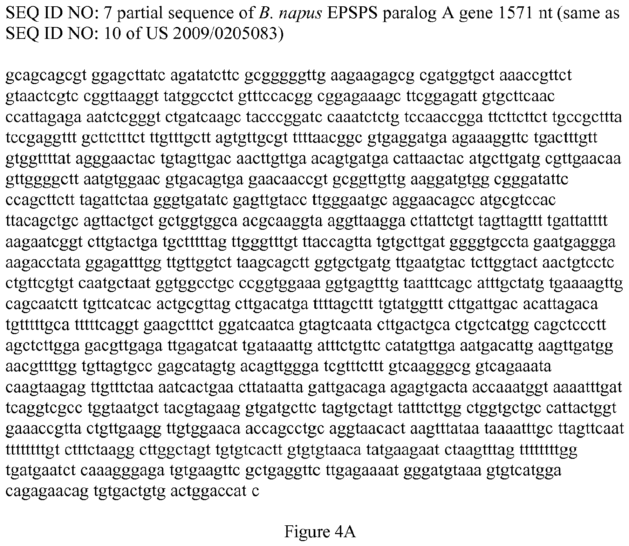

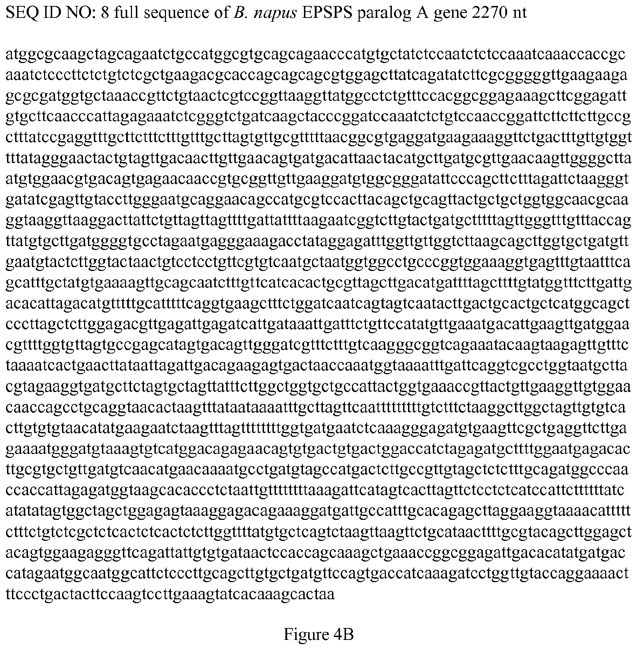

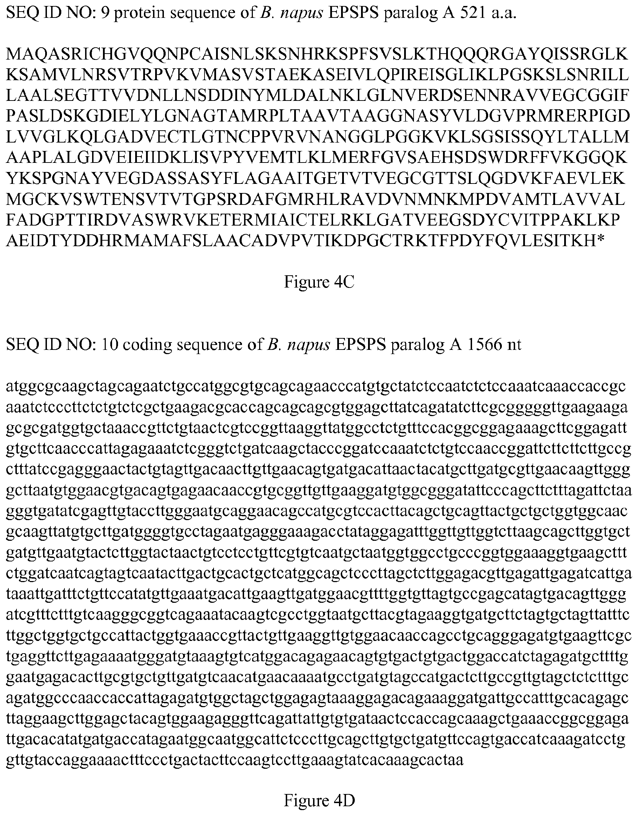

Figure 4A shows a partial sequence of EPSPS paralog A gene (SEQ ID NO: 7), which is the same as SEQ ID NO: 10 of US 2009/0205083. Figure 4B shows a full sequence of EPSPS paralog A gene. Figure 4C further shows protein sequence of EPSPS paralog A (SEQ ID NO: 9), and Figure 4D shows corresponding coding sequence of EPSPS paralog A (SEQ ID NO: 10).

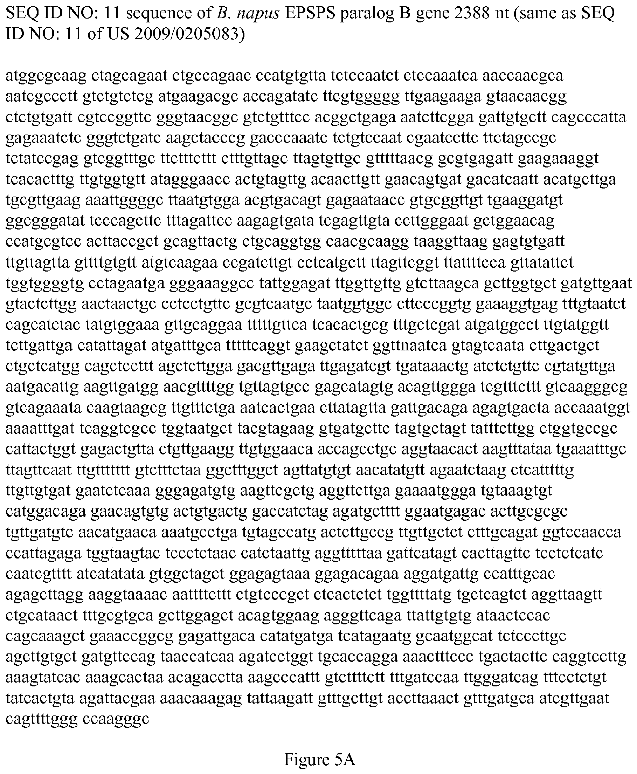

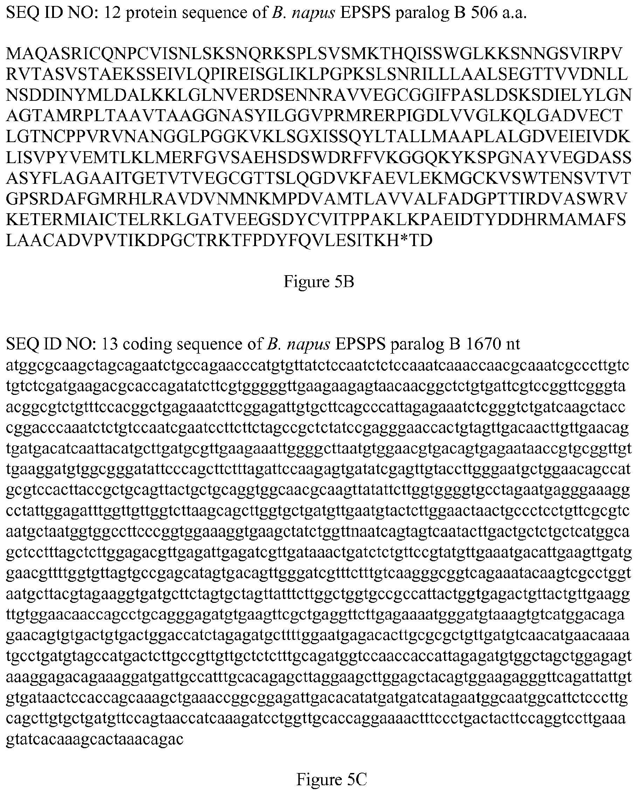

Figure 5A shows sequence of EPSPS paralog B gene (SEQ ID NO: 11), which is the same as SEQ ID NO: 11 of US 2009/0205083. Figure 5B further shows protein sequence of EPSPS paralog B (SEQ ID NO: 12), and Figure 5C shows corresponding coding sequence of EPSPS paralog B (SEQ ID NO: 13).

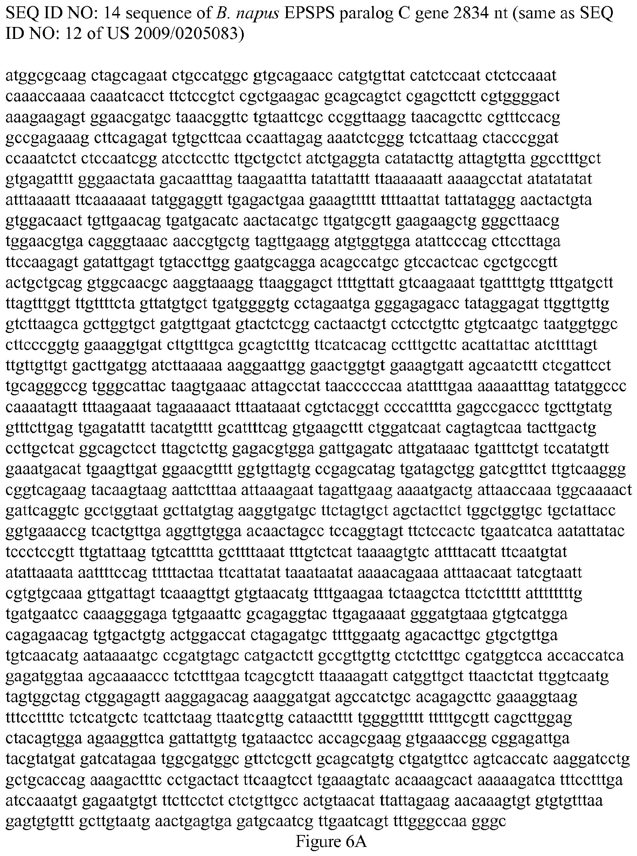

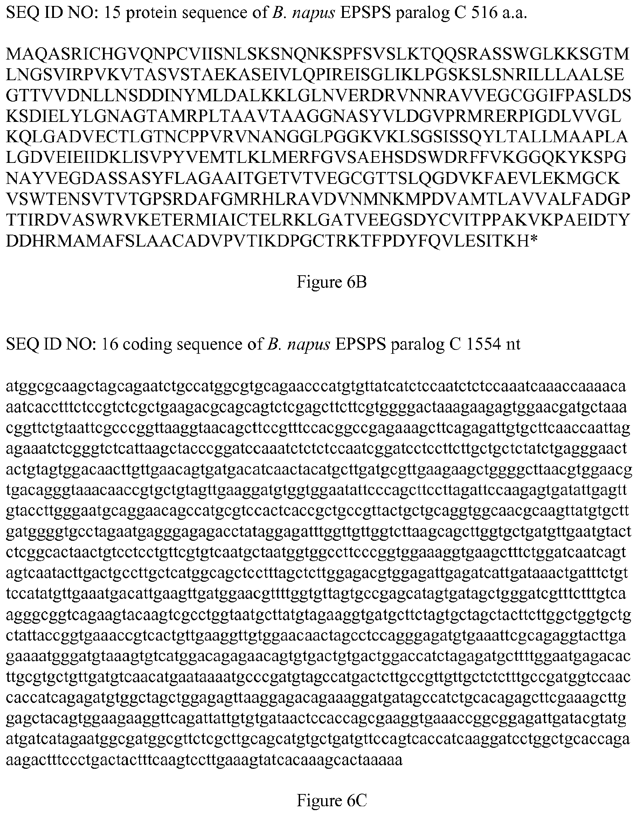

Figure 6A shows sequence of EPSPS paralog C gene (SEQ ID NO: 14), which is the same as SEQ ID NO: 12 of US 2009/0205083. Figure 6B further shows protein sequence of EPSPS paralog C (SEQ ID NO: 15), and Figure 6C shows corresponding coding sequence of EPSPS paralog C (SEQ ID NO: 16).

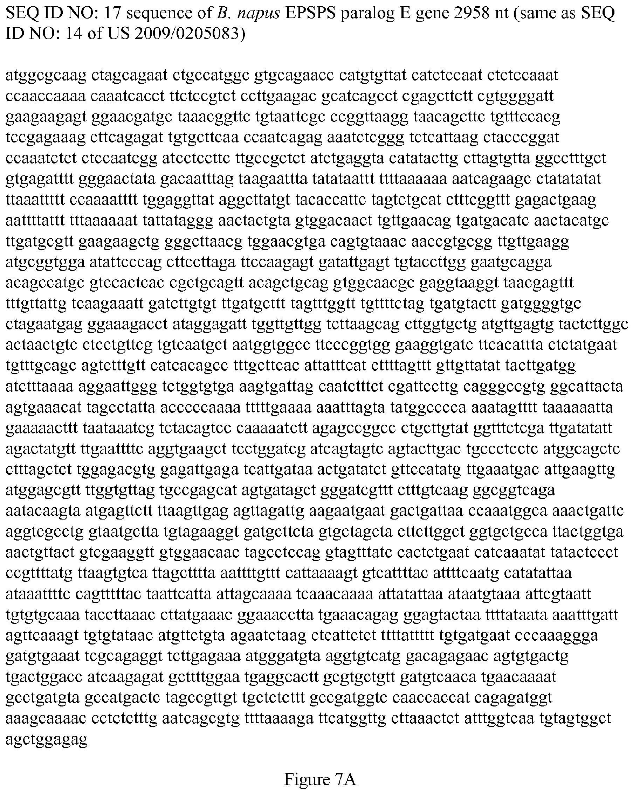

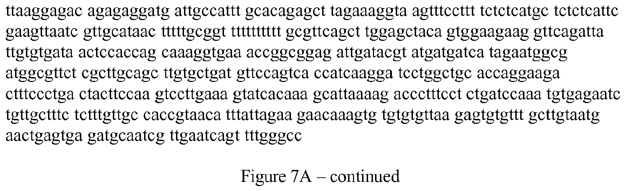

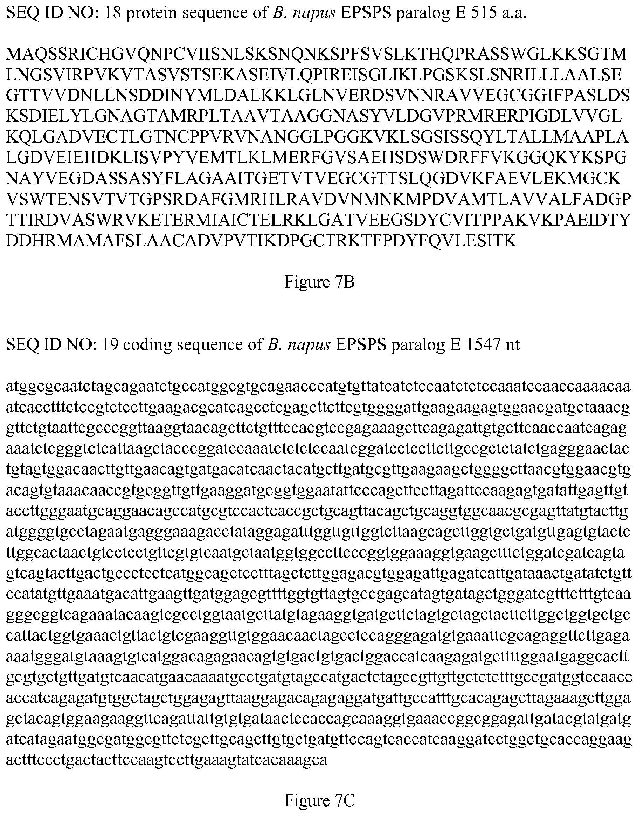

Figure 7A shows sequence of EPSPS paralog E gene (SEQ ID NO: 17), which is the same as SEQ ID NO: 14 of US 2009/0205083. Figure 7B further shows protein sequence of EPSPS paralog E (SEQ ID NO: 18), and Figure 7C shows corresponding coding sequence of EPSPS paralog C (SEQ ID NO: 19).

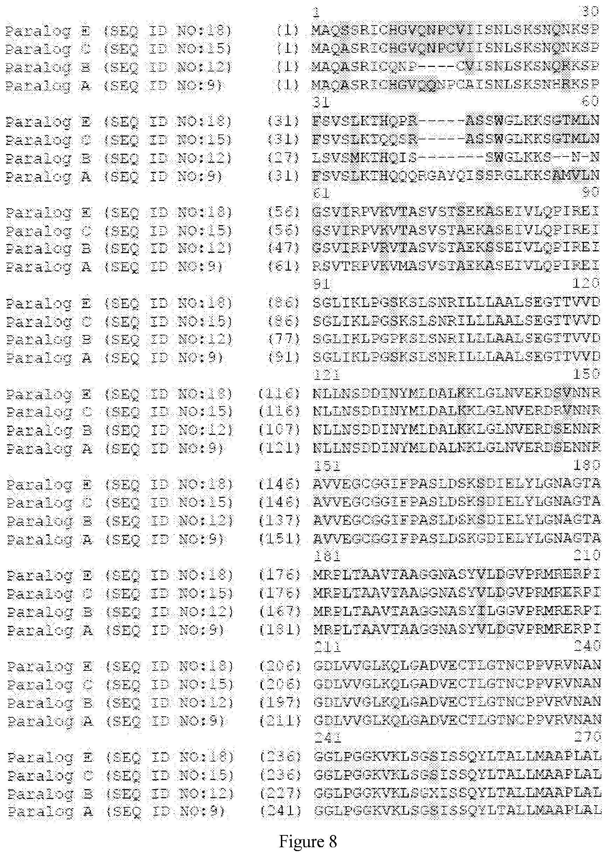

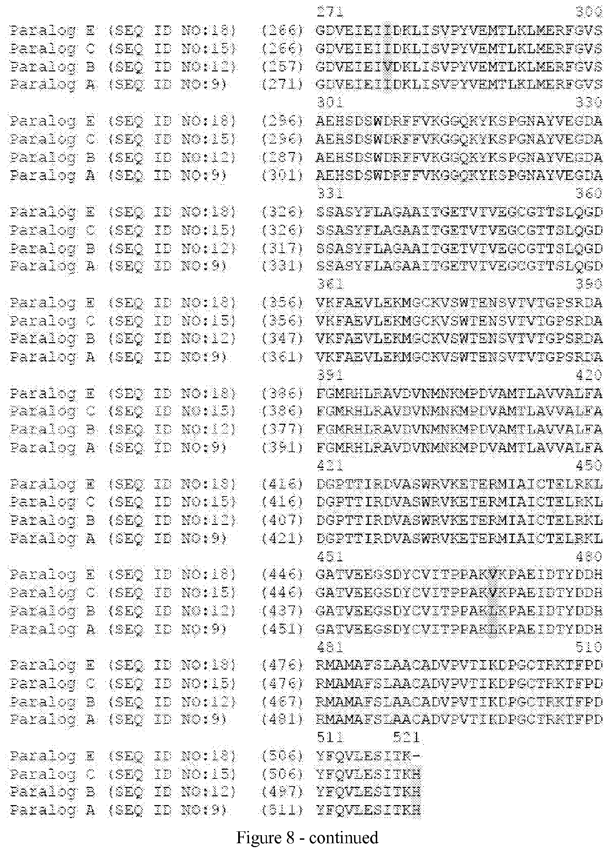

Figure 8 shows an exemplary sequence alignment among protein sequences of EPSPS paralog A (SEQ ID NO: 9), EPSPS paralog B (SEQ ID NO: 12), EPSPS paralog C (SEQ ID NO: 15), and EPSPS paralog E (SEQ ID NO: 18).

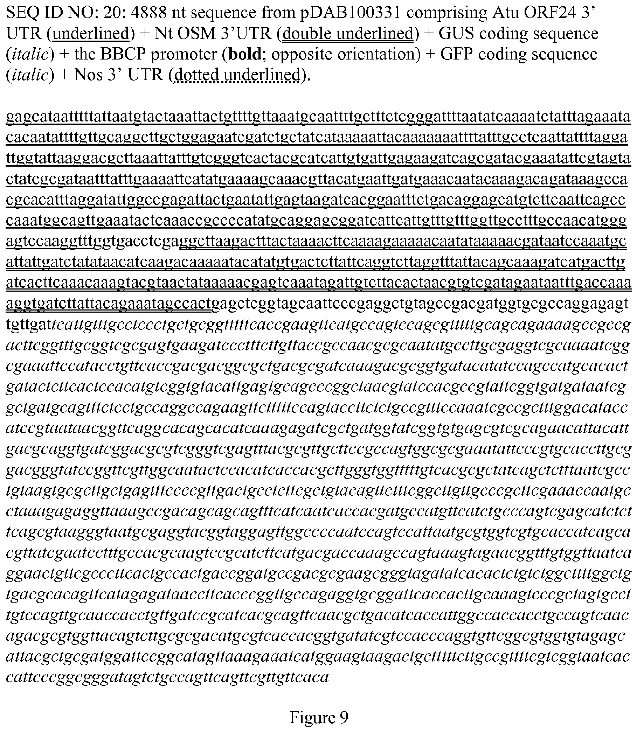

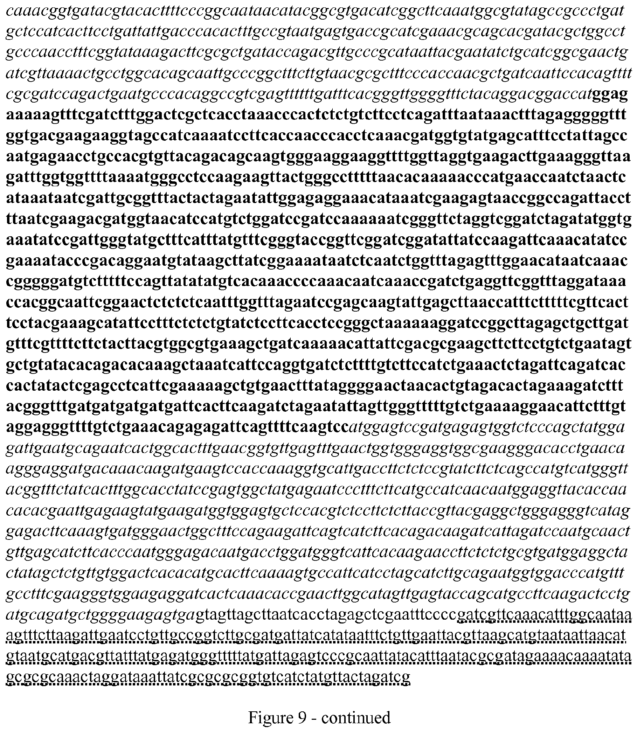

Figure 9 shows an exemplary sequence from pDAB100331 (SEQ ID NO: 20) comprising gene expression cassettes for GUS and GFP on opposite ends of BBCP. Expression of both GUS and GFP is driven by BBCP.

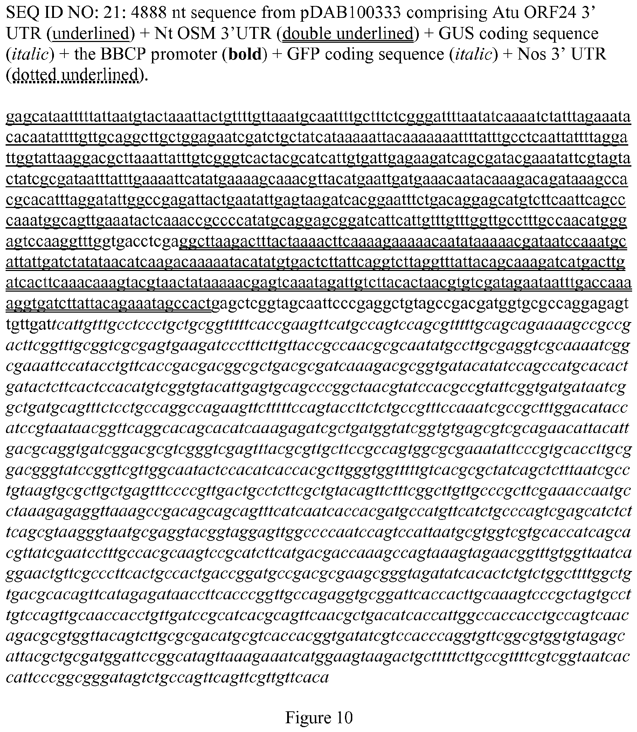

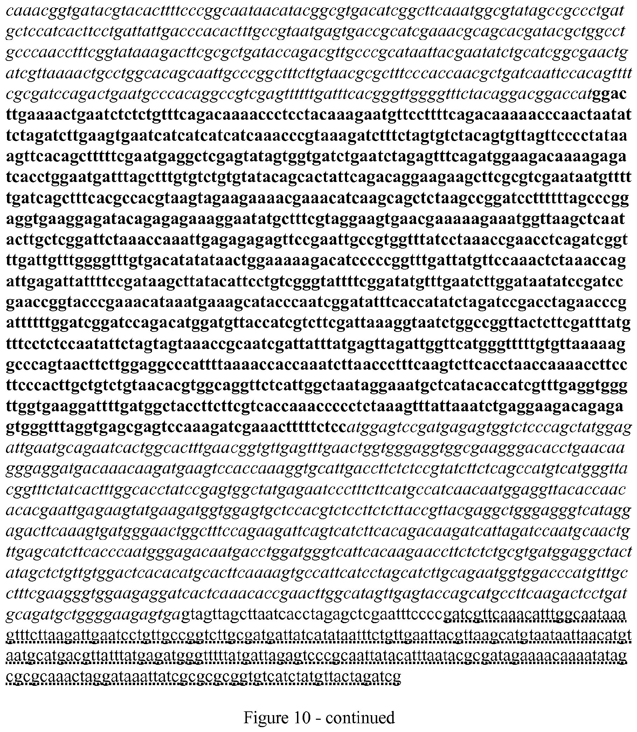

Figure 10 shows an exemplary sequence from pDAB100333 (SEQ ID NO: 21) comprising gene expression cassettes for GUS and GFP on opposite ends of BBCP. Expression of both GUS and GFP is driven by BBCP.

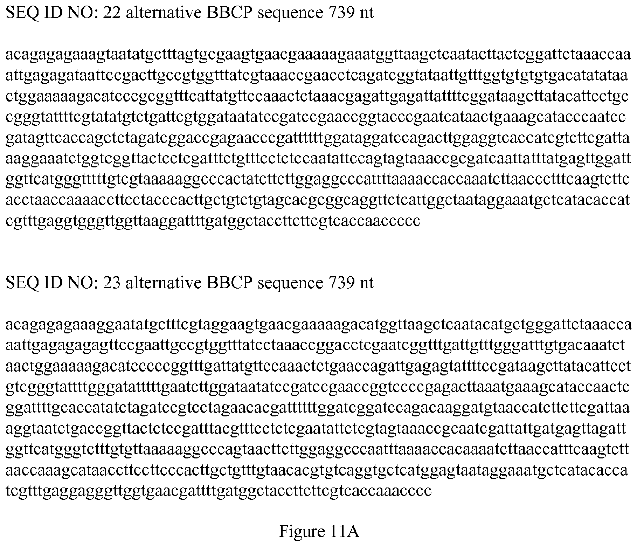

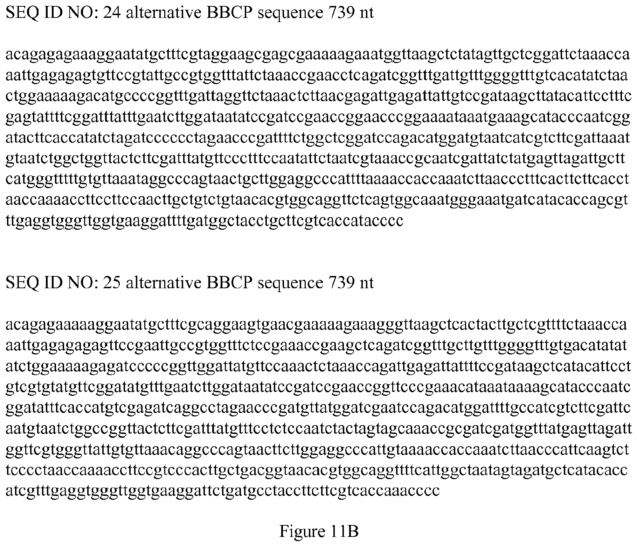

Figure 11 shows alternative BBCP sequences, including SEQ ID NOs: 22-25.
Figure 12 shows representative maps of plasmid pDAB 100331 and pDAB100333.

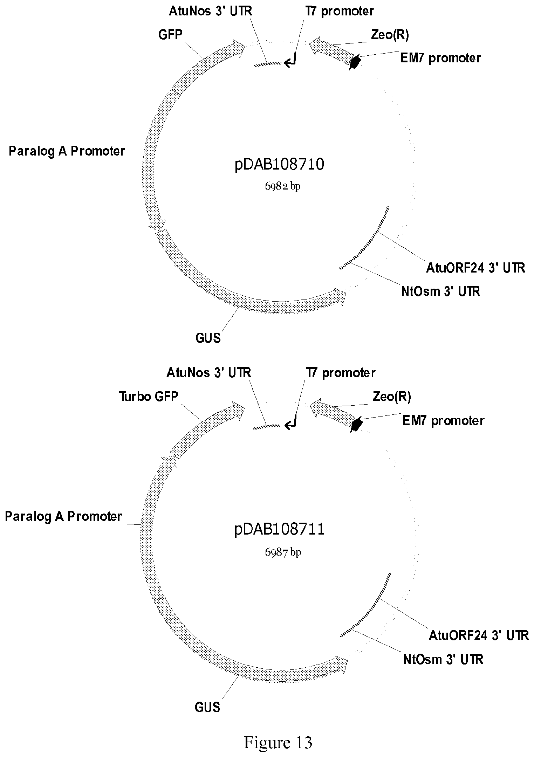

Figure 13 shows representative maps of plasmid pDAB 108710 and pDAB108711.
Figure 14 shows an exemplary EPSPS paralog A 3'UTR gene sequence (SEQ ID NO: 26), and seven paralog A intron sequences (SEQ ID Nos: 27-33).

### DETAILED DESCRIPTION

Development of transgenic products is becoming increasingly complex, which requires stacking multiple transgenes into a single locus. Traditionally each transgene usually requires a unique promoter for expression, so multiple promoters are required to express different transgenes within one gene stack. In addition to increasing the size of the gene stack, this frequently leads to repeated use of the same promoter to obtain similar levels of expression patterns of different transgenes for expression of a single polygenic trait. Multi-gene constructs driven by the same promoter are known to cause gene silencing, thus making transgenic products less efficacious in the field. Excess of transcription factor (TF)-binding sites due to promoter repetition can cause depletion of endogenous TFs leading to transcriptional inactivation. The silencing of transgenes will likely undesirably affect the performance of a transgenic plant produced to express the transgenes. Repetitive sequences within a transgene may lead to gene intra-locus homologous recombination resulting in polynucleotide rearrangements.

Provided are methods and constructs using a *Brassica* bidirectional constitutive promoter (BBCP) to express transgenes in plant. Also provided are methods and constructs combining the bidirectional promoter system with bicistronic organization of genes on either one or both ends of the promoter, for example with the use of a 2A sequence from *Thosea asigna* virus. The 2A protein, which is only 16-20 amino acids long, cleaves the polyprotein at its own carboxyl-terminus. This "self-cleavage" or "ribosome skip" property of the 2A or 2A-like peptide can be used to process artificial polyproteins produced in transgenic plants. In one embodiment, Cry34 and Cry35 genes are fused in one gene expression cassette, where GFP (or YFP or PhiYFP) and AAD 1 genes are fused into another gene expression cassette (with a single open reading frame (ORF) with a copy of the 2A protein gene placed between the two genes in each combination). For example, each of these gene expression cassettes (or gene pairs) can be placed on the either end of the bidirectional promoter to drive 4 transgenes using a single promoter. Thus, the constructs and methods provided herein are useful to avoid repeated use of the same promoter and significantly reduce the size of commercial constructs. In addition, driving four or more genes with one promoter also provides ability to co-express genes controlling a single polygenic trait.

Certain abbreviations disclosed are listed in Table 1.

| Table 1. Abbreviations used in the disclosure | |
|---|---|
| Phrase | Abbreviation |
| bicinchoninic acid | BCA |
| cauliflower mosaic virus | CaMV |
| chloroplast transit peptide | CTP |
| homology-based gene silencing | HBGS |
| ZmUbil minimal core promoter | minUbilP |
| oligo ligation amplification | OLA |
| phosphate buffered saline | PBS |
| phosphate buffered saline with 0.05% Tween 20 | PBST |
| polymerase chain reaction | PCR |
| rolling circle amplification | RCA |
| reverse transcriptase PCR | RT-PCR |
| single nucleotide primer extension | SNuPE |
| upstream regulatory sequence | URS |

Plant promoters used for basic research or biotechnological application are generally unidirectional, directing only one gene that has been fused at its 3' end (downstream). It is often necessary to introduce multiple genes into plants for metabolic engineering and trait stacking and therefore, multiple promoters are typically required in future transgenic crops to drive the expression of multiple genes. It is desirable to design strategies that can save the number of promoters deployed and allow simultaneous co-regulated expression for gene stacking. In some embodiment, the bi-directional promoters provided can drive transcription of multiple transcription units, including RNAi, artificial miRNA, or hairpin-loop RNA sequences.

As used herein, the articles, "a," "an," and "the" include plural references unless the context clearly and unambiguously dictates otherwise.

As used herein, the phrase" backcrossing" refers to a process in which a breeder crosses hybrid progeny back to one of the parents, for example, a first generation hybrid F1 with one of the parental genotypes of the F1 hybrid.

As used herein, the phrase "intron" refers to any nucleic acid sequence comprised in a gene (or expressed nucleotide sequence of interest) that is transcribed but not translated. Introns include untranslated nucleic acid sequence within an expressed sequence of DNA, as well as the corresponding sequence in RNA molecules transcribed therefrom.

The construct provided can also contain sequences that enhance translation and/or mRNA stability such as introns. An example of one such intron is the first intron of gene II of the histone H3.III variant of *Arabidopsis thaliana* or any other commonly known intron sequence. Chaubet et al. Journal of Molecular Biology, 225:569-574 (1992). It is known in the art that introns can be used in combination with a promoter sequences to enhance translation and/or mRNA stability.

As used herein, the phrase "5' untranslated region" or "5'UTR" refers to an untranslated segment in 5' terminus of the pre-mRNAs or mature mRNAs. For example, on mature mRNAs, the 5'UTR typically harbors on its 5' end a 7-methylguanosine cap and is involved in many processes such as splicing, polyadenylation, mRNA export towards the cytoplasm, identification of the 5' end of the mRNA by the translational machinery and protection of the mRNAs against degradation.

As used herein, the phrase "3' untranslated region" or "3'UTR" refers to an untranslated segment in 3' terminus of the pre-mRNAs or mature mRNAs. For example, on mature mRNAs this region harbors the poly (A) tail and is known to have many roles in mRNA stability, translation initiation, mRNA export.

As used herein, the phrase "polyadenylation signal" refers to a nucleic acid sequence present in the mRNA transcripts, that allows for the transcripts, when in the presence of the poly (A) polymerase, to be polyadenylated on the polyadenylation site, for example, located 10 to 30 bases downstream the poly (A) signal. Many polyadenylation signals are known in the art and are useful for the present invention. Examples include the human variant growth hormone polyadenylation signal, the SV40 late polyadenylation signal and the bovine growth hormone polyadenylation signal.

As used herein, the phrase "isolated" refers to biological component (including a nucleic acid or protein) has been substantially separated, produced apart from, or purified away from other biological components in the cell of the organism in which the component naturally occurs (*i.e.,* other chromosomal and extra-chromosomal DNA and RNA, and proteins), while effecting a chemical or functional change in the component (e.g., a nucleic acid may be isolated from a chromosome by breaking chemical bonds connecting the nucleic acid to the remaining DNA in the chromosome). Nucleic acid molecules and proteins that have been "isolated" include nucleic acid molecules and proteins purified by standard purification methods. The phrase "isolated" also embraces nucleic acids and proteins prepared by recombinant expression in a host cell, as well as chemically-synthesized nucleic acid molecules, proteins, and peptides.

As used herein, the phrase "gene expression" refers to a process by which the coded information of a nucleic acid transcriptional unit (including, *e.g*., genomic DNA) is converted into an operational, non-operational, or structural part of a cell, often including the synthesis of a protein. Gene expression can be influenced by external signals; for example, exposure of a cell, tissue, or organism to an agent that increases or decreases gene expression. Expression of a gene can also be regulated anywhere in the pathway from DNA to RNA to protein. Regulation of gene expression occurs, for example, through controls acting on transcription, translation, RNA transport and processing, degradation of intermediary molecules such as mRNA, or through activation, inactivation, compartmentalization, or degradation of specific protein molecules after they have been made, or by combinations thereof. Gene expression can be measured at the RNA level or the protein level by any method known in the art, including, without limitation, Northern blot, RT-PCR, Western blot, or in vitro, in situ, or in vivo protein activity assay(s).

As used herein, the phrase "homology-based gene silencing" (HBGS) refers to a generic term that includes both transcriptional gene silencing and posttranscriptional gene silencing. Silencing of a target locus by an unlinked silencing locus can result from transcription inhibition (transcriptional gene silencing; TGS) or mRNA degradation (post-transcriptional gene silencing; PTGS), owing to the production of double-stranded RNA (dsRNA) corresponding to promoter or transcribed sequences, respectively. The involvement of distinct cellular components in each process suggests that dsRNA-induced TGS and PTGS likely result from the diversification of an ancient common mechanism. However, a strict comparison of TGS and PTGS has been difficult to achieve because it generally relies on the analysis of distinct silencing loci. A single transgene locus can be described to trigger both TGS and PTGS, owing to the production of dsRNA corresponding to promoter and transcribed sequences of different target genes. See, for example, Mourrain et al. (2007) Planta 225:365-79. It is likely that siRNAs are the actual molecules that trigger TGS and PTGS on homologous sequences: the siRNAs would in this model trigger silencing and methylation of homologous sequences in *cis* and in *trans* through the spreading of methylation of transgene sequences into the endogenous promoter.

As used herein, the phrase "nucleic acid molecule" (or "nucleic acid" or "polynucleotide") refers to a polymeric form of nucleotides, which may include both sense and anti-sense strands of RNA, cDNA, genomic DNA, and synthetic forms and mixed polymers of the above. A nucleotide may refer to a ribonucleotide, deoxyribonucleotide, or a modified form of either type of nucleotide. A "nucleic acid molecule" as used herein is synonymous with "nucleic acid" and "polynucleotide." A nucleic acid molecule is usually at least 10 bases in length, unless otherwise specified. The term may refer to a molecule of RNA or DNA of indeterminate length. The term includes single- and double-stranded forms of DNA. A nucleic acid molecule may include either or both naturally-occurring and modified nucleotides linked together by naturally occurring and/or non-naturally occurring nucleotide linkages.

Nucleic acid molecules may be modified chemically or biochemically, or may contain non-natural or derivatized nucleotide bases, as will be readily appreciated by those of skill in the art. Such modifications include, for example, labels, methylation, substitution of one or more of the naturally occurring nucleotides with an analog, internucleotide modifications (e.g., uncharged linkages: for example, methyl phosphonates, phosphotriesters, phosphoramidates, carbamates, etc.; charged linkages: for example, phosphorothioates, phosphorodithioates, etc.; pendent moieties: for example, peptides; intercalators: for example, acridine, psoralen, etc.; chelators; alkylators; and modified linkages: for example, alpha anomeric nucleic acids, etc.). The term "nucleic acid molecule" also includes any topological conformation, including single-stranded, double-stranded, partially duplexed, triplexed, hairpinned, circular, and padlocked conformations.

Transcription proceeds in a 5' to 3' manner along a DNA strand. This means that RNA is made by the sequential addition of ribonucleotide-5'-triphosphates to the 3' terminus of the growing chain (with a requisite elimination of the pyrophosphate). In either a linear or circular nucleic acid molecule, discrete elements (*e.g*., particular nucleotide sequences) may be referred to as being "upstream" relative to a further element if they are bonded or would be bonded to the same nucleic acid in the 5' direction from that element. Similarly, discrete elements may be "downstream" relative to a further element if they are or would be bonded to the same nucleic acid in the 3' direction from that element.

As used herein, the phrase "base position," refers to the location of a given base or nucleotide residue within a designated nucleic acid. The designated nucleic acid may be defined by alignment (see below) with a reference nucleic acid.

As used herein, the phrase "hybridization" refers to a process where oligonucleotides and their analogs hybridize by hydrogen bonding, which includes Watson-Crick, Hoogsteen or reversed Hoogsteen hydrogen bonding, between complementary bases. Generally, nucleic acid molecules consist of nitrogenous bases that are either pyrimidines (cytosine (C), uracil (U), and thymine (T)) or purines (adenine (A) and guanine (G)). These nitrogenous bases form hydrogen bonds between a pyrimidine and a purine, and the bonding of the pyrimidine to the purine is referred to as "base pairing." More specifically, A will hydrogen bond to T or U, and G will bond to C. "Complementary" refers to the base pairing that occurs between two distinct nucleic acid sequences or two distinct regions of the same nucleic acid sequence.

As used herein, the phrases "specifically hybridizable" and "specifically complementary" refers to a sufficient degree of complementarity such that stable and specific binding occurs between the oligonucleotide and the DNA or RNA target. The oligonucleotide need not be 100% complementary to its target sequence to be specifically hybridizable. An oligonucleotide is specifically hybridizable when binding of the oligonucleotide to the target DNA or RNA molecule interferes with the normal function of the target DNA or RNA, and there is sufficient degree of complementarity to avoid non-specific binding of the oligonucleotide to non-target sequences under conditions where specific binding is desired, for example under physiological conditions in the case of in vivo assays or systems. Such binding is referred to as specific hybridization.

Hybridization conditions resulting in particular degrees of stringency will vary depending upon the nature of the chosen hybridization method and the composition and length of the hybridizing nucleic acid sequences. Generally, the temperature of hybridization and the ionic strength (especially the Na+ and/or Mg2+ concentration) of the hybridization buffer will contribute to the stringency of hybridization, though wash times also influence stringency. Calculations regarding hybridization conditions required for attaining particular degrees of stringency are discussed in Sambrook et al. (ed.), Molecular Cloning: A Laboratory Manual, 2nd ed., vol. 1-3, Cold Spring Harbor Laboratory Press, Cold Spring Harbor, New York, 1989, chs. 9 and 11.

As used herein, the phrase "stringent conditions" encompass conditions under which hybridization will only occur if there is less than 50% mismatch between the hybridization molecule and the DNA target. "Stringent conditions" include further particular levels of stringency. Thus, as used herein, "moderate stringency" conditions are those under which molecules with more than 50% sequence mismatch will not hybridize; conditions of "high stringency" are those under which sequences with more than 20% mismatch will not hybridize; and conditions of "very high stringency" are those under which sequences with more than 10% mismatch will not hybridize.

In particular embodiments, stringent conditions can include hybridization at 65 °C, followed by washes at 65 °C with 0.1x SSC/0.1% SDS for 40 minutes.

The following are representative, non-limiting hybridization conditions:
Very High Stringency: Hybridization in 5x SSC buffer at 65 °C for 16 hours; wash twice in 2x SSC buffer at room temperature for 15 minutes each; and wash twice in 0.5x SSC buffer at 65 °C for 20 minutes each.
High Stringency: Hybridization in 5-6 x SSC buffer at 65-70 °C for 16-20 hours; wash twice in 2 x SSC buffer at room temperature for 5-20 minutes each; and wash twice in 1x SSC buffer at 55-70 °C for 30 minutes each.
Moderate Stringency: Hybridization in 6x SSC buffer at room temperature to 55 °C for 16-20 hours; wash at least twice in 2x-3x SSC buffer at room temperature to 55 °C for 20-30 minutes each.

In particular embodiments, specifically hybridizable nucleic acid molecules can remain bound under very high stringency hybridization conditions. In these and further embodiments, specifically hybridizable nucleic acid molecules can remain bound under high stringency hybridization conditions. In these and further embodiments, specifically hybridizable nucleic acid molecules can remain bound under moderate stringency hybridization conditions.

As used herein, the phrase "oligonucleotide" refers to a short nucleic acid polymer. Oligonucleotides may be formed by cleavage of longer nucleic acid segments, or by polymerizing individual nucleotide precursors. Automated synthesizers allow the synthesis of oligonucleotides up to several hundred base pairs in length. Because oligonucleotides may bind to a complementary nucleotide sequence, they may be used as probes for detecting DNA or RNA. Oligonucleotides composed of DNA (oligodeoxyribonucleotides) may be used in PCR, a technique for the amplification of small DNA sequences. In PCR, the oligonucleotide is typically referred to as a "primer," which allows a DNA polymerase to extend the oligonucleotide and replicate the complementary strand.

As used herein, the phrase "sequence identity" or "identity," refers to a context where two nucleic acid or polypeptide sequences, may refer to the residues in the two sequences that are the same when aligned for maximum correspondence over a specified comparison window.

As used herein, the phrase "percentage of sequence identity" refers to the value determined by comparing two optimally aligned sequences (*e.g*., nucleic acid sequences, and amino acid sequences) over a comparison window, wherein the portion of the sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleotide or amino acid residue occurs in both sequences to yield the number of matched positions, dividing the number of matched positions by the total number of positions in the comparison window, and multiplying the result by 100 to yield the percentage of sequence identity.

Methods for aligning sequences for comparison are well-known in the art. Various programs and alignment algorithms are described in, for example: Smith and Waterman (1981) Adv. Appl. Math. 2:482; Needleman and Wunsch (1970) J. Mol. Biol. 48:443; Pearson and Lipman (1988) Proc. Natl. Acad. Sci. U.S.A. 85:2444; Higgins and Sharp (1988) Gene 73:237-44; Higgins and Sharp (1989) CABIOS 5:151-3; Corpet et al. (1988) Nucleic Acids Res. 16:10881-90; Huang et al. (1992) Comp. Appl. Biosci. 8:155-65; Pearson et al. (1994) Methods Mol. Biol. 24:307-31; Tatiana et al. (1999) FEMS Microbiol. Lett. 174:247-50. A detailed consideration of sequence alignment methods and homology calculations can be found in, *e.g.,* Altschul et al. (1990) J. Mol. Biol. 215:403-10.

The National Center for Biotechnology Information (NCBI) Basic Local Alignment Search Tool (BLAST™; Altschul *et al.* (1990)) is available from several sources, including the National Center for Biotechnology Information (Bethesda, MD), and on the internet, for use in connection with several sequence analysis programs. A description of how to determine sequence identity using this program is available on the internet under the "help" section for BLAST™. For comparisons of nucleic acid sequences, the "Blast 2 sequences" function of the BLAST™ (Blastn) program may be employed using the default parameters. Nucleic acid sequences with even greater similarity to the reference sequences will show increasing percentage identity when assessed by this method.

As used herein, the phrase "operably linked" refers to a context where the first nucleic acid sequence is operably linked with a second nucleic acid sequence when the first nucleic acid sequence is in a functional relationship with the second nucleic acid sequence. For instance, a promoter is operably linked with a coding sequence when the promoter affects the transcription or expression of the coding sequence. When recombinantly produced, operably linked nucleic acid sequences are generally contiguous and, where necessary to join two protein-coding regions, in the same reading frame. However, elements need not be contiguous to be operably linked.

As used herein, the phrase "promoter" refers to a region of DNA that generally is located upstream (towards the 5' region of a gene) that is needed for transcription. Promoters may permit the proper activation or repression of the gene which they control. A promoter may contain specific sequences that are recognized by transcription factors. These factors may bind to the promoter DNA sequences and result in the recruitment of RNA polymerase, an enzyme that synthesizes RNA from the coding region of the gene.

As used herein, the phrase "transforms" or "transduces" refers to a process where a virus or vector transfers nucleic acid molecules into a cell. A cell is "transformed" by a nucleic acid molecule "transduced" into the cell when the nucleic acid molecule becomes stably replicated by the cell, either by incorporation of the nucleic acid molecule into the cellular genome or by episomal replication. As used herein, the term "transformation" encompasses all techniques by which a nucleic acid molecule can be introduced into such a cell. Examples include, but are not limited to: transfection with viral vectors; transformation with plasmid vectors; electroporation (Fromm et al. (1986) Nature 319:791-3); lipofection (Felgner et al. (1987) Proc. Natl. Acad. Sci. USA 84:7413-7); microinjection (Mueller et al. (1978) Cell 15:579-85); *Agrobacterium-*mediated transfer (Fraley et al. (1983) Proc. Natl. Acad. Sci. USA 80:4803-7); direct DNA uptake; whiskers-mediated transformation; and microprojectile bombardment (Klein et al. (1987) Nature 327:70).

As used herein, the phrase "transgene" refers to an exogenous nucleic acid sequence. In one example, a transgene is a gene sequence (e.g., an herbicide-resistance gene), a gene encoding an industrially or pharmaceutically useful compound, or a gene encoding a desirable agricultural trait. In yet another example, the transgene is an antisense nucleic acid sequence, wherein expression of the antisense nucleic acid sequence inhibits expression of a target nucleic acid sequence. A transgene may contain regulatory sequences operably linked to the transgene (*e.g.,* a promoter). In some embodiments, a nucleic acid sequence of interest is a transgene. However, in other embodiments, a nucleic acid sequence of interest is an endogenous nucleic acid sequence, wherein additional genomic copies of the endogenous nucleic acid sequence are desired, or a nucleic acid sequence that is in the antisense orientation with respect to the sequence of a target nucleic acid molecule in the host organism.

As used herein, the phrase "vector" refers to a nucleic acid molecule as introduced into a cell, thereby producing a transformed cell. A vector may include nucleic acid sequences that permit it to replicate in the host cell, such as an origin of replication. Examples include, but are not limited to, a plasmid, cosmid, bacteriophage, or virus that carries exogenous DNA into a cell. A vector can also include one or more genes, antisense molecules, and/or selectable marker genes and other genetic elements known in the art. A vector may transduce, transform, or infect a cell, thereby causing the cell to express the nucleic acid molecules and/or proteins encoded by the vector. A vector may optionally include materials to aid in achieving entry of the nucleic acid molecule into the cell (*e.g.,* a liposome).

As used herein, the phrase "plant" includes plants and plant parts including but not limited to plant cells and plant tissues such as leaves, stems, roots, flowers, pollen, and seeds. The class of plants that can be used in the present invention is generally as broad as the class of higher and lower plants amenable to mutagenesis including angiosperms (monocotyledonous and dicotyledonous plants), gymnosperms, ferns and multicellular algae. Thus, "plant" includes dicotyledons plants and monocotyledons plants. Examples of dicotyledons plants include tobacco, Arabidopsis, soybean, tomato, papaya, canola, sunflower, cotton, alfalfa, potato, grapevine, pigeon pea, pea, Brassica, chickpea, sugar beet, rapeseed, watermelon, melon, pepper, peanut, pumpkin, radish, spinach, squash, broccoli, cabbage, carrot, cauliflower, celery, Chinese cabbage, cucumber, eggplant, and lettuce. Examples of monocotyledons plants include corn, rice, wheat, sugarcane, barley, rye, sorghum, orchids, bamboo, banana, cattails, lilies, oat, onion, millet, and triticale.

As used herein, the phrase "plant material" refers to leaves, stems, roots, flowers or flower parts, fruits, pollen, egg cells, zygotes, seeds, cuttings, cell or tissue cultures, or any other part or product of a plant. In some embodiment, plant material includes cotyledon and leaf.

As used herein, the phrase "translation switch" refers to a mechanism at end of a gene allowing translation of an immediate downstream gene. The mechanism of translation switch can function at nucleic acid level (for example, viral or eukaryotic internal ribosome entry site (IRES), an alternative splicing site, or a ribozyme cleavage site) or at peptide/protein level (for example, a 2A peptide, a 2A-like peptide, an intein peptide, or a protease cleavage site).

These mechanisms of translation switch at nucleic acid level or at peptide/protein level are well known in the art. See *e.g.,* li, Z., H. M. Schumacher, et al. (2010) J Biotechnol 145(1): 9-16; Chen, Y., K. Perumal, et al. (2000) Gene Expr 9(3): 133-143; Dinkova, T. D., H. Zepeda, et al. (2005) Plant J 41(5): 722-731; Dorokhov, Y. L., M. V. Skulachev, et al. (2002) Proc Natl Acad Sci U S A 99(8): 5301-5306; Fernandez-Miragall, O. and C. Hernandez (2011) PLoS One 6(7): e22617; Groppelli, E., G. J. Belsham, et al. (2007) J Gen Virol 88(Pt 5): 1583-1588; Ha, S. H., Y. S. Liang, et al. (2010) Plant Biotechnol J 8(8): 928-938; Karetnikov, A. and K. Lehto (2007) J Gen Virol 88(Pt 1): 286-297; Karetnikov, A. and K. Lehto (2008) Virology 371(2): 292-308; Khan, M. A., H. Yumak, et al. (2009) J Biol Chem 284(51): 35461-35470; and Koh, D. C., S. M. Wong, et al. (2003) J Biol Chem 278(23): 20565-20573. Multi-gene expression constructs containing modified inteins have been disclosed in U.S. Patent Nos. 7,026,526 and 7,741,530, as well as U.S. Patent application 2008/0115243.

As used herein, the phrase "selectable marker" or "selectable marker gene" refers to a gene that is optionally used in plant transformation to, for example, protect the plant cells from a selective agent or provide resistance/tolerance to a selective agent. Only those cells or plants that receive a functional selectable marker are capable of dividing or growing under conditions having a selective agent. Examples of selective agents can include, for example, antibiotics, including spectinomycin, neomycin, kanamycin, paromomycin, gentamicin, and hygromycin. These selectable markers include gene for neomycin phosphotransferase (npt II), which expresses an enzyme conferring resistance to the antibiotic kanamycin, and genes for the related antibiotics neomycin, paromomycin, gentamicin, and G418, or the gene for hygromycin phosphotransferase (hpt), which expresses an enzyme conferring resistance to hygromycin. Other selectable marker genes can include genes encoding herbicide resistance including Bar (resistance against BASTA^{®} (glufosinate ammonium), or phosphinothricin (PPT)), acetolactate synthase (ALS, resistance against inhibitors such as sulfonylureas (SUs), imidazolinones (IMIs), triazolopyrimidines (TPs), pyrimidinyl oxybenzoates (POBs), and sulfonylamino carbonyl triazolinones that prevent the first step in the synthesis of the branched-chain amino acids), glyphosate, 2,4-D, and metal resistance or sensitivity. The phrase "marker-positive" refers to plants that have been transformed to include the selectable marker gene.

Various selectable or detectable markers can be incorporated into the chosen expression vector to allow identification and selection of transformed plants, or transformants. Many methods are available to confirm the expression of selection markers in transformed plants, including for example DNA sequencing and PCR (polymerase chain reaction), Southern blotting, RNA blotting, immunological methods for detection of a protein expressed from the vector, *e g.,* precipitated protein that mediates phosphinothricin resistance, or other proteins such as reporter genes β-glucuronidase (GUS), luciferase, green fluorescent protein (GFP), DsRed, β-galactosidase, chloramphenicol acetyltransferase (CAT), alkaline phosphatase, and the like (See Sambrook, et al., Molecular Cloning: A Laboratory Manual, Third Edition, Cold Spring Harbor Press, N.Y., 2001.

Selectable marker genes are utilized for the selection of transformed cells or tissues. Selectable marker genes include genes encoding antibiotic resistance, such as those encoding neomycin phosphotransferase II (NEO) and hygromycin phosphotransferase (HPT) as well as genes conferring resistance to herbicidal compounds. Herbicide resistance genes generally code for a modified target protein insensitive to the herbicide or for an enzyme that degrades or detoxifies the herbicide in the plant before it can act. For example, resistance to glyphosate has been obtained by using genes coding for the mutant target enzymes, 5-enolpyruvylshikimate-3-phosphate synthase (EPSPS). Genes and mutants for EPSPS have been disclosed in U.S. Patent Nos. 4,940,835, 5,188,642, 5,310,667, 5,633,435, 5,633,448, and 6,566,587. Resistance to glufosinate ammonium, bromoxynil, and 2,4-dichlorophenoxyacetate (2,4-D) have been obtained by using bacterial genes encoding phosphinothricin acetyltransferase, a nitrilase, or a 2,4-dichlorophenoxyacetate monooxygenase, which detoxify the respective herbicides. Enzymes/genes for glufosinate resistance/tolerance have been disclosed in U.S. Patent Nos. 5,273,894, 5,276,268, 5,550,318, and 5,561,236. Enzymes/genes for 2,4-D resistance have been previously disclosed in U.S. Patent Nos. 6,100,446 and 6,153,401, as well as patent applications US 2009/0093366 and WO 2007/053482. Enzymes/genes for nitrilase has been previously disclosed in U.S. Patent Nos. 4,810,648.

Other herbicides can inhibit the growing point or meristem, including imidazolinone or sulfonylurea, and genes for resistance/tolerance of acetohydroxyacid synthase (AHAS) and acetolactate synthase (ALS) for these herbicides have been described. Genes and mutants for AHAS and mutants have been disclosed in U.S. Patent Nos. 4,761,373, 5,304,732, 5,331,107, 5,853,973, and 5,928,937. Genes and mutants for ALS have been disclosed in U.S. Patent Nos. 5,013,659 and 5,141,870.

Glyphosate resistance genes include mutant 5-enolpyruvylshikimate-3-phosphate synthase (EPSPs) genes (via the introduction of recombinant nucleic acids and/or various forms of *in vivo* mutagenesis of native EPSPs genes), aroA genes and glyphosate acetyl transferase (GAT) genes, respectively). Resistance genes for other phosphono compounds include glufosinate (phosphinothricin acetyl transferase (PAT) genes from Streptomyces species,
including Streptomyces hygroscopicus and Streptomyces viridichromogenes), and pyridinoxy or phenoxy proprionic acids and cyclohexones (ACCase inhibitor-encoding genes). Herbicide resistance/tolerance genes of acetyl coemzyme A carboxylase (ACCase) have been described in U.S. Patents 5,162,602 and 5,498,544.

A DNA molecule encoding a mutant aroA gene can be obtained under ATCC accession number 39256, and the nucleotide sequence of the mutant gene is disclosed in U.S. Pat. No. 4,769,061 to Comai, European patent application No. 0 333 033 to Kumada et al.*,* and U.S. Pat. No. 4,975,374 to Goodman et al.*,* disclosing nucleotide sequences of glutamine synthetase genes which confer resistance to herbicides such as L-phosphinothricin. The nucleotide sequence of a PAT gene is provided in European application No. 0 242 246 to Leemans et al. Also DeGreef et al., Bio/Technology 7:61 (1989), describes the production of transgenic plants that express chimeric bar genes coding for PAT activity. Exemplary of genes conferring resistance to phenoxy proprionic acids and cyclohexones, including sethoxydim and haloxyfop, are the Acc1-S1, Acc1-S2 and Acc1-S3 genes described by Marshall et al., Theon. Appl. Genet. 83:435 (1992). GAT genes capable of conferring glyphosate resistance are described in WO 2005012515 to Castle et al. Genes conferring resistance to 2,4-D, fop and pyridyloxy auxin herbicides are described in WO 2005107437 and U.S. patent application Ser. No. 11/587,893.

Other herbicides can inhibit photosynthesis, including triazine (psbA and 1s+ genes) or benzonitrile (nitrilase gene). Przibila et al., Plant Cell 3:169 (1991), describes the transformation of Chlamydomonas with plasmids encoding mutant psbA genes. Nucleotide sequences for nitrilase genes are disclosed in U.S. Pat. No. 4,810,648 to Stalker, and DNA molecules containing these genes are available under ATCC Accession Nos. 53435, 67441, and 67442. Cloning and expression of DNA coding for a glutathione S-transferase is described by Hayes et al., Biochem. J. 285:173 (1992).

For purposes of the present invention, selectable marker genes include, but are not limited to genes encoding: neomycin phosphotransferase II (Fraley et al. (1986) CRC Critical Reviews in Plant Science, 4:1-25); cyanamide hydratase (Maier-Greiner et al. (1991) Proc. Natl. Acad. Sci. USA, 88:4250-4264); aspartate kinase; dihydrodipicolinate synthase (Perl et al. (1993) Bio/Technology, 11:715-718); tryptophan decarboxylase (Goddijn et al. (1993) Plant Mol. Bio., 22:907-912); dihydrodipicolinate synthase and desensitized aspartate kinase (Perl et al. (1993) Bio/Technology, 11:715-718); bar gene (Toki et al. (1992) Plant Physiol., 100:1503-1507 and Meagher *et al.* (1996) and Crop Sci., 36:1367); tryptophan decarboxylase (Goddijn et al. (1993) Plant Mol. Biol., 22:907-912); neomycin phosphotransferase (NEO) (Southern et al. (1982) J. Mol. Appl. Gen., 1:327; hygromycin phosphotransferase (HPT or HYG) (Shimizu et al. (1986) Mol. Cell Biol., 6:1074); dihydrofolate reductase (DHFR) (Kwok et al. (1986) PNAS USA 4552); phosphinothricin acetyltransferase (DeBlock et al. (1987) EMBO J., 6:2513); 2,2-dichloropropionic acid dehalogenase (Buchanan-Wollatron et al. (1989) J. Cell. Biochem. 13D:330); acetohydroxyacid synthase (Anderson et al., U.S. Pat. No. 4,761,373; Haughn et al. (1988) Mol. Gen. Genet. 221:266); 5-enolpyruvyl-shikimate-phosphate synthase (aroA) (Comai et al. (1985) Nature 317:741); haloarylnitrilase (Stalker et al., published PCT application WO87/04181); acetyl-coenzyme A carboxylase (Parker et al. (1990) Plant Physiol. 92:1220); dihydropteroate synthase (sul I) (Guerineau et al. (1990) Plant Mol. Biol. 15:127); and 32 kD photosystem II polypeptide (psbA) (Hirschberg et al. (1983) Science, 222:1346).

Also included are genes encoding resistance to: chloramphenicol (Herrera-Estrella et al. (1983) EMBO J., 2:987-992); methotrexate (Herrera-Estrella et al. (1983) Nature, 303:209-213; Meijer et al. (1991) Plant Mol Bio., 16:807-820 (1991); hygromycin (Waldron et al. (1985) Plant Mol. Biol., 5:103-108; Zhijian et al. (1995) Plant Science, 108:219-227 and Meijer et al. (1991) Plant Mol. Bio. 16:807-820); streptomycin (Jones et al. (1987) Mol. Gen. Genet., 210:86-91); spectinomycin (Bretagne-Sagnard et al. (1996) Transgenic Res., 5:131-137); bleomycin (Hille et al. (1986) Plant Mol. Biol., 7:171-176); sulfonamide (Guerineau et al. (1990) Plant Mol. Bio., 15:127-136); bromoxynil (Stalker et al. (1988) Science, 242:419-423); 2,4-D (Streber et al. (1989) Bio/Technology, 7:811-816); glyphosate (Shaw et al. (1986) Science, 233:478-481); and phosphinothricin (DeBlock et al. (1987) EMBO J., 6:2513-2518).

The above list of selectable marker and reporter genes are not meant to be limiting. Any reporter or selectable marker gene are encompassed by the present invention. If necessary, such genes can be sequenced by methods known in the art.

The reporter and selectable marker genes are synthesized for optimal expression in the plant. That is, the coding sequence of the gene has been modified to enhance expression in plants. The synthetic marker gene is designed to be expressed in plants at a higher level resulting in higher transformation efficiency. Methods for synthetic optimization of genes are available in the art. In fact, several genes have been optimized to increase expression of the gene product in plants.

The marker gene sequence can be optimized for expression in a particular plant species or alternatively can be modified for optimal expression in plant families. The plant preferred codons may be determined from the codons of highest frequency in the proteins expressed in the largest amount in the particular plant species of interest. See, for example, EPA 0359472; EPA 0385962; WO 91/16432; Perlak et al. (1991) Proc. Natl. Acad. Sci. USA, 88:3324-3328; and Murray et al. (1989) Nucleic Acids Research, 17: 477-498; U.S. Pat. No. 5,380,831; and U.S. Pat. No. 5,436,391. In this manner, the nucleotide sequences can be optimized for expression in any plant. It is recognized that all or any part of the gene sequence may be optimized or synthetic. That is, fully optimized or partially optimized sequences may also be used.

Genes that Confer Resistance to an Herbicide:
A. Resistance/tolerance of acetohydroxyacid synthase (AHAS) and acetolactate synthase (ALS) against herbicides imidazolinone or sulfonylurea. Genes and mutants for AHAS and mutants have been disclosed in U.S. Patent Nos. 4,761,373, 5,304,732, 5,331,107, 5,853,973, and 5,928,937. Genes and mutants for ALS have been disclosed in U.S. Patent Nos. 5,013,659 and 5,141, 870.
B. Resistance/tolerance genes of acetyl coemzyme A carboxylase (ACCase) against herbicides cyclohexanediones and/or aryloxyphenoxypropanoic acid (including Haloxyfop, Diclofop, Fenoxyprop, Fluazifop, Quizalofop) have been described in U.S. Patents 5,162,602 and 5,498,544.
C. Genes for glyphosate resistance/tolerance. Gene of 5-enolpyruvyl-3-phosphoshikimate synthase (ES3P synthase) has been described in U.S. Patent No. 4,769,601. Genes of 5-enolpyruvylshikimate-3-phosphate synthase (EPSPS) and mutants have been described in U.S. Patent Nos. 4,940,835, 5,188,642, 5,310,667, 5,633,435, 5,633,448, and 6,566,587.
D. Genes for glufosinate (bialaphos, phosphinothricin (PPT)) resistance/tolerance. Gene for phosphinothricin acetyltransferase (Pat) has been described in U.S. Patent Nos. 5,273,894, 5,276,268, and 5,550,318; and gene for bialaphos resistance gene (Bar) has been described in U.S. Patent Nos. 5,561,236 and 5,646,024, 5,648,477, and 7,112,665. Gene for glutamine synthetase (GS) has been described in U.S. Patent No. 4,975,372 and European patent application EP 0333033 A1.
E. Resistance/tolerance genes of hydroxy phenyl pyruvate dioxygenase (HPPD) against herbicides isoxazole, diketonitriles, and/or triketones including sulcotrione and mesotrione have been described in U.S. Patent Nos. 6,268,549 and 6,069,115.
F. Genes for 2,4-D resistance/tolerance. Gene of 2,4-D-monooxygenase has been described in U.S. Patent No. 6,100,446 and 6,153,401. Additional genes for 2,4-D resistance/tolerance are disclosed in US 2009/0093366 and WO 2007/053482.
G. Gene of imidazoleglycerol phosphate dehydratase (IGPD) against herbicides imidazole and/or triazole has been described in U.S. Patent No. 5,541,310. Genes of Dicamba degrading enzymes (oxygenase, ferredoxin, and reductase) against herbicide Dicamba have been disclosed in U.S. Patent Nos. 7,022,896 and 7,105,724.
H. Genes for herbicides that inhibit photosynthesis, including triazine (psbA and 1 s+ genes) or a benzonitrile (nitrilase gene). See *e.g.,* Przibila et al., Plant Cell 3:169 (1991) disclosing transformation of *Chlamydomonas* with plasmids encoding mutant psbA genes. Nucleotide sequences for nitrilase genes are disclosed in U.S. Patent No. 4,810,648 and DNA molecules containing these genes are available under ATCC Accession Nos. 53435, 67441, and 67442. Cloning and expression of DNA coding for a glutathione S-transferase is described by Hayes et al., Biochem. J. 285:173 (1992).

Unless otherwise specifically explained, all technical and scientific terms used herein have the same meaning as commonly understood by those of ordinary skill in the art to which this disclosure belongs. Definitions of common terms in molecular biology can be found in, for example: Lewin, Genes V, Oxford University Press, 1994 (ISBN 0-19-854287-9); Kendrew et al. (eds.), The Encyclopedia of Molecular Biology, Blackwell Science Ltd., 1994 (ISBN 0-632-02182-9); and Meyers (ed.), Molecular Biology and Biotechnology: A Comprehensive Desk Reference, VCH Publishers, Inc., 1995 (ISBN 1-56081-569-8).

Provided are constructs and methods relating to three EPSPS gene paralogs (A, B, and C) and their genetic components, such as 5' UTRs, promoters and transit peptides in *Brassica napus.* Also disclosed are transgenic and non-transgenic (in its native environment) uses of the genes and its genetic elements. In some embodiments, transgenic use of these genes or elements can confer traits of herbicide (for example, glyphosate or 2,4-D) tolerance in plant. Paralogs A and B share a high degree of homology or identity (∼92%) and so do C and E (∼95%). Paralog A is the highest expressing paralog in multiple tissue types and at different plant growth stages. The EPSPS paralog A gene is expressed constitutively in all tested plant tissues, for example leaves, roots, stems, apical meristem, flowers, flower buds etc, at 4 -8 leaf stages. In addition, paralog A has a unique transit peptide sequence, compared to the other four paralogs. In some embodiments, the transit peptide of EPSPS paralog A is used to provide effective translocation of protein precursors from cytoplasm to plastids. Further, the transit peptide of EPSPS paralogs B, C and E can be useful to provide translocation of protein precursors from cytoplasm to plastids. The EPSPS enzymes represent the sixth key enzyme of the shikimate pathway for synthesis of aromatic amino acids and aromatic metabolite in plants, fungi and microorganisms. Hence the EPSPS genes can be up- or down-regulated in plants by any existing or future technologies which are applied to manipulate the amino acid content in plants. See, for example, WO 2009/042164. All of these features, either alone or in combination makes the EPSPS paralogs important for use in transgenic or non-transgenic (native gene environment) applications to confer traits such as herbicide tolerance and/or alterations in the amino acid, carbon and nitrogen contents as a result of the manipulation of the shikimate and associated pathways. Of special interest is transgenic canola.

Provided is the promoter sequence of EPSPS paralog A from *B. napus* variety Nex710. This promoter of EPSPS paralog A is bidirectional based on results shown in transgenic *B. napus* callus tissue and plants, and is therefore designated as Brassica bidirectional constitutive promoter (BBCP). Use of BBCP in transgenic plants can provide at least one of the following advantages: (a) more genes can be stacked in one round of transformation into plant genome; (b) transgenes can be constitutively expressed in all plant tissues and parts; and (c) new genes can be further added or exchanged at the targeted locus with zinc finger-mediated precision gene stacking. For example, use of BBCP can enable expression of a selectable marker/herbicide resistance trait in one direction and a gene of interest (for example, trait of crop protection or yield enhancement) in another. Further provided is the unique transit peptide contained in the paralog A gene sequence to enable protein targeting to plastid for example chloroplast.

*B. napus* is an amphidiploid species resulting from the combination of two chromosome sets of *B. rapa* (*2n* = 20, AA) and *B. oleracea* (*2n* = 18, CC). Therefore, multiple EPSPS gene paralogs provided could either be homeologous or paralogous genes depending on their origin either from the A or C genomes (homeologous) or as a result of their duplication within a genome following speciation (paralogous).

Methods and constructs provided can be used to express any trait in canola or dicot/monocot plants, such as input traits (*e.g*., insect resistance and herbicide tolerance traits), agronomic traits (*e.g.,* yield enhancement), output traits (*e.g.,* healthy oil) *etc.* All methods pertaining to construction of specific vectors using BBCP and its transformation into canola or other plants are also provided.

Delivery and/or transformation: Suitable methods for transformation of plants include any method by which DNA can be introduced into a cell, for example and without limitation: electroporation (*see, e.g.,* U.S. Patent 5,384,253); microprojectile bombardment (*see, e.g.,* U.S. Patents 5,015,580, 5,550,318, 5,538,880, 6,160,208, 6,399,861, and 6,403,865); *Agrobacterium-*mediated transformation (*see, e.g.,* U.S. Patents 5,635,055, 5,824,877, 5,591,616; 5,981,840, and 6,384,301); and protoplast transformation (*see, e.g.,* U.S. Patent 5,508,184). Through the application of techniques such as the foregoing, the cells of virtually any plant species may be stably transformed, and these cells may be developed into transgenic plants by techniques known to those of skill in the art. For example, techniques that may be particularly useful in the context of cotton transformation are described in U.S. Patents 5,846,797, 5,159,135, 5,004,863, and 6,624,344; techniques for transforming *Brassica* plants in particular are described, for example, in U.S. Patent 5,750,871; techniques for transforming soya are described, for example, in U.S. Patent 6,384,301; and techniques for transforming maize are described, for example, in U.S. Patents 7,060,876 and 5,591,616, and International PCT Publication WO 95/06722.

After effecting delivery of an exogenous nucleic acid to a recipient cell, the transformed cell is generally identified for further culturing and plant regeneration. In order to improve the ability to identify transformants, one may desire to employ a selectable or screenable marker gene with the transformation vector used to generate the transformant. In this case, the potentially transformed cell population can be assayed by exposing the cells to a selective agent or agents, or the cells can be screened for the desired marker gene trait.

Cells that survive the exposure to the selective agent, or cells that have been scored positive in a screening assay, may be cultured in media that supports regeneration of plants. In some embodiments, any suitable plant tissue culture media (*e.g*., MS and N6 media) may be modified by including further substances, such as growth regulators. Tissue may be maintained on a basic media with growth regulators until sufficient tissue is available to begin plant regeneration efforts, or following repeated rounds of manual selection, until the morphology of the tissue is suitable for regeneration (*e.g*., at least 2 weeks), then transferred to media conducive to shoot formation. Cultures are transferred periodically until sufficient shoot formation has occurred. Once shoots are formed, they are transferred to media conducive to root formation. Once sufficient roots are formed, plants can be transferred to soil for further growth and maturity.

To confirm the presence of the desired nucleic acid molecule comprising constructs provided in the regenerating plants, a variety of assays may be performed. Such assays may include: molecular biological assays, such as Southern and Northern blotting and PCR; biochemical assays, such as detecting the presence of a protein product, *e.g.,* by immunological means (ELISA, Western blots, and/or LC-MS MS spectrophotometry) or by enzymatic function; plant part assays, such as leaf or root assays; and/or analysis of the phenotype of the whole regenerated plant.

Targeted integration events may be screened, for example, by PCR amplification using, *e.g*., oligonucleotide primers specific for nucleic acid molecules of interest. PCR genotyping is understood to include, but not be limited to, polymerase-chain reaction (PCR) amplification of genomic DNA derived from isolated host plant callus tissue predicted to contain a nucleic acid molecule of interest integrated into the genome, followed by standard cloning and sequence analysis of PCR amplification products. Methods of PCR genotyping have been well described (*see*, *e.g.,* Rios et al. (2002) Plant J. 32:243-53), and may be applied to genomic DNA derived from any plant species or tissue type, including cell cultures. Combinations of oligonucleotide primers that bind to both target sequence and introduced sequence may be used sequentially or multiplexed in PCR amplification reactions. Oligonucleotide primers designed to anneal to the target site, introduced nucleic acid sequences, and/or combinations of the two may be produced. Thus, PCR genotyping strategies may include, for example and without limitation: amplification of specific sequences in the plant genome; amplification of multiple specific sequences in the plant genome; amplification of non-specific sequences in the plant genome; and combinations of any of the foregoing. One skilled in the art may devise additional combinations of primers and amplification reactions to interrogate the genome. For example, a set of forward and reverse oligonucleotide primers may be designed to anneal to nucleic acid sequence(s) specific for the target outside the boundaries of the introduced nucleic acid sequence.

Forward and reverse oligonucleotide primers may be designed to anneal specifically to an introduced nucleic acid molecule, for example, at a sequence corresponding to a coding region within a nucleotide sequence of interest comprised therein, or other parts of the nucleic acid molecule. These primers may be used in conjunction with the primers described above. Oligonucleotide primers may be synthesized according to a desired sequence, and are commercially available (e.g., from Integrated DNA Technologies, Inc., Coralville, IA). Amplification may be followed by cloning and sequencing, or by direct sequence analysis of amplification products. One skilled in the art might envision alternative methods for analysis of amplification products generated during PCR genotyping. In one embodiment, oligonucleotide primers specific for the gene target are employed in PCR amplifications.

### EXAMPLES

### Example 1

### Identification of the EPSPS Paralog A Promoter (BBCP) Sequence

Five 5-enolpyruvyl shikimate-3-phosphate synthase (EPSPS) gene sequences (paralogs or homologues) in *Brassica napus* have been described in US 2009/0205083A1. Among these five genes, the promoter of the EPSPS paralog A drives strongest expression in various plant tissues. In order to expand the sequence of the 1571 nt EPSPS paralog A (SEQ ID NO: 7), additional sequences of the EPSPS paralog A gene are obtained via genome using a GenomeWalker™ universal kit (Clonetech Laboratories, Palo Alto, CA) to obtain the full sequence of the EPSPS paralog A (SEQ ID NO: 8) including its promoter region and the 3' untranslated region (for example SEQ ID NO: 26).

To identify promoter sequence of the EPSPS paralog A gene, the full sequence is searched using a Basic Local Alignment Search Tool (BLAST) against various plant and *Brassica* databases. Six cDNA and/or mRNA sequences of *Brassica napus* and *Brassica rapa* are identified that aligned in the direction of the EPSPS paralog A gene expression. The GenBank identification numbers (IDs) for these sequences are ES937178, ES904055, CD825798, CD835768, CD837464 and EV121915. These cDNA and/or mRNA sequences can be detected from leaf, root or embryo libraries of the target species but no specific role of these cDNA or mRNA has been annotated in GenBank. Interestingly, three cDNAs and/or mRNAs are identified matching the 5' sequence of EPSPS paralog A gene in the opposite direction to the expression of the EPSPS paralog A gene. The GenBank IDs of these sequences are: CD836095, EV100366 and EE568337. *Brassica napus* cDNA and/or mRNA sequences are the sources of these sequences, again with no specific function assigned to them in GenBank.

Sequence analysis of this example shows the promoter sequence of the EPSPS paralog A gene is a bidirectional promoter, which is designated as Brassica bidirectional constitutive promoter (BBCP).

### Example 2

### Design and Construction of BBCP Constructs

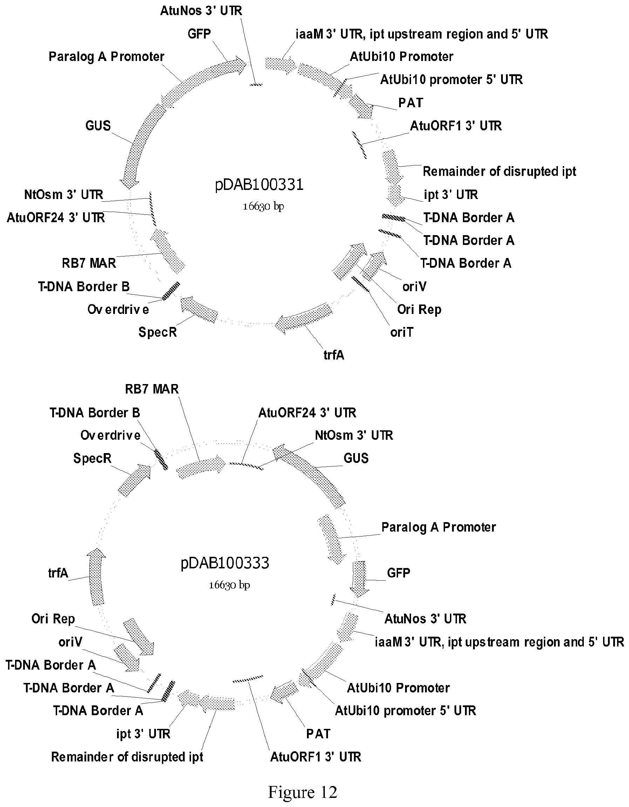

A single binary vector labeled as pDAB100333 (Figure 12) is constructed using art recognized procedures. Binary pDAB100333 contains two sets of gene expression cassettes or Plant Transcription Units (PTUs). The first PTU set consists of the bi-directional *Brassica napus* Paralog A promoter (BBCP) which drives two reporter genes. One end of the BBCP is constructed to drive the expression of the β-glucuronidase reporter gene (GUS), and is terminated by the *Agrobacterium tumefaciens* open reading frame-24 3' untranslated region (AtuORF24 3'UTR). The opposite end of the BBCP is constructed to drive the green fluorescent protein reporter gene (GFP) and is terminated with the *Agrobacterium tumefaciens* nopaline synthase 3' untranslated region (Atu Nos 3'UTR).

The second PTU set of pDAB 100333 includes a selectable marker cloned within the isopentenyltransferase coding sequence (ipt CDS; Genbank Acc No. X00639.1) thereby interrupting the jpt coding sequence, where the *Arabidopsis thaliana* Ubiquitin 10 promoter (AtUbi10 promoter) is used to drive the phosphinothricin acetyl transferase coding sequence (PAT), and the PTU is terminated by the *A. tumefaciens* open reading frame-1 3' untranslated region (AtuORF1 3'UTR). The resulting binary vector contains two visual reporter genes (GUS and GFP) driven by the bi-directional promoter and a selectable marker gene (PAT).

The binary vector, pDAB100333, is mobilized into *Agrobacterium tumefaciens* using electroporation. Individual colonies are identified on YEP media containing the antibiotic spectinomycin. Single colonies are isolated and the presence of the pDAB100333 binary vector can be confirmed via restriction enzyme digestion.

Another binary vector pDAB100331 (Figure 12) is also constructed using art recognized procedures. Binary pDAB 100331 is constructed to contain the BBCP in the reverse orientation as in pDAB100333 but with the same features as pDAB100333. Accordingly, binary vector pDAB100331 consists of two sets of gene expression cassettes or Plant Transcription Units (PTUs). The first PTU set consists of the bi-directional *Brassica napus* Paralog A promoter (BBCP in reverse orientation as compared to pDAB100333) which drives two reporter genes. One end of the BBCP is constructed to drive the green fluorescent protein reporter gene (GFP) and is terminated with the *Agrobacterium tumefaciens* nopaline synthase 3' untranslated region (Atu Nos 3'UTR). The opposite end of the BBCP is constructed to drive the expression of the β-glucuronidase reporter gene (GUS), and is terminated by the *Agrobacterium tumefaciens* open reading frame-24 3' untranslated region (AtuORF24 3'UTR).

The second PTU set ofpDAB100331 also includes a selectable marker cloned within the isopentenyltransferase coding sequence (ipt CDS; Genbank Acc No. X00639.1) thereby interrupting the jpt coding sequence, where the *Arabidopsis thaliana* Ubiquitin 10 promoter (AtUbi10 promoter) is used to drive the phosphinothricin acetyl transferase coding sequence (PAT), and the PTU is terminated by the *A. tumefaciens* open reading frame-1 3' untranslated region (AtuORF1 3'UTR). The resulting binary vector contains two visual reporter genes (GUS and GFP) driven by the bi-directional promoter and a selectable marker gene (PAT).

Similarly, the binary vector, pDAB100331, is mobilized into *Agrobacterium tumefaciens* using electroporation. Individual colonies are identified on YEP media containing the antibiotic spectinomycin. Single colonies are isolated and the presence of the pDAB100331 binary vector can be confirmed via restriction enzyme digestion.

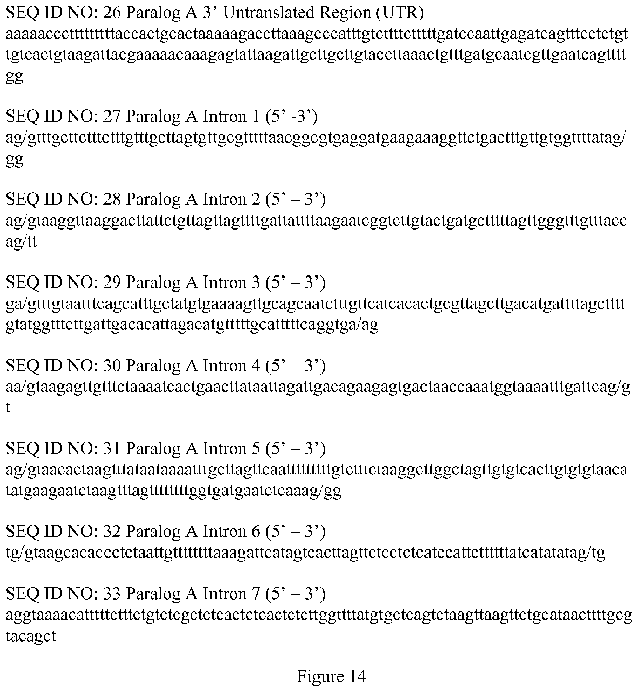

Direct DNA delivery vectors which are cloned into high copy number pUC based plasmids are constructed using only the first PTU containing the BBCP promoter which is described above. Plasmids pDAB108710 and pDAB108711 (Figure 14) are constructed using art recognized procedures. The two vectors differ as they are constructed to contain the BBCP in the different orientation to drive the same features. The single PTU consists of the bi-directional *Brassica napus* paralog A promoter which drives two reporter genes. One end of the BBCP is constructed to drive the green fluorescent protein reporter gene (GFP) and is terminated with the *Agrobacterium tumefaciens* nopaline synthase 3' untranslated region (AtuNos 3'UTR). The opposite end of the BBCP is constructed to drive the expression of the β-glucuronidase reporter gene (GUS), and is terminated by the *Agrobacterium tumefaciens* open reading frame-24 3' untranslated region (AtuORF24 3'UTR). The direct DNA delivery vectors are used for particle bombardment of maize tissues.

### Example 3

### Expression of BBCP Construct in Brassica napus

Canola transformation - Preparation of hypocotyl segment and pre-treatment: Seeds of the elite canola genotype, Nex710, are surface-sterilized with 10% commercial bleach for 10 minutes and rinsed 3 times with sterile distilled water. The seeds are dried via a sterile paper towel then placed in a Phyta-tray containing "germination medium" consisting of one half concentration of MS basal medium [Phytotech Cat# M 519 (PhytoTechnology Laboratories, Shawnee Mission, KS)], 20 g/L sucrose, and 8 g/L TC Agar and maintained under growth regime set at 23°C with a photoperiod of 16 hours light/8 hours dark.

On day five, seedlings are checked for sterility and the Phyta-tray is placed inside a laminar flow hood (The Baker Company EdgeGARD) to maintain sterility. Using sterile forceps and dissecting scissors, plants are removed from the Phyta-tray and the aerial (meristem and cotyledon) region and roots are detached and discarded. Hypocotyls are placed into a 100 x 25 mm petri dish containing sterile distilled water which is required to prevent drying. Hypocotyls are cut transversely into 2 mm segment, and lay horizontally on sterile filter paper over lay on "callus induction media MSK1D1" consisting of MS medium (Phytotech M519), 30 g/L sucrose, 1 mg/L kinetin, and 1 mg/L 2,4-D solidified with 7g/L TC Agar. The plates are placed into a clear Sterilite® tub and maintained under the same growth regime for three days, as a pre-treatment.

Preparation of *Agrobacterium*: Four days before *Agrobacterium* infection, pDAB10333 and pDAB10331 in *Agrobacterium* strain DA2552 (see, for example, WO 2012/016222) are streaked out from a glycerol stock, on to YEP medium (10 g/L Peptone, 10 g/L Yeast Extract, 5 g/L NaCl, 10 g/L Sucrose plus 100 mg/L spectinomycin and 150 mg/L erythromycin and solidified with 15 g/L Bacto Agar) and grown for two days in an incubator (Fisher Scientific Isotemp Incubator) at 28°C. Two days after, a small loop of *Agrobacterium* is placed into a 500mL sterile disposable baffled flask containing 150mL "liquid bacterial growth medium" (same medium as above but minus solidifying agent). The culture is grown for sixteen hours at 28°C in the dark on an enclosed shaker (New Brunswick Scientific Innova 4330 refrigerated incubator shaker) at 200 rpm. After sixteen hours the *Agrobacterium* culture is removed from the shaker and aliquoted into 50mL centrifuge tubes. The centrifuge tubes are placed into a centrifuge (Beckman Model J2-21 centrifuge) and centrifuged at 6,000 rpm for 15 minutes and subsequently re-suspended in the "liquid culture medium M" consisting of LS salts (Phytotech L689), 3% glucose, modified Gamborg B5 vitamins (Phytotech G249), 215 mg/L Kinetin, and 221 mg/L 2,4-D at pH 5.

Infection and callus induction: On the day of infection, canola hypocotyl segments are transferred into a 100 x 25 sterile petri plate containing 20 mL of the "liquid culture medium" while waiting for *Agrobacterium* to be ready. The "liquid culture medium" is then removed from the hypocotyl segments and 40 mL of *Agrobacterium* suspension is vortexed briefly and poured into the 100 x 25mm petri dish containing hypocotyl segments for a 30 minutes treatment. After the 30 minutes treatment, all of the *Agrobacterium* suspensions are removed using a double stacked pipette. The treated hypocotyls are placed back onto filter paper overlay on the "callus induction medium MSK1D1." The culture is returned to the Sterilite® tub, covered with a dark lid and returned to the culture room under the same growth regime as above, for a three days co-cultivation period. After the three days co-cultivation period, the hypocotyls are placed directly onto "selection 1 medium MSK1D1H1" (consisting of "callus induction medium" plus 1 mg/L Herbiace), placed back into the tub with a clear lid and returned to the culture room, maintaining the same growth regime as above. After one week, the hypocotyls are then transferred directly to "selection 2 medium MSK1D1H3" (consisting of "callus induction medium" plus 3 mg/L Herbiace). After two weeks, the hypocotyls are transferred to "selection 3 medium MSK1D1H5" (consisting of "callus induction media" plus 5 mg/l Herbiace). The hypocotyl segments are continued to be transferred every two weeks onto fresh selection 3 medium until enough callus are formed on the both ends of hypocotyls. The calluses are then assayed for GUS.

GUS stain of canola hypocotyl segments: The GUS stain procedure is known in the art with slight modification of GUS stain solution as follows: 0.1M NaPO₄ buffer at pH 8, 0.5 mM K₃(Fe(CN)₆, 10 mM Na₂EDTA, 1 mg/ml X-Gluc, and 0.06 % Triton X-100. Chlorophyll from the stained tissue is removed by 70% ethanol. GUS assay is done in hypocotyl segments after being on selection 3 media for at least two weeks. The calli are immersed in GUS staining solution and incubated overnight in dark at 37°C. Uninfected tissue and GUS positive control are routinely included in the assay for negative and positive controls.

The results show significant GUS expression in transgenic callus samples obtained from both pDAB100331 and pDAB100333 transformation. No blue color is visible in non-transgenic control samples. Thus, the transgenic experimental results confirm that BBCP is a bidirectional promoter.

### Example 4

### Expression of BBCP Construct in Soybean

*Agrobacterium tumefaciens* strain EHA105 is electroporated with the binary vector pDAB9381 (a control binary vector which does not contain the BBCP bidirectional promoter), pDAB100331 and pDAB100333 separately. Isolated colonies are identified on YEP media containing the antibiotic spectinomycin. Single colonies are isolated and the presence of the pDAB9381, pDAB100331 and pDAB100333 binary vector can be confirmed via restriction enzyme digestion. *Agrobacterium*-mediated transformation of soybean (*Glycine max* c.v. Maverick) can be performed according to methods well known in the art.

After transformation, once roots are developed, the rooted plantlets are photographed for GFP expression with a 482 nm/502 nm GFP filter covering excitation/emission. Leaves are sampled from the plantlets for GUS staining according to the protocol adapted from Jefferson, R., (1987) "Histochemical localization of β-glucuronidase (GUS) reporter activity in plant tissues" Plant Mol. Biol. Reporter, 5: 387-405. Leaves are then immersed in staining solution comprised of: 2X Phosphate buffer pH7.0 (1 x made of 0.1 M NaH₂PO₄ and 0.1 M Na₂HPO₄), 0.5 mM K₃(Fe(CN)₆, 10 mM Na₂EDTA, 1 mg/ml X-Gluc and 0.06% Triton X-100, and incubated overnight at 37°C. After incubation, staining solution is removed and tissue are washed with several changes of 70% ethanol and left overnight in ethanol before photographs are taken.

Results of four rounds of experiments are shown in Table 2 for explants and transgenic plantlets tested. Table 2 shows the number of transgenic shoots that are confirmed to contain expressed protein products of GFP and GUS reporter genes driven by BBCP. Soybean transgenic plants stably transformed with constructs pDAB100331 and pDAB100333 are regenerated with a frequency of 3-14 %. Approximately 81-99 % of the regenerated shoots (plantlets) express GFP while 41 % of them express both GFP and GUS. In the plants regenerated with pDAB100333, GUS expression is more uniform throughout the leaf and not primarily expressed in the midrib tissue and veins. Comparatively in the plants regenerated from pDAB100331, GUS expression appears more localized in the midrib and veins of the leaves as observed in studies completed on multiple leaves. Transgenic plants transformed with a control construct, pDAB9381, do not show any GFP or GUS expression. The results confirm that the BBCP drives transgene expression in both directions at a reasonable level, although there might be some minor directional differences in the expression patterns within leaf tissue.

| Table 2. GFP and GUS reporter genes expression by BBCP in explants and transgenic plantlets tested. | | | | |
|---|---|---|---|---|
| Construct | No. of Explants infected | No. of shoots regenerated | No. of shoots showing GFP | No. of shoots showing GUS |
| pDAB9381 | 1096 | 246 (22.4%) | 236 (96%) | 0 |
| pDAB100331 | 1158 | 139 (12.0%) | 138 (99%) | 57 (41%) |
| pDAB100333 | 1153 | 160 (14.0%) | 154 (96%) | 63(41%) |

### Example 5

### Transient Transformation of Maize Leaf Tissue

Tissue Preparation: Dark grown leaf tissue is harvested three to four hours prior to bombardment and placed on a bombardment preparation medium described in Table 3. Plates are wrapped and stored at 28 °C in dark until ready for bombardment.

Microparticles (gold) preparation: 30 mg gold (1 µm in size purchased from Bio-Rad, Hercules, CA) is washed in 500 µl cold ethanol, sonicated for fifteen seconds, and then vortexed for fifteen seconds. The particles are centrifuged for sixty seconds at 3000 rpm after settled for ten minutes. Supernatant is then discarded and pellet is washed with cold water (disrupt pellet with pipette tip and/or finger vortex) followed by centrifugation for sixty seconds at 3000 rpm. This wash and centrifugation step can be repeated two more times. After final rinse, 250 µl 50 % glycerol (final concentration ∼120 mg/ml) is added. The samples are sonicated for fifteen seconds, vortexed for fifteen seconds, and made aliquot into eppendorf tubes.

Preparing Microcarriers: For each plate of tissue to be bombarded, the gold/DNA reaction is prepared as follows: 5-15 µg DNA (plasmid pDAB108710, dab108711, or control plasmid DNA), 6 mg gold, final concentrations of 1 M CaCl₂ and 16 mM spermidine, in a total reaction volume of 125 µl. Immediately after sonicating and vortexing, 50 µl gold suspension aliquots are made into each reaction tube. The reaction tubes are vortexed before addition of 50 µl pre-chilled CaCl₂, and then vortexed again before addition of 20 µl 0.1 M Spermidine. The reaction tubes are then vortexed for up to ten minutes, and let sit for ten minutes on bench before centrifuge for fifteen seconds at 5000 rpm. Supernatant is discarded and pellets are resuspended in 150 µl 70% ethanol. Pellets are then disrupted with pipette and/or finger vortex before centrifuged for fifteen seconds at 5000 rpm. Supernatant is then discarded and pellets are resuspended in 150 µl ethanol. Pellets are then again disrupted with pipette and/or finger vortex before centrifuged for fifteen seconds at 5000 rpm. Finally, pellets are resuspended in 36 µl ethanol (36 µl per 6 mg gold) before bombardment experiment. Prior to aliquoting onto the microcarriers, pellets are sonicated for fifteen seconds and vortexed until the gold appears well suspended. Aliquot pellets onto thee microcarriers for 10 µl each. Sonication and vortex between aliquots is recommended.

| Table 3. Ingredients for bombardment preparation medium. | | |
|---|---|---|
| **Ingredient** | **Amount** | **Unit** |
| MS salts | 4.330 | g/L |
| 1,2,3,5/4,6-hexahydroxycyclohexane | 100.000 | mg/L |
| MES | 0.500 | g/L |
| EPS0000063-L-proline | 700.000 | mg/L |
| casein enzymatic hydrolysate | 100.000 | mg/L |
| sucrose | 30.000 | g/L |
| Gelzan (Gelrite) 714246 | 2.300 | g/L |
| EPS0000200-isu modified MS vitamin (1000x) | 1.000 | mL/L |
| EPS0000205-dicamba-KOH - 6.6 mg/ml | 3.300 | mg/L |
| EPS0000206-silver nitrate - 8.5 mg/ml | 15.000 | mg/L |
| Mannitol | 45.50 | g/L |

Bombardment Conditions: Helium-based microcarrier disks and a microcarrier holder are prepped and autoclaved prior to use. DNA-coated gold particles (10 µl well suspended) are placed on microcarriers for five minutes until dry. To assemble the microcarrier launch for shooting, the stop screen is first placed in the assembly unit. Then the microcarrier is placed upside-down on the assembly unit and then the assembly microcarrier lid is closed before a freshly and briefly rinsed (in 70% ethanol) 1350 psi rupture disk is placed in retaining cap and attached to the gas-acceleration tube. The microcarrier-assembly unit is then immediately placed at the top-level slot before chamber door is closed. Vacuum is activated in evacuation chamber before bombardment. Samples are bombarded by pressing and holding fire button until 1350 psi rupture disk bursts and helium pressure gauge drops to zero. Each plate can be bombarded up to three times with the same plasmid, where the plates can be turned so that the bombardment may take place at a different site/direction on the plate. After bombardment, plates are stored in the dark at 28 °C for at least twenty four hours before GFP observation and/or GUS staining using a Sigma-Aldrich staining kit.

Results: GUS expression is observed in leaf tissues bombarded with plasmids containing GUS gene driven by BBCP. Maize leaf tissues bombarded with a control plasmid do not show any GUS expression. GFP expression is observed in maize leaf samples transformed with plasmids having GFP gene driven by BBCP. Maize leaf tissues bombarded with a control construct do not show any GFP expression. The results confirm that BBCP is a bidirectional promoter driving expression of the reporter genes, GUS and GFP, in both directions. In conclusion, bidirectional expression from BBCP can be observed in both dicot (soybean *and B. napus*) and monocot (maize) plant tissues.

## Claims

1. A nucleic acid construct for expressing multiple genes in plant cells and/or tissues, comprising,
(a) a bi-directional promoter comprising a nucleotide sequence selected from SEQ ID NO: 2 or 3; and
(b) two gene expression cassettes on opposite ends of the bi-directional promoter.

2. The nucleic acid construct of claim 1, wherein the bi-directional promoter comprises at least one enhancer or wherein the nucleic acid construct comprises a binary vector for *Agrobacterium-mediated* transformation or wherein the bi-directional promoter comprises at least one intron or wherein the bi-directional promoter comprises at least one 5' untranslated region.

3. The nucleic acid construct of claim 1, wherein at least one of the gene expression cassettes comprises two or more genes linked via a translation switch.

4. The nucleic acid construct of claim 1, wherein both the gene expression cassettes comprise two or more genes linked via a translation switch or wherein both the gene expression cassettes do not comprise a EPSPS gene or paralog.

5. The nucleic acid construct of claim 3, wherein the translation switch is selected from the group consisting of an internal ribosome entry site (IRES), an alternative splicing site, a polynucleotide sequence coding a 2A peptide, a polynucleotide sequence coding a 2A-like peptide, a polynucleotide sequence coding an intein, a polynucleotide sequence coding a protease cleavage site, and combinations thereof or wherein a gene upstream of the translational switch does not comprise a translation stop codon.

6. The nucleic acid construct of claim 1, wherein the nucleic acid construct enables expression of at least four genes or wherein the nucleic acid construct enables expression of between three and twenty genes and in particular wherein the nucleic acid construct enables expression of between four and eight genes.

7. A method for generating a transgenic plant, comprising transforming a plant cell with the nucleic acid construct of claim 1.

8. A method for generating a transgenic cell, comprising transforming the cell with the nucleic acid construct of claim 1.

9. A plant cell comprising the nucleic acid construct of claim 1 and in particular wherein the nucleic acid construct is stably transformed into the plant cell.

10. A transgenic plant or seed comprising the nucleic acid construct of claim 1.

11. The transgenic plant or seed of claim 10, wherein the nucleic acid construct is stably transformed into cells of the transgenic plant or seed or wherein the transgenic plant is a dicotyledonous plant or wherein the transgenic plant is a monocotyledonous plant.

12. A method for expressing multiple genes in plant cells and/or tissues, comprising introducing into the plant cells and/or tissues the nucleic acid construct of claim 1 and in particular, wherein the plant cells and/or tissues are stably transformed with the nucleic acid construct of claim 1.

13. A binary vector for *Agrobacterium-mediated* transformation comprising the nucleic acid construct of claim 1.

14. The use of a bi-directional promoter in the manufacturing of transgenic plants or seeds, wherein the bi-directional promoter comprises a nucleotide sequence selected from SEQ ID NO: 2 or 3.

## Patentansprüche

1. Nukleinsäurekonstrukt zum Exprimieren mehrerer Gene in Pflanzenzellen und oder -geweben, umfassend
(a) einen bidirektionalen Promotor, umfassend eine Nukleotidsequenz, ausgewählt aus SEQ ID NO: 2 oder 3; und
(b) zwei Genexpressionskassetten an entgegengesetzten Enden des bidirektionalen Promotors.

2. Nukleinsäurekonstrukt nach Anspruch 1, worin der bidirektionale Promotor mindestens einen Enhancer umfasst, oder worin des Nukleinsäurekonstrukt einen binären Vektor für *Agrobacterium*-vermittelte Transformation umfasst, oder worin der bidirektionale Promotor mindestens ein Intron umfasst, oder worin der bidirektionale Promotor mindestens eine 5'-nichttranslatierte Region umfasst.

3. Nukleinsäurekonstrukt nach Anspruch 1, worin mindestens eine der Genexpressionskassetten zwei oder mehr Gene umfasst, die über einen Translationsschalter verbunden sind.

4. Nukleinsäurekonstrukt nach Anspruch 1, worin beide Genexpressionskassetten zwei oder mehr Gene umfassen, die über einen Translationsschalter verbunden sind, oder worin die beiden Genexpressionskassetten kein EPSPS-Gen oder Paralogon umfassen.

5. Nukleinsäurekonstrukt nach Anspruch 3, worin der Translationsschalter ausgewählt ist aus der Gruppe, bestehend aus einer internen Ribosomen-Eintrittsstelle (IRES), einer alternativen Spleißstelle, einer Polynukleotidsequenz, die für ein 2A-Peptid codiert, einer Polynukleotidsequenz, die für ein 2A-ähnliches Peptid codiert, einer Polynukleotidsequenz, die für ein Intein codiert, einer Polynukleotidsequenz, die für eine Proteasespaltungsstelle codiert, und Kombinationen davon, oder worin ein Gen stromaufwärts von dem Translationsschalter kein Translationsstoppcodon umfasst.

6. Nukleinsäurekonstrukt nach Anspruch 1, worin das Nukleinsäurekonstrukt Expression von mindestens vier Genen ermöglicht, oder worin das Nukleinsäurekonstrukt Expression von zwischen drei und zwanzig Genen ermöglicht, oder insbesondere, worin das Nukleinsäurekonstrukt Expression von zwischen 4 und 8 Genen ermöglicht.

7. Verfahren zum Herstellen einer transgenen Pflanze, umfassend das Transformieren einer Pflanzenzelle mit dem Nukleinsäurekonstrukt nach Anspruch 1.

8. Verfahren zum Herstellen einer transgenen Zelle, umfassend das Transformieren der Zelle mit dem Nukleinsäurekonstrukt nach Anspruch 1.

9. Pflanzenzelle, umfassend das Nukleinsäurekonstrukt nach Anspruch 1 und insbesondere, worin das Nukleinsäurekonstrukt stabil in die Pflanzenzelle transformiert ist.

10. Transgene Pflanze oder transgener Samen, umfassend das Nukleinsäurekonstrukt nach Anspruch 1.

11. Transgene Pflanze oder transgener Samen nach Anspruch 10, worin das Nukleinsäurekonstrukt stabil transformiert ist in Zellen der transgenen Pflanze oder des transgenen Samens, oder worin die transgene Pflanze eine dikotyle Pflanze ist, oder worin die transgene Pflanze eine monokotyle Pflanze ist.

12. Verfahren zum Exprimieren mehrerer Gene in Pflanzenzellen und/oder -geweben, umfassend das Einbringen des Nukleinsäurekonstrukts nach Anspruch 1 in die Pflanzenzellen und oder Gewebe, worin insbesondere die Pflanzenzellen und oder Gewebe stabil transformiert werden mit dem Nukleinsäurekonstrukt nach Anspruch 1.

13. Binärer Vektor für *Agrobacterium*-vermittelte Transformation, umfassend das Nukleinsäurekonstrukt nach Anspruch 1.

14. Verwendung eines bidirektionalen Promotors bei der Herstellung transgener Pflanzen oder Samen, worin der bidirektionale Promotor eine Nukleinsäuresequenz umfasst, ausgewählt aus SEQ ID NO: 2 oder 3.

## Revendications

1. Construction d'acide nucléique pour l'expression de gènes multiples dans des cellules et/ou des tissus végétaux, comprenant :
a) un promoteur bidirectionnel comprenant une séquence de nucléotides choisie parmi les Séquences N° 2 et N° 3,
b) et deux cassettes d'expression de gènes, placées aux extrémités opposées du promoteur bidirectionnel.

2. Construction d'acide nucléique conforme à la revendication 1, dans laquelle le promoteur bidirectionnel comporte au moins un amplificateur, ou laquelle construction d'acide nucléique comporte un vecteur binaire pour transformation au moyen d'*Agrobacterium*, ou dans laquelle le promoteur bidirectionnel comporte au moins un intron, ou dans laquelle le promoteur bidirectionnel comporte au moins une région 5' non-traduite.

3. Construction d'acide nucléique conforme à la revendication 1, dans laquelle au moins l'une des cassettes d'expression de gènes comporte deux gènes, ou plus, reliés par un aiguilleur de traduction.

4. Construction d'acide nucléique conforme à la revendication 1, dans laquelle les deux cassettes d'expression de gènes comportent deux gènes ou plus, reliés par un aiguilleur de traduction, ou dans laquelle les deux cassettes d'expression de gènes ne comportent pas de gène d'EPSPS ou de gène paralogue.

5. Construction d'acide nucléique conforme à la revendication 3, dans laquelle l'aiguilleur de traduction est choisi dans l'ensemble constitué par un site interne de liaison du ribosome (IRES), un site d'épissage alternatif, une séquence polynucléotidique codant un peptide 2A, une séquence polynucléotidique codant un peptide de type 2A, une séquence polynucléotidique codant une intéine, une séquence polynucléotidique codant un site de clivage par protéase, et les combinaisons de tels éléments, ou dans laquelle un gène en amont de l'aiguilleur de traduction ne comporte pas de codon d'arrêt de traduction.

6. Construction d'acide nucléique conforme à la revendication 1, laquelle construction d'acide nucléique permet l'expression d'au moins quatre gènes, ou laquelle construction d'acide nucléique permet l'expression de trois à vingt gènes, ou laquelle construction d'acide nucléique, en particulier, permet l'expression de quatre à huit gènes.

7. Procédé de génération d'un végétal transgénique, comprenant le fait de transformer une cellule végétale avec une construction d'acide nucléique conforme à la revendication 1.

8. Procédé de génération d'une cellule transgénique, comprenant le fait de transformer une cellule avec une construction d'acide nucléique conforme à la revendication 1.

9. Cellule végétale comprenant une construction d'acide nucléique conforme à la revendication 1, en particulier dans laquelle la construction d'acide nucléique est insérée de manière stable dans la cellule végétale ainsi transformée.

10. Végétal ou semence transgénique, comprenant une construction d'acide nucléique conforme à la revendication 1.

11. Végétal ou semence transgénique conforme à la revendication 10, dans lequel ou laquelle la construction d'acide nucléique est insérée de manière stable dans des cellules du végétal ou de la semence transgénique, ou lequel végétal transgénique est une dicotylédone, ou lequel végétal transgénique est une monocotylédone.

12. Procédé d'expression de plusieurs gènes dans des cellules et/ou tissus végétaux, comprenant le fait d'introduire dans ces cellules et/ou tissus végétaux une construction d'acide nucléique conforme à la revendication 1, et en particulier, dans lequel les cellules et/ou tissus végétaux sont transformées de manière stable avec une construction d'acide nucléique conforme à la revendication 1.

13. Vecteur binaire pour transformation au moyen d'*Agrobacterbium*, comprenant une construction d'acide nucléique conforme à la revendication 1.

14. Utilisation d'un promoteur bidirectionnel dans la production de végétaux ou semences transgéniques, lequel promoteur bidirectionnel comprend une séquence de nucléotides choisie parmi les Séquences N° 2 et N° 3.
